# EUROPÄISCHE PATENTSCHRIFT

(11) **EP 1 053 224 B1**
(45) Veröffentlichungstag und Bekanntmachung des Hinweises auf die Patenterteilung: **21.08.2002**
(21) Anmeldenummer: 99913138.6
(22) Anmeldetag: 04.02.1999
(51) Int. Cl.: C07C 311/46, C07C 311/37, C07D 333/24, C07D 333/60, C07D 209/18, A61K 31/18

(54) **BIPHENYLSULFONYLCYANAMIDE, VERFAHREN ZU IHRER HERSTELLUNG UND IHRE VERWENDUNG ALS MEDIKAMENT**
BIPHENYLSULFONYL CYANAMIDES, METHOD FOR THE PRODUCTION THEREOF AND THEIR UTILIZATION AS A MEDICAMENT
BIPHENYLSULFONYLCYANAMIDES, LEUR PROCEDE DE PREPARATION ET LEUR UTILISATION COMME MEDICAMENT

(30) Priorität: 04.02.1998 DE 19804251
(43) Veröffentlichungstag der Anmeldung: 22.11.2000
(73) Patentinhaber: Aventis Pharma Deutschland GmbH, 65929 Frankfurt am Main (DE)
(72) Erfinder: KLEEMANN, Heinz-Werner, D-65474 Bischofsheim (DE); LANG, Hans-Jochen, D-65719 Hofheim (DE); SCHWARK, Jan-Robert, D-65779 Kelkheim (DE); WEICHERT, Andreas, D-63329 Egelsbach (DE); FABER, Sabine, D-65510 Idstein (DE); JANSEN, Hans-Willi, D-65527 Niedernhausen (DE); PETRY, Stefan, D-65929 Frankfurt am Main (DE)
(86) Internationale Anmeldenummer: EP9900724
(87) Internationale Veröffentlichungsnummer: WO99040064

(56) Entgegenhaltungen:
- FR-A- 2 716 883
- US-A- 5 281 614
- US-A- 5 412 097

## Beschreibung

Die Erfindung betrifft Verbindungen der Formel (I), 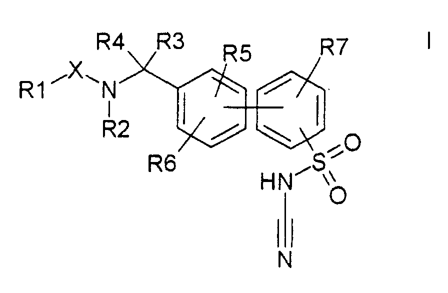 in welcher die Symbole folgende Bedeutung haben:
- R(1): 1. Alkyl mit 1, 2, 3, 4, 5, 6, 7 oder 8 C-Atomen;
2. Alkyl mit 1, 2, 3, 4, 5, 6, 7 oder 8 C-Atomen, worin ein bis alle Wasserstoffatome durch Fluor ersetzt sind;
3. Alkenyl mit 2, 3, 4, 5, 6, 7, 8, 9, 10, 11 oder 12 C-Atomen; oder
4. -CₙH₂ₙ₋ₙₙ -Y,
   nn Null oder 2; und
   n Null, 1, 2, 3 oder 4; wobei n ungleich Null oder 1 ist, wenn nn gleich 2 ist;
5. -CₙH₂ₙ₋ₙₙ -Y,
   nn Null oder 2; und
   n 1, 2, 3 oder 4; wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl;
   2. Amino;
   3. N(R(22)R(23);
   4. Alkoxycarbonyl;
   5. COOR(16);
   6. Alkyl mit 1, 2, 3 oder 4 C-Atomen;
   7. (C₆-C₁₄)-Aryl-(C₁-C₄)-alkyl-carbonyl, vorzugsweise Phenylacetyl;
- R(2): 1. Wasserstoff;
2. Alkyl mit 1, 2, 3, 4, 5, 6, 7 oder 8 C-Atomen;
3. Alkyl mit 1, 2, 3, 4, 5, 6, 7 oder 8 C-Atomen, worin ein bis alle Wasserstoffatome durch Fluor ersetzt sind;
4. Alkenyl mit 2, 3, 4, 5, 6, 7, 8, 9, 10, 11 oder 12 C-Atomen;
5. Alkinyl mit 2, 3, 4, 5, 6, 7 oder 8 C-Atomen;
6. -CₙH₂ₙ₋ₙₙ -Z,
   nn Null oder 2; und
   n Null, 1, 2, 3 oder 4; wobei n ungleich Null oder 1 ist, wenn nn gleich 2 ist;
7. -CₙH₂ₙ₋ₙₙ -Z,
   nn Null oder 2; und
   n 1, 2, 3 oder 4, wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl;
   2. Amino;
   3. N(R(22)R(23);
   4. (C₁-C₄)-Alkoxycarbonyl;
   5. COOR(16);
   6. Alkyl mit 1, 2, 3 oder 4 C-Atomen;
- R(3) und R(4): unabhängig voneinander Wasserstoff oder Alkyl mit 1, 2, 3, 4, 5, 6, 7 oder 8 C-Atomen;
- R(5), R(6) und R(7): unabhängig voneinander Wasserstoff, Alkyl mit 1, 2, 3, 4, 5, 6, 7 oder 8 C-Atomen, F, Cl, Br, I, CF₃, -CN, -NO₂, SO_{q}-R(8), CO-R(21) oder O-R(10);
- R(8): Alkyl mit 1, 2, 3, 4, 5, 6, 7 oder 8 C-Atomen, NR(11)R(12) oder Phenyl, das unsubstituiert ist oder substituiert ist mit 1, 2 oder 3 gleichen oder verschiedenen Resten aus der Reihe F, Cl, Br, I, CF₃, Methyl, Methoxy, Hydroxy oder NR(11)R(12);
- R(9) und R(21): unabhängig voneinander Wasserstoff, Alkyl mit 1, 2, 3, 4, 5, 6, 7 oder 8 C-Atomen oder OR(13);
- R(10): Wasserstoff, Alkyl mit 1, 2, 3, 4, 5, 6, 7 oder 8 C-Atomen gegebenenfalls substituiert mit (C₁-C₄)-Alkoxy; oder Phenyl, das unsubstituiert ist oder substituiert ist mit 1, 2 oder 3 gleichen oder verschiedenen Resten aus der Reihe F, Cl, Br, I, CF₃, Methyl, Methoxy, Hydroxy oder NR(11)R(12);
- R(11), R(12), R(19) und R(20): unabhängig voneinander Wasserstoff, Alkyl mit 1, 2, 3 oder 4 C-Atomen oder (C₁-C₄)-Alkanoyl, vorzugsweise Acetyl;
- R(13): Wasserstoff oder Alkyl mit 1, 2, 3, 4, 5, 6, 7 oder 8 C-Atomen;
- X: Carbonyl, -CO-NH-, -CO-CO- oder Sulfonyl;
- Y und Z: unabhängig voneinander
1. Aryl mit 6, 7, 8, 9, 10, 11, 12, 13 oder 14 C-Atomen, bevorzugt Phenyl, 1-Naphthyl oder 2-Naphthyl;
2. einen wie unter 1. definierten Rest, der mit 1, 2, 3, 4 oder 5 gleichen oder verschiedenen Resten aus der Reihe Alkyl mit 1, 2, 3, 4, 5, 6, 7 oder 8 C-Atomen, Aryl mit 6, 7, 8, 9, 10, 11, 12, 13 oder 14 C-Atomen, bevorzugt Phenyl, 1-Naphthyl oder 2-Naphthyl, F, Cl, Br, I, CF₃, SO_{q}R(18), OR(16), NR(19)R(20), -CN, NO₂ oder CO-R(9) substituiert ist; oder wobei zwei Reste gemeinsam einen anellierten Heterocyclylrest, vorzugsweise Methylendioxy, bilden.
3. Heteroaryl mit 1, 2, 3, 4, 5, 6, 7, 8 oder 9 C-Atomen;
4. einen wie unter 3. definierten Rest, der mit 1, 2 oder 3 gleichen oder verschiedenen Resten aus der Reihe F, Cl, Br, I, CF₃, CH₃, Methoxy, Hydroxy oder NR(11)R(12) substituiert ist;
5. Cycloalkyl mit 3, 4, 5, 6, 7, 8, 9 oder 10 C-Atomen, bevorzugt Cyclopropyl, Cyclopentyl, Cyclohexyl. 1,2,3,4-Tetrahydronaphthyl oder Indanyl;
6. einen wie unter 5. definierten Rest, substituiert mit Aryl mit 6, 7, 8, 9, 10, 11, 12, 13 oder 14 C-Atomen, bevorzugt Phenyl, 1-Naphthyl oder 2-Naphthyl;
7. O-R(14);
8. O-R(17);
9. -SO₂-R(14);
10.1 Arylalkylcarbonyl, vorzugsweise Phenyl-CH₂-CO-; oder
11. Heterocyclyl;
- R(14) und R(17): unabhängig voneinander
1. Wasserstoff;
2. Alkyl mit 1, 2, 3, 4, 5, 6, 7 oder 8 C-Atomen;
3. Alkenyl mit 2, 3, 4, 5, 6, 7, 8, 9, 10, 11 oder 12 C-Atomen;
4. -CₙH₂ₙ₋ₙₙ - Phenyl,
   nn Null oder 2; und
   n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3, 4, 5, 6, 7 oder 8 C-Atomen, F, Cl, Br, I, CF₃, SO_{q}R(15), OR(16), NR(11)R(12), -CN, -NO₂ oder CO-R(9); oder
- R(15) und R(18): unabhängig voneinander Alkyl mit 1, 2, 3 oder 4 C-Atomen, Alkyl mit 1, 2, 3 oder 4 C-Atomen, worin ein bis alle Wasserstoffatome durch Fluor ersetzt sind, bevorzugt CF₃, oder NR(11)R(12);
- R(16): 1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind; bevorzugt CF₃;
5. Aryl mit 6, 7, 8, 9, 10, 11, 12, 13 oder 14 C-Atomen, bevorzugt Phenyl, 1-Naphthyl oder 2-Naphthyl;
6. einen wie unter 5. definierten Rest, der mit 1, 2 oder 3 gleichen oder verschiedenen Resten aus der Reihe F, Cl, Br, I, CF₃, NR(19)R(20), -CN, NO₂ substituiert ist;
- R(22) und R(23): unabhängig voneinander Wasserstoff oder CO-OR(24);
- R(24): Wasserstoff, Alkyl mit 1, 2, 3, 4, 5, 6, 7 oder 8 C-Atomen oder -CₙH₂ₙ- Phenyl mit n gleich 1, 2, 3 oder 4;
- q: unabhängig voneinander Null, 1 oder 2;
sowie deren physiologisch verträglichen Salze.

Bevorzugt sind Verbindungen der Formel (I) mit
- R(1): 1. Alkyl mit 1, 2, 3, 4 oder 5 C-Atomen;
2. Alkyl mit 1, 2, 3, 4 oder 5 C-Atomen, worin ein bis alle Wasserstoffatome durch Fluor ersetzt sind;
3. Alkenyl mit 2, 3, 4, 5, 6, 7, 8, 9, 10, 11 oder 12 C-Atomen; oder
4. -CₙH₂ₙ₋ₙₙ -Y,
   nn Null oder 2; und
   n Null, 1, 2, 3 oder 4; wobei n ungleich Null oder 1 ist, wenn nn gleich 2 ist;
5. -CₙH₂ₙ₋ₙₙ -Y,
   nn Null oder 2; und
   n 1, 2, 3 oder 4; wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl;
   2. Amino;
   3. NR(22)R(23);
   4. Alkoxycarbonyl;
   5. COOR(16);
   6. Alkyl mit 1, 2, 3 oder 4 C-Atomen;
   7. (C₆-C₁₄)-Aryl-(C₁-C₄)-alkyl-carbonyl, vorzugsweise Phenylacetyl;
- R(2): 1. Wasserstoff;
2. Alkyl mit 1, 2, 3, 4, 5 oder 5 C-Atomen;
3. Alkyl mit 1, 2, 3, 4, oder 5 C-Atomen, worin ein bis alle Wasserstoffatome durch Fluor ersetzt sind;
4. Alkenyl mit 2, 3, 4, 5, 6, 7, 8, 9, 10, 11 oder 12 C-Atomen;
5. Alkinyl mit 2, 3, 4, oder 5 C-Atomen;
6. -CₙH₂ₙ₋ₙₙ -Z,
   nn Null oder 2; und
   n Null, 1, 2, 3 oder 4; wobei n ungleich Null oder 1 ist, wenn nn gleich 2 ist;
7. -CₙH₂ₙ₋ₙₙ -Z,
   nn Null oder 2; und
   n 1, 2, 3 oder 4, wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl;
   2. Amino;
   3. N(R(22)R(23);
   4. (C₁-C₄)-Alkoxycarbonyl;
   5. COOR(16);
   6. Alkyl mit 1, 2, 3 oder 4 C-Atomen;
- R(3) und R(4): unabhängig voneinander Wasserstoff oder Alkyl mit 1, 2, 3, oder 4 C-Atomen;
- R(5), R(6) und R(7): unabhängig voneinander Wasserstoff, Alkyl mit 1, 2, 3 oder 4 C-Atomen, F, Cl, Br, CF₃, -CN, SO_{q}-R(8), CO-R(21) oder O-R(10);
- R(8): Alkyl mit 1, 2, 3 oder 4 C-Atomen, NR(11)R(12) oder Phenyl, das unsubstituiert ist oder substituiert ist mit 1 oder 2 gleichen oder verschiedenen Resten aus der Reihe F, Cl, Br, CF₃, Methyl, Methoxy, Hydroxy oder NR(11)R(12);
- R(9) und R(21): unabhängig voneinander Wasserstoff, Alkyl mit 1, 2, 3 oder 4 C-Atomen oder OR(13);
- R(10): Wasserstoff, Alkyl mit 1, 2, 3 oder 4 C-Atomen gegebenenfalls substituiert mit (C₁-C₄)-Alkoxy; oder Phenyl, das unsubstituiert ist oder substituiert ist mit 1 oder 2 gleichen oder verschiedenen Resten aus der Reihe F, Cl, Br, CF₃, Methyl, Methoxy, Hydroxy oder NR(11)R(12);
- R(11), R(12), R(19) und R(20): unabhängig voneinander Wasserstoff, Alkyl mit 1, 2, 3 oder 4 C-Atomen oder (C₁-C₄)-Alkanoyl, vorzugsweise Acetyl;
- R(13): Wasserstoff oder Alkyl mit 1, 2, 3 oder 4 C-Atomen;
- X: Carbonyl, -CO-NH-, -CO-CO- oder Sulfonyl;
- Y und Z: unabhängig voneinander
1. Phenyl, 1-Naphthyl oder 2-Naphthyl;
2. einen wie unter 1. definierten Rest, der mit 1, 2, 3, 4 oder 5 gleichen oder verschiedenen Resten aus der Reihe Alkyl mit 1, 2, 3 oder 4 C-Atomen, Phenyl, 1-Naphthyl oder 2-Naphthyl, F, Cl, Br, CF₃, SO_{q}R(18), OR(16), NR(19)R(20), -CN oder CO-R(9) substituiert ist; oder wobei zwei Reste gemeinsam einen anellierten Heterocyclylrest, vorzugsweise Methylendioxy, bilden;
3. Heteroaryl mit 1, 2, 3, 4, 5, 6, 7, 8 oder 9 C-Atomen;
4. einen wie unter 3. definierten Rest, der mit 1 oder 2 gleichen oder verschiedenen Resten aus der Reihe F, Cl, Br, CF₃, CH₃, Methoxy, Hydroxy oder NR(11)R(12) substituiert ist;
5. Cycloalkyl mit 3, 4, 5, 6, 7, 8, 9 oder 10 C-Atomen, bevorzugt Cyclopropyl, Cyclopentyl, Cyclohexyl. 1,2,3,4-Tetrahydronaphthyl oder Indanyl;
6. einen wie unter 5. definierten Rest, substituiert mit Phenyl, 1-Naphthyl oder 2-Naphthyl;
7. O-R(14);
8. O-R(17);
9. -SO₂-R(14);
10. Arylalkylcarbonyl, vorzugsweise Phenyl-CH₂-CO-; oder
11. Heterocyclyl;
- R(14) und R(17): unabhängig voneinander
1. Wasserstoff;
2. Alkyl mit 1, 2, 3 oder 4 C-Atomen;
3. Alkenyl mit 2, 3, 4, 5 oder 6 C-Atomen;
4. -CₙH₂ₙ₋ₙₙ- Phenyl,
   nn Null oder 2; und
   n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3 oder 4 C-Atomen, F, Cl, Br, CF₃, SO_{q}R(15), OR(16), NR(11)R(12), -CN, oder CO-R(9); oder
- R(15) und R(18): unabhängig voneinander Alkyl mit 1, 2, 3 oder 4 C-Atomen, Alkyl mit 1, 2, 3 oder 4 C-Atomen, worin ein bis alle Wasserstoffatome durch Fluor ersetzt sind, bevorzugt CF₃, oder NR(11)R(12);
- R(16): 1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind, bevorzugt CF₃;
5. Phenyl, 1-Naphthyl oder 2-Naphthyl;
6. einen wie unter 5. definierten Rest, der mit 1, 2 oder 3 gleichen oder verschiedenen Resten aus der Reihe F, Cl, Br, CF₃, NR(19)R(20), -CN, substituiert ist;
- R(22) und R(23): unabhängig voneinander Wasserstoff oder CO-OR(24);
- R(24): Wasserstoff, Alkyl mit 1, 2, 3 oder 4 C-Atomen oder -CₙH₂ₙ - Phenyl mit n gleich 1, 2 oder 3;
- q: unabhängig voneinander Null, 1 oder 2;
sowie deren physiologisch verträglichen Salze.

Besonders bevorzugt sind Verbindungen der Formel (I) mit
- R(1): 1. Alkyl mit 1, 2, 3, 4 oder 5 C-Atomen,
2. Alkenyl mit 2, 3 oder 4 C-Atomen,
3. -CₙH₂ₙ₋ₙₙ - Y;
   Y
   1. Phenyl;
   2. einen wie unter 1. definierten Rest, der mit 1, 2, 3, 4 oder 5 gleichen oder verschiedenen Resten aus der Reihe Alkyl mit 1, 2, 3 oder 4 C-Atomen, F, Cl, Br, Cyano, CF₃, Hydroxy, NO₂, SO₂R(18), OR(16), SCF₃, NR(19)R(20), CO-R(9);
   3. OR(14), oder
   4. SO₂-R(14);
   5. 1-Naphthyl oder 2-Naphthyl;
   6. einen wie unter 5 definierten Rest, der mit einem Rest aus der Reihe Alkyl mit 1, 2, 3 oder 4 C-Atomen, F, Cl, CF₃, SO₂R(18), OR(16), NR(19)R(20) oder CO-R(9) substituiert ist;
   7. Heteroaryl mit 1, 2, 3, 4, 5, 6, 7, 8 oder 9 C-Atomen; vorzugsweise Thienyl, Benzothiophenyl, Indolyl oder Furyl;
   8. einen wie unter 3. definierten Rest, der mit einem Rest aus der Reihe F, Cl, CF₃, CH₃, Methoxy oder N(CH₃)₂ substituiert ist;
   9. Cycloalkyl mit 3, 4, 5, 6 oder 7 C-Atomen;

   nn Null oder 2; und
   n Null, 1, 2, 3 oder 4, wobei n ungleich Null oder 1 ist, wenn nn gleich 2 ist;
4. -CₙH₂ₙ₋ₙₙ -Y,
   Y
   1. Phenyl;
   2. OR(14); oder
   3. Heteroaryl, vorzugsweise Thienyl;

   nn Null oder 2; und
   n 1, 2 oder 3, wobei n ungleich 1 ist, wenn nn gleich 2 ist; worin 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl; oder Phenylacetyl;
   2. Amino;
   3. NR(22)R(23); oder
   4. Alkyl mit 1, 2, 3 oder 4 C-Atomen;
- R(2): 1. Wasserstoff
2. Alkyl mit 1, 2, 3, 4 oder 5 C-Atomen;
3. Alkyl mit 1, 2, 3, 4 oder 5 C-Atomen, worin ein bis alle Wasserstoffatome durch Fluor ersetzt sind;
4. Alkenyl mit 2, 3, 4, 5, 6, 7, 8, 9 oder 10 C-Atomen;
5. Alkinyl mit 2, 3, 4 oder 5 C-Atomen
6. -CₙH₂ₙ₋ₙₙ - Z;
   Z
   1. Phenyl;
   2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, Br, CF₃, SO₂R(18), OR(16), Nitro, Cyano, NR(19)R(20), CO-R(9), oder wobei zwei Reste gemeinsam einen Methylendioxy-Rest bilden;
   3. 1-Naphthyl, oder 2-Naphthyl;
   4. einen wie unter 3. definierten Rest, der mit einem Rest aus der Reihe Alkyl mit 1, 2, 3 oder 4 C-Atomen, F, Cl, CF₃, SO₂R(18), OR(16), Nitro, Cyano, NR(19)R(20) oder CO-R(9) substituiert ist;
   5. Heteroaryl mit 1, 2, 3, 4, 5, 6, 7, 8 oder 9 C-Atomen, vorzugsweise Benzimadozolyl, Pyridyl, Thienyl, Furyl, Tetrahydrofuryl, Pyrrolidinyl, Pyrrolidine-1-carbonyl-4,5 dihydro-isoxazolyl, Benzofuranyl, beispielsweise 1,3-Dihydro-1-oxo-Benzo[c]furanyl, Quinazolinyl; beispielsweise 3,4-Dihydroquinazolinyl;
   6. einen wie unter 5. definierten Rest, der mit einem Rest aus der Reihe F, Cl, CF₃, CH₃, Methoxy, Hydroxy oder N(CH₃)₂ substituiert ist;
   7. Cycloalkyl mit 3, 4, 5, 6, 7, 8, 9 oder 10 C-Atomen; vorzugsweise, Cyclopropyl, Cyclopentyl, Cyclohexyl, 1,2,3,4-Tetrahydronaphthyl oder Indanyl;
   8. einen wie unter 7. definierten Rest, der mit Phenyl substituiert ist; vorzugsweise Phenylcyclopentyl;

   nn Null oder 2; und
   n Null, 1, 2 oder 3, wobei n ungleich Null oder 1 ist, wenn nn gleich 2 ist;
7. -CₙH₂ₙ₋ₙₙ -Z,
   Z
   1. Phenyl;
   2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, CF₃, SO₂R(18), -OR(16), Nitro, Cyano, NR(19)R(20) oder CO-R(9) substituiert ist;

   nn Null oder 2; und
   n 1, 2 oder 3, wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. (C₁-C₄)-Alkoxycarbonyl;
   2. COOR(16); oder
   3. Alkyl mit 1, 2, 3 oder 4 C-Atomen;
8. -CₙH₂ₙ -OR(17);
   n Null, 1,2 oder 3 ;
- R(3) und R(4): Wasserstoff oder Methyl;
- R(5), R(6) und R(7): unabhängig voneinander Wasserstoff, Alkyl mit 1, 2, 3 oder 4 C-Atomen, F, Cl, CF₃, CN, SO₂-R(8), CO-R(21) oder O-R(10);
- R(8): Alkyl mit 1, 2, 3 oder 4 C-Atomen, N(CH₃)₂ oder Phenyl, das unsubstituiert ist oder substituiert ist mit einem Rest aus der Reihe F, Cl, CF₃, Methyl, Methoxy, Hydroxy oder N(CH₃)₂ ;
- R(9) und R(21): unabhängig voneinander Wasserstoff, Methyl oder OR(13);
- R(10): Wasserstoff, Alkylmit 1, 2, 3 oder 4 C-Atomen, gegebenenfalls substituiert mit (C₁-C₄)-Alkoxy, oder Phenyl, das unsubstituiert ist oder substituiert ist mit einem Rest aus der Reihe F, Cl, CF₃, Methyl, Methoxy oder N(CH₃)₂;
- R(11) und R(12): unabhängig voneinander Wasserstoff, Alkyl mit 1, 2, 3 oder 4 C-Atomen oder (C₁-C₄)-Alkanoyl, vorzugsweise Acetyl;
- R(13): Wasserstoff oder Alkyl mit 1, 2, 3 oder 4 C-Atomen;
- X: Carbonyl, -CO-CO-, -NH-CO- oder Sulfonyl;
1.Wasserstoff;
2. Alkyl mit 1, 2, 3 oder 4 C-Atomen;
3. Alkenyl mit 2, 3, 4, 5 oder 6 C-Atomen;
4. -CₙH₂ₙ₋ₙₙ - Phenyl,
   nn Null oder 2; und
   n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3 oder 4 C-Atomen, F, Cl, Br, CF₃, SO_{q}R(15), OR(16), NR(11)R(12), -CN, oder CO-R(9);
- R(15): Alkyl mit 1, 2, 3 oder 4 C-Atomen oder N(CH₃)₂;
- R(16): 1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind, bevorzugt CF₃;
5. Phenyl, 1-Naphthyl oder 2-Naphthyl;
6. einen wie unter 5. definierten Rest, der mit 1, 2 oder 3 gleichen oder verschiedenen Resten aus der Reihe F, Cl, Br, CF₃, NR(19)R(20), -CN, substituiert ist;
- R(17): 1. Wasserstoff;
2. Alkyl mit 1, 2, 3 oder 4 C-Atomen;
3. Alkenyl mit 2, 3, oder 4 C-Atomen;
4. -CₙH₂ₙ₋ₙₙ - Phenyl,
   nn Null oder 2; und
   n Null, 1, 2, 3 oder 4; wobei n ungleich Null oder 1 ist, wenn nn gleich 2 ist;
5. einen wie unter 4. definierten Rest, wobei der Phenylteil substituiert ist mit einem Rest aus der Reihe Alkyl mit 1, 2, 3 oder 4 C-Atomen, F, Cl, Br, CF₃, SO_{q}R(15), OR(16), NR(11)R(12), -CN, oder CO-R(9); oder
- R(18): Alkyl mit 1, 2, 3 oder 4 C-Atomen, Alkyl mit 1, 2, 3 oder 4 C-Atomen, worin ein bis alle Wasserstoffatome durch Fluor ersetzt sind, bevorzugt CF₃, oder NR(11)R(12);
- R(19) und R(20): unabhängig voneinander Wasserstoff, Alkyl mit 1, 2, 3 oder 4 C-Atomen oder (C₁-C₄)-Alkanoyl, vorzugsweise Acetyl;
- R(22) und R(23): unabhängig voneinander Wasserstoff oder CO-OR(24);
- R(24): Wasserstoff, Alkyl mit 1, 2, 3 oder 4 C-Atomen oder -CₙH₂ₙ - Phenyl mit n gleich 1 oder 2;
- q: unabhängig voneinander Null, 1 oder 2;
sowie deren physiologisch verträglichen Salze.

Ganz besonders bevorzugt sind Verbindungen der Formel (I) mit
- R(1): 1. Alkyl mit 1, 2, 3, 4 oder 5 C-Atomen,
2. Alkenyl mit 2, 3 oder 4 C-Atomen,
3. -CₙH₂ₙ₋ₙₙ - Y;
   Y
   1. Phenyl;
   2. einen wie unter 1. definierten Rest, der mit 1, 2, 3, 4 oder 5 gleichen oder verschiedenen Resten aus der Reihe Alkyl mit 1, 2, 3 oder 4 C-Atomen, F, Cl, Br, Cyano, CF₃, Hydroxy, NO₂, SO₂R(18), OCH₃, OCF₃, SCF₃, N(CH₃)₂, NH-CO-CH₃,CO-R(9), Phenoxy oder Phenoxy, einfach oder mehrfach substituiert mit Halogen, bevorzugt Cl oder F, substituiert ist;
   3. OR(14), oder
   4. SO₂-R(14);

   nn Null oder 2; und
   n Null, 1, 2, 3 oder 4, wobei n ungleich Null oder 1 ist, wenn nn gleich 2 ist;
4. -CₙH₂ₙ₋ₙₙ -Y,
   Y
   1. Phenyl;
   2. OR(14); oder
   3. Heteroaryl, vorzugsweise Thienyl;
      nn Null oder 2; und

   n 1, 2 oder 3, wobei n ungleich 1 ist, wenn nn gleich 2 ist; worin 1, 2 oder 3 Wasserstoffatome in dem zweiwertigen Rest -CₙH₂ₙ₋ₙₙ- unabhängig voneinander ersetzt sind durch einen Rest aus der Reihe
   1. Aryl mit 6, 7, 8, 9, 10, 11, 12, 13 oder 14 C-Atomen, bevorzugt Phenyl, 1-Naphtyhl oder 2-Naphthyl; oder Phenylacetyl;
   2. Amino;
   3. NR(22)R(23); oder
   4. Alkyl mit 1, 2, 3 oder 4 C-Atomen;
5. -CₙH₂ₙ-Y;
   Y
   1. 1-Naphthyl oder 2-Naphthyl;
   2. einen wie unter 1. definierten Rest, der mit einem Rest aus der Reihe Alkyl mit 1, 2, 3 oder 4 C-Atomen, F, Cl, CF₃, SO₂R(18), OCH₃, N(CH₃)₂ oder CO-R(9) substituiert ist;
   3. Heteroaryl mit 1, 2, 3, 4, 5, 6, 7, 8 oder 9 C-Atomen; vorzugsweise Thienyl, Benzothiophenyl, Indolyl oder Furyl;
   4. einen wie unter 3. definierten Rest, der mit einem Rest aus der Reihe F, Cl, CF₃, CH₃, Methoxy oder N(CH₃)₂ substituiert ist;
   5. Cycloalkyl mit 3, 4, 5, 6 oder 7 C-Atomen;

   n Null, 1, 2, 3 oder 4;
6. -CₙH₂ₙ-OR(14);
   n Null 1 oder 2;
- R(2): 1. Alkyl mit 1, 2, 3, 4 oder 5 C-Atomen;
2. Alkyl mit 1, 2, 3, 4 oder 5 C-Atomen, worin ein bis alle Wasserstoffatome durch Fluor ersetzt sind;
3. Alkenyl mit 2, 3, 4, 5, 6, 7, 8, 9 oder 10 C-Atomen;
4. Alkinyl mit 2, 3, 4 oder 5 C-Atomen
5 -CₙH₂ₙ₋ₙₙ - Z;
   Z
   1. Phenyl;
   2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, Br, CF₃, SO₂R(18), -OCH₃, -O(C₂H₄)OCH₃, Ethoxy, Hydroxy, Nitro, Cyano, N(CH₃)₂, -NH-CO-CH₃, CO-R(9), Phenoxy oder Phenoxy, einfach oder mehrfach substituiert mit Halogen, bevorzugt Cl oder F, substituiert ist; oder wobei zwei Reste gemeinsam einen Methylendioxy-Rest bilden;

   nn Null oder 2; und
   n Null, 1, 2 oder 3, wobei n ungleich Null oder 1 ist, wenn nn gleich 2 ist;
6. -CₙH₂ₙ₋ₙₙ -Z,
   Z
   1. Phenyl;
   2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, CF₃, SO₂R(18), -OCH₃, -O(C₂H₄)OCH₃, Ethoxy, Hydroxy, Nitro, Cyano, N(CH₃)₂ oder CO-R(9) substituiert ist;

   nn Null oder 2; und
   n 1, 2 oder 3, wobei n ungleich 1 ist, wenn nn gleich 2 ist; wobei 1, 2 oder 3 Wasserstoffatome in dem zweiwertigen Rest -CₙH₂ₙ₋ₙₙ- unabhängig voneinander ersetzt sind durch einen Rest aus der Reihe
   1. (C₁-C₄)-Alkoxycarbonyl;
   2. COOR(16); oder
   3. Alkyl mit 1, 2, 3 oder 4 C-Atomen;
7. -CₙH₂ₙ -Z;
   Z
   1. 1-Naphthyl, oder 2-Naphthyl;
   2. einen wie unter 1. definierten Rest, der mit einem Rest aus der Reihe Alkyl mit 1, 2, 3 oder 4 C-Atomen, F, Cl, CF₃, SO₂R(18), OCH₃, -O(C₂H₄)OCH₃, Ethoxy, Hydroxy, Nitro, Cyano, N(CH₃)₂, -NHCOCH₃ oder CO-R(9) substituiert ist;
   3. Heteroaryl mit 1, 2, 3, 4, 5, 6, 7, 8 oder 9 C-Atomen, vorzugsweise Benzimadozolyl, Pyridyl, Thienyl, Furyl, Tetrahydrofuryl, Pyrrolidinyl, Pyrrolidine-1-carbonyl-4,5 dihydro-isoxazolyl, Benzofuranyl, beispielsweise 1,3-Dihydro-1-oxo-Benzo[c]furanyl, Quinazolinyl; beispielsweise 3,4-Dihydroquinazolinyl;
   4. einen wie unter 3. definierten Rest, der mit einem Rest aus der Reihe F, Cl, CF₃, CH₃, Methoxy, Hydroxy oder N(CH₃)₂ substituiert ist;
   5. Cycloalkyl mit 3, 4, 5, 6, 7, 8, 9 oder 10 C-Atomen; vorzugsweise, Cyclopropyl, Cyclopentyl, Cyclohexyl, 1,2,3,4-Tetrahydronaphthyl oder Indanyl;
   6. einen wie unter 5. definierten Rest, der mit Phenyl substituiert ist; vorzugsweise Phenylcyclopentyl;

   n Null, 1, 2 oder 3;
8. -CₙH₂ₙ-OR(17);
   n 2 oder 3;
- R(3) und R(4): Wasserstoff;
- R(5), R(6) und R(7): unabhängig voneinander Wasserstoff, Alkyl mit 1, 2, 3 oder 4 C-Atomen, F, Cl, CF₃, SO₂-R(8), CO-R(21) oder O-R(10);
- R(8): Methyl oder N(CH₃)₂;
- R(9) und R(21): unabhängig voneinander Wasserstoff, Methyl oder OR(13);
- R(10): Wasserstoff, Methyl oder Ethyl, gegebenenfalls substituiert mit Methoxy, oder Phenyl, das unsubstituiert ist oder substituiert ist mit einem Rest aus der Reihe F, Cl, CF₃, Methyl, Methoxy oder N(CH₃)₂;
- R(13): Wasserstoff oder Alkyl mit 1, 2, 3 oder 4 C-Atomen;
- X: Carbonyl, -CO-CO-, -NH-CO- oder Sulfonyl;
- R(14): 1. Wasserstoff;
2. Methyl oder Ethyl;
3. Alkenyl mit 2, 3, 4, 5 oder 6 C-Atomen, vorzugsweise Allyl;
4. -CₙH₂ₙ - Phenyl mit n gleich Null oder 1;
5. einen wie unter 4. definierten Rest, wobei der Phenylteil substituiert ist mit einem Rest aus der Reihe Alkyl mit 1, 2, 3 oder 4 C-Atomen, F, Cl, CF₃, SO₂R(15), OCH₃, N(CH₃)₂ oder CO-R(9); oder
6. Alkenyl mit 2, 3 oder 4 C-Atomen;
- R(15): Methyl oder N(CH₃)₂;
- R(16): Wasserstoff oder Alkyl mit 1, 2, 3, oder 4 C-Atomen, bevorzugt Methyl oder tert-Butyl
- R(17)
1. Wasserstoff;
2. Methyl;
3. -CₙH₂ₙ - Phenyl mit n gleich Null oder 1;
4. einen wie unter 3. definierten Rest, wobei der Phenylteil substituiert ist mit einem Rest aus der Reihe Alkyl mit 1, 2, 3 oder 4 C-Atomen, F, Cl, CF₃, SO₂R(15), OCH₃, N(CH₃)₂ oder CO-R(9); oder
5. Alkenyl mit 2, 3 oder 4 C-Atomen;
- R(18): Methyl, CF₃, Amino oder N(CH₃)₂;
- R(22) und R(23): unabhängig voneinander Wasserstoff oder CO-OR(24);
- R(24): Wasserstoff, Alkyl mit 1, 2, 3 oder 4 C-Atomen oder -CₙH₂ₙ- Phenyl mit n gleich 1 oder 2;
sowie deren physiologisch verträglichen Salze.

Bevorzugt sind auch Verbindungen der Formel la, 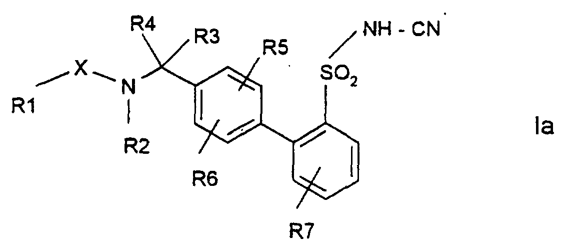 wobei die Reste X und R(1) bis R(7) die oben genannte Bedeutung haben, sowie deren physiologisch verträglichen Salze.

Weiterhin bevorzugt sind Verbindungen der Formel I und/oder la, worin die Reste X, R(1), R(2), R(3), R(4), R(5), R(6) und R(7) eine der in den Beispielen 1-568 beschriebene Bedeutung haben.

Die Erfindung betrifft somit auch Verbindungen der Formel I und/oder la mit
R(1)
   CH₃-CH₂-CH₂-, Cyclohexyl-CH₂-CH₂-, cyclohexyl-, 3-Methoxy-phenyl-CH₂-, 2-Methylphenyl-CH₂-, 2-Methylphenyl, 2-Thienyl-CH₂-, 4-t-Butyl-phenyl-, 2-Fluorophenyl-, Allyl-O-, 3, 4, 5-Trimethoxy-phenyl-, 2-Chloro-phenyl-, Phenyl-, CH₃-, E-CH₃-CH=CH-, CH₃-(CH₂)₃-, Phenyl-CH₂-CH₂-, 4-Methyl-phenyl-, Benzo[b]thiophen-2-yl-, Thien-2-yl-, (Phenyl)CH₂-, Phenyl-CH₂-CO-CH(Phenyl)-, (4-Chlorophenyl)-CH₂-, (CH₃)₃C-CH₂-, Phenyl-O-CH₂-, (4-Methoxyphenyl)-CH₂-, 2-Thienyl-CH₂-CH₂-CH₂-, (2-Thienyl)-, (Indol-3-yl)-CH₂-, Benzo[b]thiophen-3-yl)-CH₂-, (3-Thienyl)-CH₂-, Phenyl-CH(NH₂)-, Phenyl-CH(NHCO-O-Benzyl)-, (2-Nitrophenyl)-CH₂-, Phenyl-(CH₂)₄-, (2-Furyl)-, Cyclohexyl-CH₂-CH₂-, (2-Bromophenyl)-CH₂-, Phenyl-SO₂-CH₂-CH₂-, (4-Hydroxyphenyl)-CH₂-, Phenyl-CH₂-NH-, N-Cyclohexyl-NH-, N-(2,6-Difluorophenyl)-NH-, N-Isopropyl-NH-, N-Cyclohexyl-NH-, Methoxy-CH₂-CH₂-, 2-Methoxy-phenyl-, Phenoxy-CH₂-CH₂-, Cyclohexyl-CH₂-, 4-Fluorophenyl-, Phenyl-CH₂-, Cyclopentyl-CH₂-, 4-Methylphenyl-, 3, 4-Dimethoxyphenyl-, 2, 5-Dimethoxyphenyl-, 2, 3, 4, 5, 6-Pentamethylphenyl-, 3-Fluoro-2, 4-dimethylphenyl-, 4-Fluoro-3, 5-dimethylphenyl-, 2, 4, 5-Trichlorophenyl-, 4-tert. Butylphenyl-, 2, 3-Dichlorophenyl-, (3-Thienyl)-CH-(CH₃)-, (2-Thienyl)-CH-(CH₃)-, 4-Chlorophenyl-, 4-Trifluoromethyl-phenyl-, 4-Methoxyphenyl-, 2-Chlorophenyl-, 2-Fluorophenyl-, (2-Thienyl)-(CH₂)₄-, Phenyl-(CH₂)₄-, (2-Thienyl)-CH₂-CH₂-, (2-Chlorophenyl)-CH₂-, 3, 4-Dichlorophenyl-, 2, 4-Dichlorophenyl-, 2, 5-Dichlorophenyl-, 2, 4-Difluorophenyl-, (2-Fluorophenyl)-CH₂-, (2-Furyl)-CH₂-, 2-Chlorophenyl-, 2-Chloro-6-methylphenyl-, (2-Thienyl)-CH₂-, (3-Thienyl)-CH₂-, 4-Acetylaminophenyl-, 3-Trifluoromethyl-phenyl-, 2-Trifluoromethyl-phenyl-, 5-Methyl-2thienyl-, 2-Trifluoromethoxyphenyl, 2-Trifluoromethylphenyl-CH₂, 2-Trifluoromethoxyphenyl-CH₂-, 2-Trifluoromethylthiophenyl-CH₂-, 2-Naphtyl-, 4-Fluoro-3, 5-dimethylphenyl-, (5-methyl-2-thienyl)-CH-(CH₃)-;
R(2)
   Benzyl, CH₂-CH₂-OH, 4-Methoxy-benzyl, 4-Chloro-benzyl, Phenyl, Cyclohexyl, 2-Methoxy-benzyl, 2-Hydroxy-ethyl, 2-Chloro-benzyl, Isobutyl, 1 (S)-Phenylethyl, 4-Methyl-benzyl, 3-Methoxy-benzyl, 3, 4-Methylenedioxy-benzyl, 5-Fluor-(2-Methoxy)ethoxy-benzyl, 1(R)-Phenyl-ethyl, 4-Trifluoromethyl-benzyl, 2-(4-Methoxyphenyl)-ethyl, 1(S)-(4-Methylphenyl)-ethyl, 2-Furylmethyl, 1(R)-(4-Methylphenyl)-ethyl, 4-(Dimethylamino)-benzyl, 2-(2-Thienyl)-ethyl, 2-Phenoxyethyl, 2,3-Dichloro-benzyl, Cyclopropylmethyl, 3, 4, 5-Trimethoxy-benzyl, (4-Pyridyl)-methyl, 4-Fluoro-benzyl, 2, 4-Dimethoxy-benzyl, 3-Phenyl-propyl, 2-Methoxy-ethyl, Methyl, Ethyl, (2-Pyridyl)-methyl, Cyclopropyl, (2-Benzimidazolyl)-methyl, 1-Methoxycarbonyl-2-phenyl-ethyl, (3, 4-Dihydro-quinazolin-2-yl)-methyl, Propargyl, (2-Thienyl)-methyl, Cyclohexyl-methyl, 3, 4-Dichloro-benzyl, Phenyl-CH(COOC(CH₃)3), Phenyl-CH(COOCH₃), 2, 4-Dichloro-benzyl, (1-Naphthyl)-methyl, 2-(4-H₂NSO₂-Phenyl)-ethyl, 3-Chloro-benzyl, 2, 3-Dichloro-benzyl, 3-Methyl-benzyl, 2-Methylbenzyl, (CH₃)2C=CH-CH₂-CH₂-C(CH₃)=CH-CH₂, 1-Indanyl, 1, 2, 3, 4-Tetrahydro-1-naphthyl, 2-Fluoro-benzyl, 3-Fluoro-benzyl, (1, 2, 3, 4-Tetrahydro-2-furyl)-methyl, 2, 4-Difluorophenyl, 4-tert.-Butylphenyl, 2, 6-Dichlorophenyl, 3-Chloro-4-fluorophenyl, 4-Chloro-2, 5-dimethyloxyphenyl, 2-Cyanophenyl, 2-Chlorophenyl, 2, 3-Dichlorophenyl, 4-Fluorophenyl, 2, 3-Dichlorophenyl, 4-(4-Fluorophenoxy)-phenyl, 3-Trifluoromethyl-phenyl, 2-Chloro-4-cyanophenyl, 2-Acetyl-phenyl, (1-Phenylcylopentyl)-methyl, 2-Ethyl-2-(4-Methoxyphenyl)-butyl, [3-(Pyrrolidine-1-carbonyl)-4, 5-dihydro-isoxazol-5-yl]-methyl, 4-Acetylamino-2-methylphenyl, 5-Acetylamino-2-methylphenyl, 4-Acetylamino-2, 6-dimethylphenyl, 4-Chlorophenyl, 3-Acetylphenyl, 4-Trifluoromethyl-phenyl, 2, 5-Difluorophenyl, 4-Isopropylphenyl, 4-Methoxyphenyl, 4-Ethoxyphenyl, 5-Chloro-2-methoxyphenyl, 2-Fluorophenyl, 1-Naphthyl, 2-Trifluoromethyl-phenyl, 2-Ethylphenyl, 2, 4-Dimethylphenyl, 2, 4, 5-Trimethylphenyl, 2, 5-Dimethylphenyl, 3-Chloro-2-methylphenyl, 4-Chloro-2-methylphenyl, 3-Trifluoromethyl-phenyl, 2, 6-dimethylphenyl, 2, 3-Dimethylphenyl, 4-Ethoxyphenyl, 3, 5-Dichlorophenyl, 3, 4-Dichlorophenyl, 3, 4-Dimethoxyphenyl, 4-Bromophenyl, 2-Chlorophenyl, 2-Methylphenyl, 2-Methoxyphenyl, 2, 6-Diethylphenyl, 2, 6-Diisopropylphenyl, 2-Biphenylyl, 2-Naphthyl, 4-Methylphenyl, 2, 4-Dimethoxyphenyl, 2, 5-Dimethylphenyl, 2-Chloro-4-methoxyphenyl, 2-Methoxy-5-methylphenyl, 3-Methoxy-4-methylphenyl, 2-Methoxyphenyl, 3-Hydroxyphenyl, 2-Chloro-5-trifluoromethyl-phenyl, 1-Acetylamino-2-naphthyl, 4-Ethylphenyl, 2, 5-Dichlorophenyl, 4-Trifluoromethoxyphenyl, 2 Chlorophenyl, 2-Methyl-1-naphthyl-, 1, 3-Dihydro-1-oxobenzo[c]furan-6-yl, Neopentyl, Isopropyl, 3-Nitro-benzyl, 2-Ethoxy-benzyl, 2, 2, 2-Trifluoroethyl, 2-(3-Trifluoromethyl-phenyl)ethyl, 1-Indanyl, 2-Phenyl-ethyl, (2-Naphthyl)-methyl, 2-Phenylethyl, Phenyl-CH(COOH)-, 3-Phenyl-benzyl-, 4-(4-Chlorophenoxy)-phenyl, 5-Chloro-2-methylphenyl-, 2-Fluorophenyl, 1-Naphthyl-;
R(3)
   Wasserstoff;
R(4)
   Wasserstoff;
R(5)
   Wasserstoff, Chloro, -SO₂CH₃, (2-Methoxy)ethoxy-;
R(6)
   Wasserstoff;
R(7)
   Wasserstoff, Methyl-, Chloro;
X
   -CO-, -CO-CO-, -CO-NH-, -SO₂-;
sowie deren physiologisch verträglichen Salze.

Können Gruppen oder Substituenten mehrfach in den Verbindungen der Formel I vorkommen, so können sie alle unabhängig voneinander die angegebenen Bedeutungen haben und können jeweils gleich oder verschieden sein.

Sowohl Alkyl, Alkenyl als auch Alkinyl können unabhängig voneinander geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind.

Beipiele für Alkylreste mit 1, 2, 3, 4, 5, 6, 7 oder 8 C-Atomen sind: Methyl, Ethyl, Propyl, Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl.

Beispiele für Alkenylreste sind Vinyl, 1-Propenyl, 2-Propenyl (Allyl), Butenyl, 3-Methyl-2-butenyl, 2-Butenyl, 2-Methyl-2-propenyl. Die Alkenylreste können auch zwei oder mehrere Doppelbindungen enthalten, wie zum Beispiel Butadienyl oder (CH₃)₂C=CH-CH₂-CH₂-C(CH₃)=CH-CH₂-.

Beispiele für Alkinylreste sind Ethinyl, der 2-Propinyl (Propargyl oder der 3-Butinyl. Die Alkinylreste können auch zwei oder mehrere Dreifachbindungen enthalten.

Cycloalkyl umfasst gesättigte und teilweise ungesättigte Cycloalkylreste, die mono-, bi- oder auch tricyclisch sein können. Beispiele für solche Cycloalkylreste sind Cyclopropyl, Cyclobutyl, Cyclopentyl, Cyclohexyl, Cycloheptyl, 1,2,3,4-Tetrahydronaphthalin und Indanyl, die alle auch zum Beispiel durch einen oder mehrere gleiche oder verschiedene (C₁-C₄)-Alkylreste, insbesondere durch Methyl, substituiert sein können. Beispiele für solche substituierten Cycloalkylreste sind 4-Methylcyclohexyl, 4-tert-Butylcyclohexyl oder 2,3-Dimethylcyclopentyl.

Arylgruppen mit 6, 7, 8, 9, 10, 11, 12, 13 oder 14 C-Atomen sind beispielsweise Phenyl, Naphthyl, Biphenyl, Antryl oder Fluorenyl, wobei 1-Naphthyl, 2-Naphthyl und insbesondere Phenyl bevorzugt sind.

Heteroarylreste und Heterocyclylreste leiten sich bevorzugt von Heterocyclen ab, die ein, zwei, drei oder vier gleiche oder verschiedene Ring-Heteroatome enthalten, besonders bevorzugt von Heterocyclen, die ein oder zwei oder drei, insbesondere ein oder zwei, gleiche oder verschiedene Heteroatome enthalten. Soweit nicht anders angegeben, können die Heterocyclen monocyclisch oder polycyclisch sein, zum Beispiel monocyclisch, bicyclisch oder tricyclisch. Bevorzugt sind sie monocyclisch oder bicyclisch. Die Ringe sind bevorzugt 5-Ringe, 6-Ringe oder 7-Ringe. Beispiele für monocyclische und bicyclische heterocyclische Systeme, von denen sich in den Verbindungen der Formel I vorkommende Reste ableiten können, sind Pyrrol, Furan, Thiophen, Imidazol, Pyrazol, 1,2,3-Triazol, 1,2,4-Triazol, 1,3-Dioxol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin, 1,3-Thiazepin, Indol, Benzothiophen, Benzofuran, Benzothiazol, Benzimidazol, Chinolin, Isochinolin, Cinnolin, Chinazolin, Chinoxalin, Phthalazin, Thienothiophene, 1,8-Naphthyridin und andere Naphthyridine, Pteridin, oder Phenothiazin, alle jeweils in gesättigter Form (Perhydro-Form) oder in teilweise ungesättigter Form (zum Beispiel Dihydro-Form und Tetrahydro-Form) oder in maximal ungesättigter Form, soweit die betreffenden Formen bekannt und stabil sind. Zu den in Betracht kommenden Heterocyclen gehören somit beispielsweise auch die gesättigten Heterocyclen Pyrrolidin, Piperidin, Piperazin, Morpholin und Thiomorpholin. Ungesättigte Heterocyclen können zum Beispiel eine, zwei oder drei Doppelbindungen im Ringsystem enthalten. 5-Ringe und 6-Ringe in monocyclischen und polycyclischen Heterocyclen können insbesondere auch aromatisch sein.

Die von diesen Heterocyclen abgeleiteten Reste können über jedes geeignete Kohlenstoffatom gebunden sein. Stickstoffheterocyclen, die an einem RingStickstoffatom ein Wasserstoffatom oder einen Substituenten tragen, zum Beispiel Pyrrol, Imidazol, Pyrrolidin, Morpholin, Piperazin etc., können auch über ein RingStickstoffatom gebunden sein, insbesondere, wenn der betreffende Stickstoffheterocyclus an ein Kohlenstoffatom gebunden ist. Beispielsweise kann ein Thienylrest als 2-Thienylrest oder 3-Thienylrest vorliegen, ein Furanrest als 2-Furylrest oder 3-Furylrest, ein Pyridylrest als 2-Pyridylrest, 3-Pyridylrest oder 4-Pyridylrest, ein Piperidinrest als 1-Piperidylrest, 2-Piperidylrest, 3-Piperidylrest oder 4-Piperidylrest, ein Thiomorpholinrest als 2-Thiomorpholinylrest, 3-Thiomorpholinylrest oder 4-Thiomorpholinylrest (= Thiomorpholinorest). Ein über ein Kohlenstoffatom gebundener Rest, der sich vom 1,3-Thiazol oder vom Imidazol ableitet, kann über die 2-Position, die 4-Position oder die 5-Position gebunden sein.

Soweit nicht anders angegeben, können die heterocyclischen Gruppen unsubstituiert sein oder einen oder mehrere, zum Beispiel einen, zwei, drei oder vier, gleiche oder verschiedene Substituenten tragen. Die Substituenten in Heterocyclen können sich in beliebigen Positionen befinden, beispielsweise in einem 2-Thienylrest oder 2-Furylrest in der 3-Position und/oder in der 4-Position und/oder in der 5-Position, in einem 3-Thienylrest oder 3-Furylrest in der 2-Position und/oder in der 4-Position und/oder in der 5-Position, in einem 2-Pyridylrest in der 3-Position und/oder in der 4-Position und/oder in der 5-Position und/oder in der 6-Position, in einem 3-Pyridylrest in der 2-Position und/oder in der 4-Position und/oder in der 5-Position und/oder in der 6-Position, in einem 4-Pyridylrest in der 2-Position und/oder in der 3-Position und/oder in der 5-Position und/oder in der 6-Position. Soweit nicht anders angegeben, können als Substituenten zum Beispiel die in der Definition der Gruppe Aryl angegebenen Substituenten auftreten, im Falle von gesättigten oder teilweise ungesättigten Heterocyclen als weiterere Substituenten auch die Oxogruppe und die Thioxogruppe. Substituenten an einem Heterocyclus wie auch Substituenten an einem Carbocyclus können auch einen Ring bilden, es können also an ein Ringsystem weitere Ringe ankondensiert sein, so daß zum Beispiel cyclopenta-kondensierte, cyclohexa-kondensierte oder benzo-kondensierte Ringe vorliegen können. Als Substituenten an einem substituierbaren Stickstoffatom eines Heterocyclus kommen insbesondere zum Beispiel unsubstituierte (C₁-C₅)-Alkylreste und arylsubstituierte Alkylreste, Arylreste, Acylreste wie CO-(C₁-C₅)-Alkyl, oder Sulfonylreste wie SO₂-(C₁-C₅)-Alkyl in Betracht. Geeignete Stickstoffheterocyclen können auch als N-Oxide oder als Quartärsalze mit einem von einer physiologisch verträglichen Säure abgeleiteten Anion als Gegenion vorliegen. Pyridylreste können zum Beispiel als Pyridin-N-oxide vorliegen.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Die Heteroarylreste können auch ganz oder teilweise hydriert sein. Beispielsweise seien Pyrrolidine-1-carbonyl-4,5 dihydro-isoxazolyl, 1,3-Dihydro-1-oxo-Benzo[c]furanyl oder 3,4-Dihydroquinazolinyl genannt.

Phenylreste, Naphthylreste und heterocyclische Reste, zum Beispiel Heteroarylreste, können, soweit nicht anders angegeben, unsubstituiert sein oder einen oder mehrere, zum Beispiel ein, zwei, drei oder vier, gleiche oder verschiedene Substituenten tragen, die sich in beliebigen Positionen befinden können. Soweit nicht anders angegeben, können in diesen Resten als Substituenten zum Beispiel die in der Definition der Gruppe Aryl angegebenen Substituenten auftreten. Sind zum Beispiel in Arylresten wie zum Beipiel Phenylresten und/oder in heterocyclischen Resten Phenylreste, Phenoxyreste, Benzylreste oder Benzyloxyreste als Substituenten vorhanden, so kann in diesen der Benzolring auch wiederum unsubstituiert sein oder durch einen oder mehrere, zum Beispiel ein, zwei, drei oder vier, gleiche oder verschiedene Reste substituiert sein, zum Beispiel durch Reste aus der Reihe (C₁-C₄)-Alkyl, Halogen, Hydroxy, (C₁-C₄)-Alkoxy, Trifluormethyl, Cyan, Hydroxycarbonyl, ((C₁-C₄)-Alkoxy)carbonyl, Aminocarbonyl, Nitro, Amino, (C₁-C₄)-Alkylamino, Di-((C₁-C₄)-alkyl)amino und ((C₁-C₄)-Alkyl)carbonylamino.

In monosubstituierten Phenylresten kann sich der Substituent in der 2-Position, der 3-Position oder der 4-Position befinden, in disubstituierten Phenylresten können sich die Substituenten in 2,3-Position, 2,4-Position, 2,5-Position, 2,6-Position, 3,4-Position oder 3,5-Position befinden. In trisubstituierten Phenylresten können sich die Substituenten in 2,3,4-Position, 2,3,5-Position, 2,3,6-Position, 2,4,5-Position, 2,4,6-Position oder 3,4,5-Position befinden. Tolyl (= Methylphenyl) ist 2-Tolyl, 3-Tolyl oder 4-Tolyl. Naphthyl kann 1-Naphthyl oder 2-Naphthyl sein. In monosubstituierten 1-Naphthylresten kann sich der Substituent in der 2-Position, der 3-Position, der 4-Position, der 5-Position, der 6-Position, der 7-Position oder der 8-Position befinden, in monosubstituierten 2-Naphthylresten in der 1-Position, der 3-Position, der 4-Position, der 5-Position, der 6-Position, der 7-Position oder der 8-Position.

Halogen bedeutet Fluor, Chlor, Brom oder lod, vorzugsweise Fluor oder Chlor.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine (Zwitterionen) zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen. Unter physiologisch verträglichen Salzen von Verbindungen der Formel (I) werden beispielsweise deren organische als auch anorganische Salze, wie sie in Remington's Pharmaceutical Sciences (17. Auflage, Seite 1418 (1985)) beschrieben sind, verstanden. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung umfaßt weiterhin alle Solvate von Verbindungen der Formel I, zum Beispiel Hydrate oder Addukte mit Alkoholen, sowie Derivate der Verbindungen der Formel I wie zum Beispiel Ester, und Pro-Drugs und aktive Metabolite.

Die Erfindung betrifft auch Verfahren zur Herstellung der neuen Verbindungen der Formel (I), sowie deren physiologisch verträglichen Salze.

So können die Verbindungen der Formel I beispielsweise mittels Festphasensynthese hergestellt werden.

Die Synthese erfolgt hierbei allgemein durch eine geeignete Anbindung der Benzolsulfonylstrukturen der allgemeinen Formel (II) über ein chemisches Bindeglied (Linker) an eine polymere Matrix nach den dem Fachmann bekannten Methoden der Sulfonamid-Synthese aus Sulfonsäurechlorid und Amin. Als polymere Matrix eignen sich z.B. Polystyrol, Polytetrafluorethylen, Polyacrylamid, etc., die gegebenenfalls mit Polyoxyethylenketten (Spacer) zur Verbesserung der Quellbarkeit verlängert sein können. Als Linker-Einheit eignen sich Strukturen, die spezifisch durch Säure, Base, Reduktion, Oxidation, durch Licht oder mit Fluoridionen die synthetisierten Verbindungen freisetzen, wobei die Linker-Einheit an der polymeren Matrix verbleibt (für eine Übersicht über Linkergruppen und Polymere in der Festphasensynthese siehe J. Früchtel, G. Jung, Angew. Chemie Int. Ed. 1996, 35, 17-42).

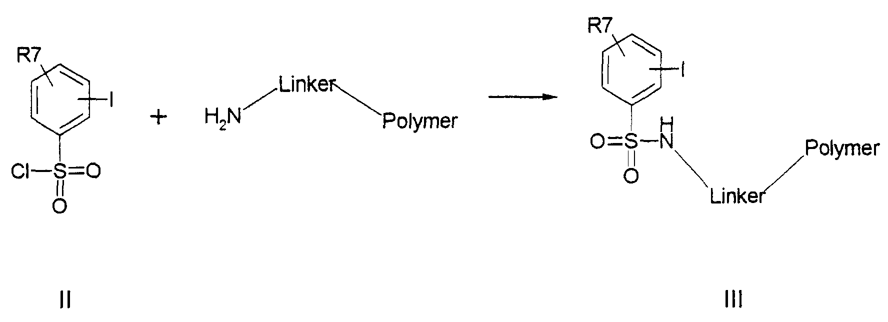

Die solchermaßen über die Sulfonamidgruppe an das Polymer geknüpften Grundkörper der allgemeinen Formel (III) können mit Arylboronsäurederivaten der allgemeinen Formel (IV) zu Biphenylderivaten der allgemeinen Formel (V) umgesetzt werden. Hierzu werden die literaturbekannten Palladium-katalysierten Reaktionsbedingungen gewählt, wie sie beispielsweise in Organometallics 1984, 3, 1261 oder in Synth. Commun. 11 (7), 513 (1981) beschrieben sind.

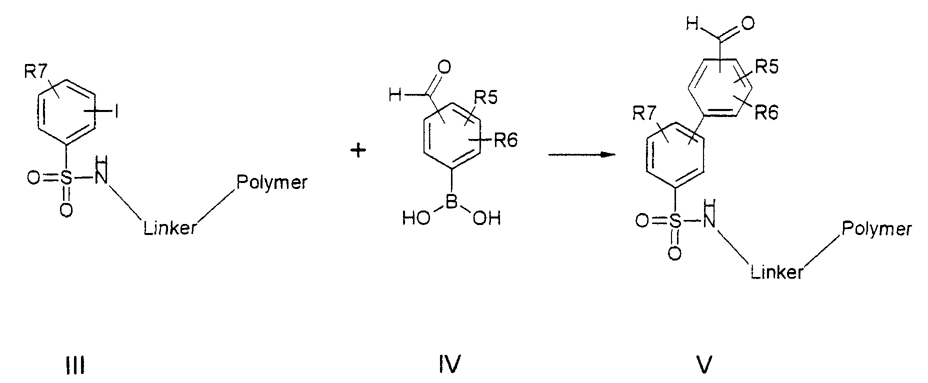

Die Synthese der Benzolboronsäure der allgemeinen Formel (IV) erfolgt beispielsweise analog der Synthese der 4-Formyl-benzolboronsäure, wie in Liebigs Ann. 1995, 1253 beschrieben.

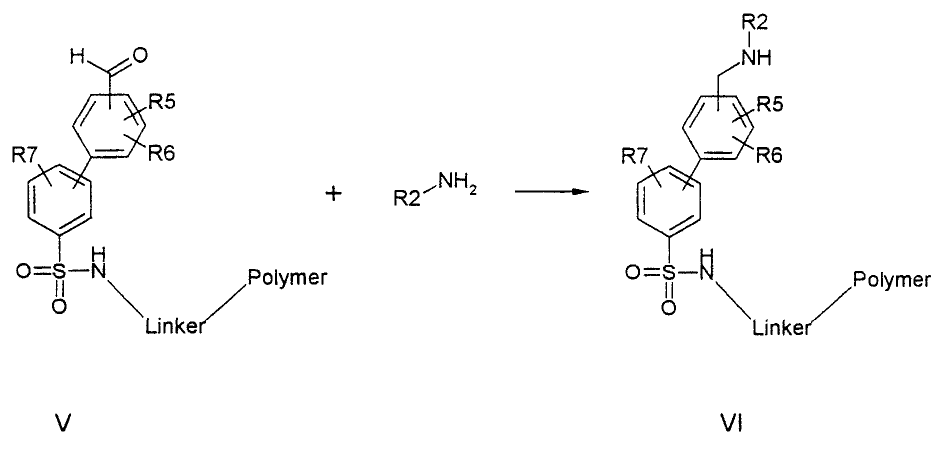

Reduktive Aminierung mit NaBH₃CN (Review über NaBH₃CN in Synthesis 1975, 135) liefert den Baustein (VI), der im Anschluß mit einem Säurechlorid R¹-X-Cl zum Baustein der allgemeinen Formel (VII) umgesetzt werden kann. Die Synthese an der festen Phase hat dabei den Vorteil, daß Reagenzien und Reaktanten im großen Überschuß angewendet werden können, Lösungsmittel breit variiert werden können und eine Reinigung durch einfaches Waschen der Harzpartikel erfolgt.

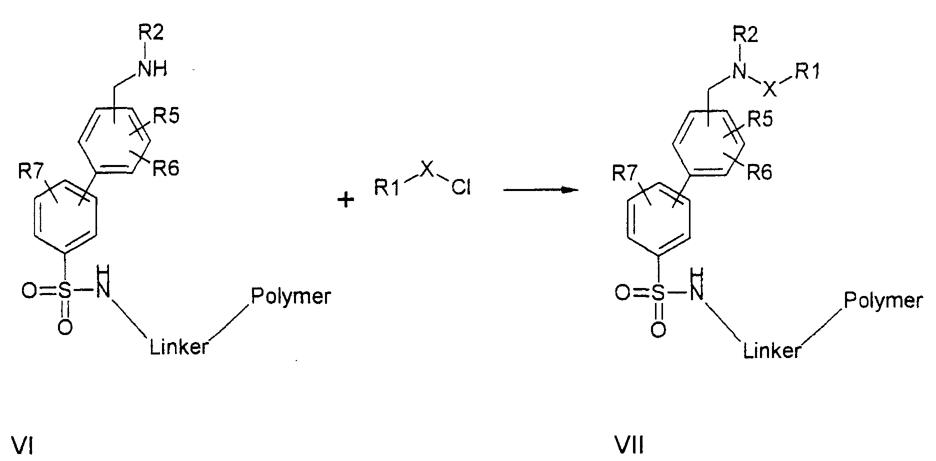

Im Falle der Reste R(3), R(4) ungleich Wasserstoff ist es nötig, die betreffenden Reste einzuführen. Dies geschieht entweder, indem man das bei der reduktiven Aminierung durchlaufene Imin der allgemeinen Formel (X) ohne Reduktionsmittel aus dem Aldehyd (V) und dem entsprechenden Amin synthetisiert und mit einer metallorganischen, den Rest R(3) oder den Rest R(4) tragenden Verbindung wie zum Beispiel einer Grignard-Verbindung oder einer Alkyllithium-Verbindung in der dem Fachmann bekannten Art umsetzt. Oder die Aldehydfunktion der Verbindungen der allgemeinen Formel (V) wird zum Nitril der allgemeinen Formel (XI) oxydiert, wie es zum Beispiel in Synthesis 1982, 190 beschrieben ist und anschließend nacheinander mit einer lithium-oder einer magnesiumorganischen Verbindung in einer dem Fachmann bekannten Weise die Reste R(3) und R(4) eingeführt. In letzterem Fall muß außerdem noch in dem Fachmann bekannter Weise über eine Amin-Arylierung oder -Alkylierung der Rest R(2) eingeführt werden.

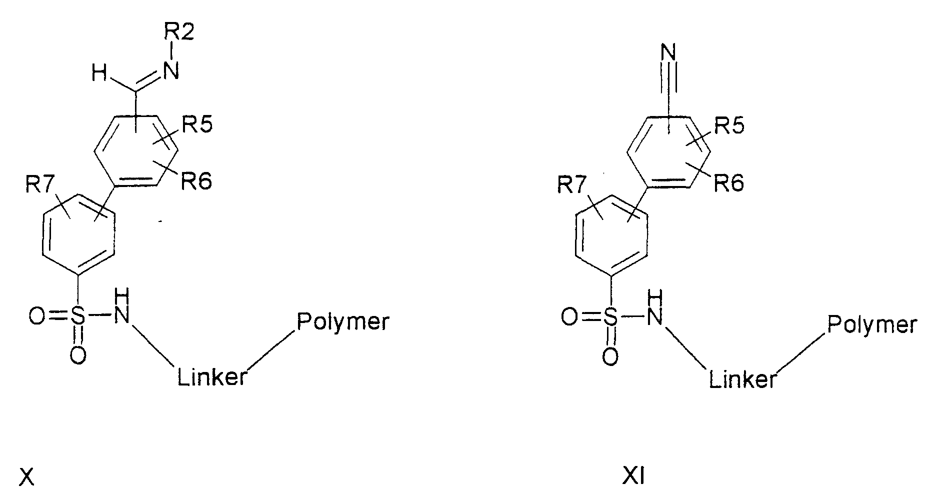

Nach sequentieller Durchführung der Synthesestufen erfolgt eine Abspaltung der neu synthetisierten Verbindungen mit Hilfe spezifischer Reagenzien, entsprechend der Linkerauswahl (für eine Beschreibung der Festphasensynthese siehe: J. Früchtel, G. Jung, Angew. Chemie Int. Ed. 1996, 35, 17-42). Die Abspaltung vom Harz erfolgt abgestimmt für den verwendeten Linker in einer dem Fachmann bekannten Weise. Als bevorzugtes Polymer ist beispielsweise Aminomethylpolystyrol der Firma Fluka geeignet (1.1 mmol Amin/g Harz; 2% crosslinked DVB). Als bevorzugter Linker ist beispielsweise die literaturbekannte Verbindung (VIII) 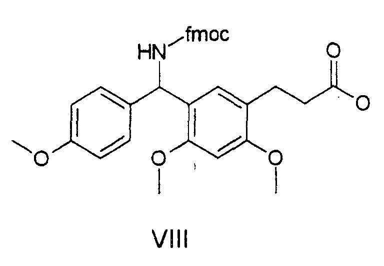 geeignet. (G. Breipohl, J. Knolle, W. Stüber, Int. J. Peptide Protein Res. 34, 1989, 262f). In diesem Fall erfolgt die Abspaltung vom Harz unter sauren Bedingungen. Die Verbindungen der allgemeinen Formel (VII) werden hierzu in einem inerten Lösungsmittel, bevorzugt CH₂Cl₂ mit einer Säure mit einem pKₐ < 5, bevorzugt ist eine Säure mit einem pKₐ < 2, besonders bevorzugt ist Trifluoressigsäure, behandelt und man erhält die Sulfonamide der allgemeinen Formel (IX). Typische Reaktionszeit ist 5 Minuten bis 10 Stunden bei einer Temperatur zwischen -30°C und dem Siedepunkt des Lösungsmittels, bevorzugt ist eine Reaktionszeit zwischen 20 Minuten und einer Stunde bei Raumtemperatur.

Der letzte Syntheseschritt ist dadurch gekennzeichnet, daß man Verbindungen der Formel (IX) 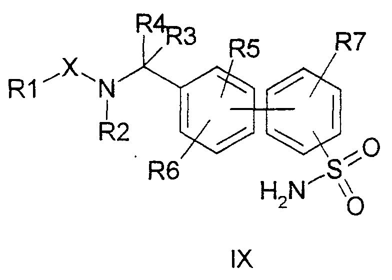 worin die Reste wie oben definiert sind mit Bromcyan zu den Titelverbindungen der allgemeinen Formel (I) umsetzt. Die Reaktion erfolgt in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur zwischen 0 °C und dem Siedepunkt des verwendeten Lösungsmittels in Frage, bevorzugt ist eine Temperatur zwischen 40 °C und 100 °C.

Die Verbindungen der Formel I können auch durch klassische Synthese, d.h. in Lösung, nach dem Fachmann bekannten Methoden synthetisiert werden.

Die erfindungsgemäßen Verbindungen der Formel I eignen sich als Inhibitoren des Natrium-abhängigen Bicarbonat/Chlorid-Austauschers (NCBE) bzw. des Natrium/Bicarbonat-Symporters.

In EP-A 855392 werden Imidazol-Derivate mit einer Biphenylsulfonylcyanamid-Seitenkette als NCBE-Inhibitoren beschrieben.

In der europäischen Patentanmeldung 98117529.2 sind Biphenylsulfonylcyanamide als NCBE-Inhibitoren vorgeschlagen worden, die sich in der Substitution am Biphenylringsystem von den Verbindungen der Formel I unterscheiden.

Des weiteren betrifft die Erfindung die Verwendung einer Verbindung der Formel I zur Herstellung eines Medikaments zur Behandlung oder Prophylaxe von durch ischämische Zustände hervorgerufenen Krankheiten;
sowie die Verwendung einer Verbindung der Formel I zur Herstellung eines Medikaments zur Behandlung oder Prophylaxe des Herzinfarkts;
sowie die Verwendung einer Verbindung der Formel I zur Herstellung eines Medikaments zur Behandlung oder Prophylaxe der Angina Pectoris;
sowie die Verwendung einer Verbindung der Formel I zur Herstellung eines Medikaments zur Behandlung oder Prophylaxe von ischämischen Zuständen des Herzens;
sowie die Verwendung einer Verbindung der Formel I zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I zur Herstellung eines Medikaments zur Behandlung oder Prophylaxe von ischämischen Zuständen peripherer Organe und Gliedmaßen;
sowie die Verwendung einer Verbindung der Formel I zur Herstellung eines Medikaments zur Behandlung von Schockzuständen;
sowie die Verwendung einer Verbindung der Formel I zur Herstellung eines Medikaments zum Einsatz bei chirurgischen Operationen und Organtransplantationen;
sowie die Verwendung einer Verbindung der Formel I zur Herstellung eines Medikaments zur Konservierung und Lagerung von Transplantaten für chirurgische Maßnahmen;
sowie die Verwendung einer Verbindung der Formel I zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I zur Herstellung eines Medikaments zur Behandlung von gestörtem Atemantrieb;
sowie ein Arzneimittel, gekennzeichnet durch einen wirksamen Gehalt einer Verbindung der Formel I.

Die erfindungsgemäßen Verbindungen der Formel I zeigen sehr gute antiarrhythmische Eigenschaften, wie sie beispielsweise für die Behandlung von Krankheiten wichtig sind, welche bei Sauerstoffmangelerscheinungen auftreten.

Die Verbindungen I sind wegen ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.

Wegen ihrer schützenden Wirkungen gegen pathologische hypoxische und ischämische Situationen können die erfindungsgemäßen Verbindungen der Formel I infolge Inhibition des zellulären Na⁺-abhängigen Cl⁻/HCO₃⁻-Austauschmechanismus bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie schützen akut oder chronisch sauerstoffmangelversorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei Organtransplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen.

Die Verbindungen der Formel I sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüber hinaus eignen sich die erfindungsgemäßen Verbindungen der Formel I ebenfalls zur Behandlung von Formen des Schocks, wie beispielsweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Es wurde gefunden, daß Inhibitoren des Na⁺-abhängigen Cl⁻/HCO₃⁻-Austauschers bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren.

Eine Verbesserung des Atemantriebes durch Stimulation der Chemorezeptoren mit Substanzen, die den Na⁺-abhängigen Cl⁻/HCO₃⁻ Austausch hemmen, führt zu einer Verbesserung der Atmung bei folgenden klinischen Zuständen und Krankheiten: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Die erfindungsgemäßen Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Herstellung von Medikamenten dafür. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen pharmazeutisch einwandfreien Träger- und Hilfsstoffen enthalten. Die pharmazeutischen Zubereitungen enthalten normalerweise 0,1 bis 99 Gewichtsprozent, bevorzugt 0,5 bis 95 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die eine Verbindung der Formel (I) und/oder ihre physiologisch verträglichen Salze enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpafienten auf der Intensivstation können bis zu 200 mg pro Tag notwendig werden.

Die Verbindungen der Formel I können als einzige Wirkstoffe oder in Kombination mit anderen pharmakologisch wirksamen Verbindungen eingesetzt werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft desweiteren die Kombination von a) NCBE-Inhibitoren der Formel I und/oder deren physiologisch verträglichen Salze mit NHE-Inhibitoren und/oder deren physiologisch verträglichen Salze; b) NCBE-Inhibitoren der Formel I und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren der Formel I und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., "DIURETICS, Chemistry, Pharmacology and Medicine", J. WILEY & Sons (1983), 303 - 341 beschrieben sind oder um die in EP98115754.8 aufgeführten NHE-Inhibitoren.
Geeignete NHE-Inhibitoren sind beispielsweise auch Benzoylguanidine, wie sie in US 5292755, US 5373024, US 5364868, US 5591754, US 5516805, US 5559153,US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, EP-A 814077, EP-A 869116 beschrieben sind; ortho-substituierte Benzoylguanidine, wie sie in EP-A 556673, EP-A 791577, EP-A 794172 beschrieben sind; ortho-amino-substituierte Benzoylguanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkyl- bzw. Alkenyl-carbonsäure-Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind; Indenoylguanidine, wie sie in EP-A 738712 beschrieben sind; Benzyloxycarbonyl-guanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäurediguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäure-guanidine, wie sie in EP-A 790245 beschrieben sind; Bis-ortho-substituierte Benzoylguanidine, wie sie in EP-A 810207 beschrieben sind; Substituierte 1- oder 2-Naphthylguanidine, wie sie in EP-A 810205 und EP-A 810206 beschrieben sind; Indanylidinacetylguanidine, wie sie in EP-A 837055 beschrieben sind; Phenylsubstituierte Alkenylcarbonsäure-guanidine, wie sie in EP-A 825178 beschrieben sind; Aminopiperidyl-Benzoylguanidine, wie sie in EP-A 667341 beschrieben sind; Heterocycloxy-Benzylguanidine, wie sie in EP-A 694537 beschrieben sind; ortho-substituierte Benzoylguanidine, wie sie in EP704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie in EP-A 708088 beschrieben sind; ortho-substituierte 5-Alkylsulfonyl-benzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, Polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyloder 3-Dihalophenyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonyl-benzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoyiguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie JP 9067332 beschrieben sind; Propenoylguanidine mit Indolsubstituenten, wie sie JP 9067340 beschrieben sind; oder Heteroaryl-substituierte Acryloylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am meisten bevorzugt ist Cariporid oder ein anderes physiologisch verträgliches Salz des N-(4-lsopropyl-3-methansulfonyl-benzoyl)-guanidin.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridinaktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.

Ein Beispiel eines solchen besonders vorteilhaften Kombinationspartners mit NCBE-Inhibitoren der Formel I sind Blocker des nicht-inaktivierenden-Natriumkanals (Veratridin-aktivierbarer Natriumkanal). Die Kombinationen eines NCBE-Inhibitors mit einem Blocker des nicht-inaktivierenden-Natriumkanals (Veratridin-aktivierbarer Natriumkanal) eignet sich zur Infarkt- und Reinfarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors mit einem Blocker des nichtinaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors der Formel I mit einem Blocker des nichtinaktivierenden-Natriumkanals infolge verstärkter Inhibierung des Na⁺ Einstroms in die Zelle als Arzneimittel zur Behandlung aller akuten oder chronischen, durch Ischämie ausgelösten Schäden oder dadurch primär oder sekundär induzierter Krankheiten verwendet werden. Dies betrifft ihre Verwendung als Arzneimittel für operative Eingriffe, z. B. bei Organtransplantationen, wobei die Kombinationen eines NCBE-Inhibitors der Formel I mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors der Formel I mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors der Formel I mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NCBE-Inhibitors der Formel I mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren der Formel I mit NHE-Inhibitoren und/oder einer zusätzlichen aktiven Substanzen aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf-Wirkstoffklasse, und/oder deren physiologisch verträglichen Salze.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf-Wirkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichstverhältnis vom NCBE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100, vorzugsweise 1:0,1 bis 1:10.

Die erfindungsgemäßen Kombination und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschieden Wirkstoffkomponenten im Berreich von 0,001 bis 100 mg/kg/Tag.

### Liste der Abkürzungen:

- BCECF: 2',7'-Bis(2-carboxyethyl)-5,6-carboxyfluorescein
- Bn: Benzyl
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- El: electron impact
- eq: Äquivalent
- ES: Elektrospray-lonisation
- ESneg: Elektrospray, negative Ionisation
- Et: Ethyl
- EtOH: Ethanol
- FAB: Fast Atom Bombardment
- fmoc: 9-Fluorenylmethoxycarbony
- HEP: n-Heptan
- HOAc: Essigsäure
- HOOBt: 3-Hydroxy-1,2,3-benzotriazin-4(3H)-on
- KOtBu: Kalium-*t*-butylat
- Me: Methyl
- MeOH: Methanol
- mp: Schmelzpunkt
- MTB: Methyltertiärbutylether
- NCBE: Natriumabhängiger Chlorid/Bicarbonat-Austauscher
- NHE E: Natrium/Wasserstoff-Austauscher
- NMP: N-Methylpyrrolidon
- PS: Polystyrol
- RT: Raumtemperatur
- THF: Tetrahydrofuran
- TMU: N,N,N',N'-Tetramethylharnstoff
- Tol: Toluol
- ZNS: Zentralnervensystem

### Beispiele:

### Allgemeine Synthesevorschrift für Festphasensynthese:

Die Synthese der Verbindungen der Formel I an fester Phase erfolgt durch eine geeignete Anbindung der Sulfonamidstrukturen über ein chemisches Bindeglied (Linker) an eine polymere Matrix nach dem Fachmann bekannten Methoden. Die solchermaßen über die Sulfonamidgruppe an das Polymer geknüften Grundkörper lassen sich weiteren üblichen organischen Reaktionen der organischen Chemie zuführen.

Im einzelnen werden folgende Schritte durchlaufen (beispielhaft beschrieben für eine Verbindung der Formel la, 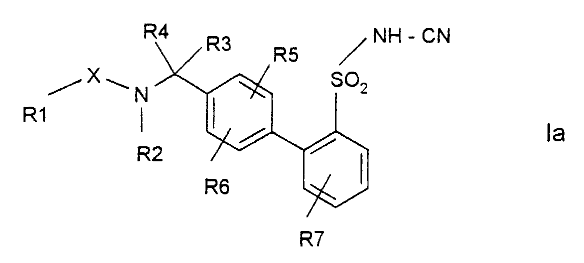 mit R(1) gleich 2-Chlorophenyl, R(2) gleich Benzyl, X gleich Carbonyl und R(3) bis R(7) gleich Wasserstoff.

### A. Synthese der Linker/Polymer-Einheit

Als käufliches Polymer wurde Aminomethylpolystyrol der Firma Fluka verwendet (1.1 mmol Amin/g Harz; 2% crosslinked DVB). Als Linker wurde die literaturbekannte Verbindung 1 verwendet (G. Breipohl, J. Knolle, W. Stüber, Int. J Peptide Protein Res. 34, 1989, 262f).

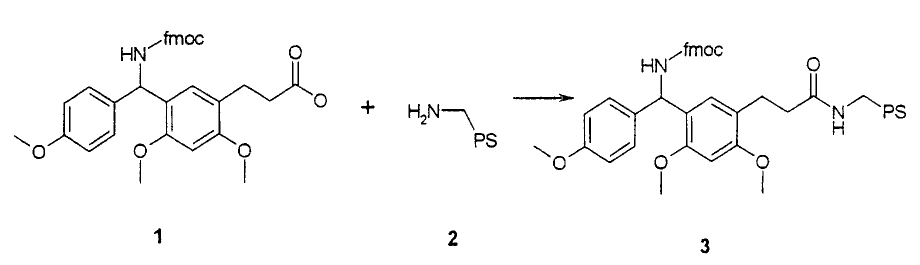

Zur Verknüpfung von Polymer und Linker wurden 14.4 g Aminomethylpolystyrol 2, 28.2 g 1, 3.2 g HOOBt, 11.5 g Diisopropylcarbodiimid in 105 ml DMF und 45 ml Methylenchlorid zusammengebracht und über 48 h geschüttelt. Es wurde anschließend abgesaugt und mit DMF und MtB gründlich gewaschen.

### B. Synthese des Harz gebundenen Sulfonamids

5 g 3 wurde mit Piperidin/DMF (1:3) 30 min bei RT behandelt, die Lösung abgesaugt und gründlich mit DMF gewaschen. Zum DMF feuchten Harz wurde 3.1 g der literaturbekannten Verbindung 4 ( Gilman, Marker, JACS 74, 1952, 5317) in 20 ml DMF addiert. Nach Zugabe von 0.7 ml Pyridin ließ man 18 h bei RT reagieren. Es wurde abgesaugt und mit DMF und MtB gewaschen.

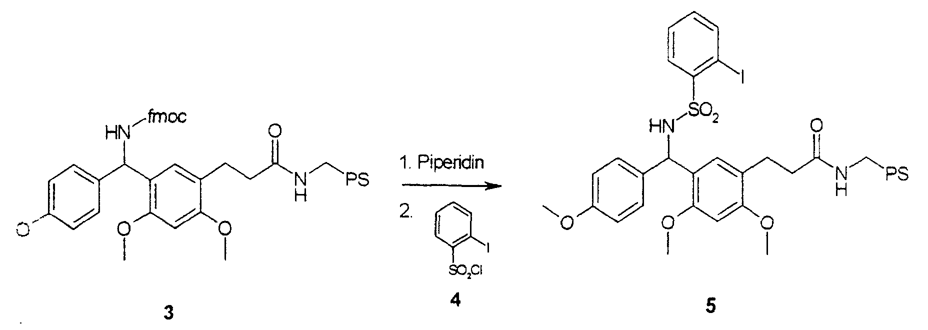

### C. Synthese des Harz gebundenen Biarylsulfonamids 7

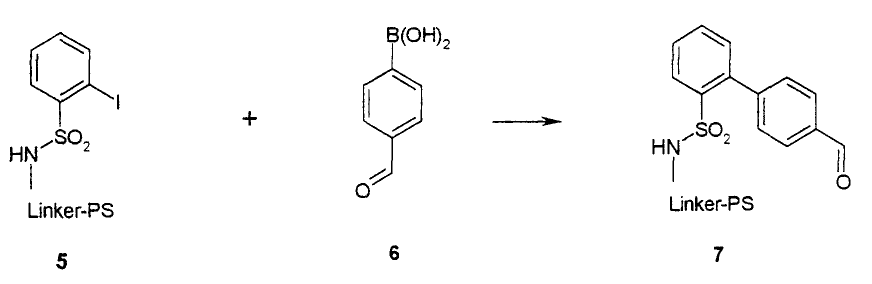

1 g 5 wurde in 15 ml DMF gequollen und mit 1.5 g der Boronsäure 6, 50 mg Pd(PPh₃)₄ und 3.5 ml 2 m Na₂CO₃- Lösung versetzt. Man ließ 24h bei 100 □C unter Argon Atmosphäre reagieren. Es wurde abgesaugt und gründlich mit Wasser, DMF und MtB gewaschen.

### D. Reduktive Aminierung an der festen Phase hier: beispielhaft Benzylamin

200 mg des Harz gebundenen Bausteins 7 wurden mit 1 mmol des Amins, gelöst in 1 ml Dimethoxyethan/Methanol (3:1), versetzt und 0.2 ml einer 1 m Essigsäure-Lösung in Dimethoxyethan sowie 0.5 ml einer 1 m NaBH₃CN Lösung in Dimethoxyethan zuaddiert. Man ließ 4h bei RT reagieren. Es wurde abgesaugt und mit DMF und MtB gründlich gewaschen.
Nach Abspaltung einer Probe (10mg) vom Harz durch Behandlung mit 1 ml Methylenchlorid/Trifluoressigsäure (3:1) wurde das freie Amin 8 durch HPLC und MS charakterisiert.

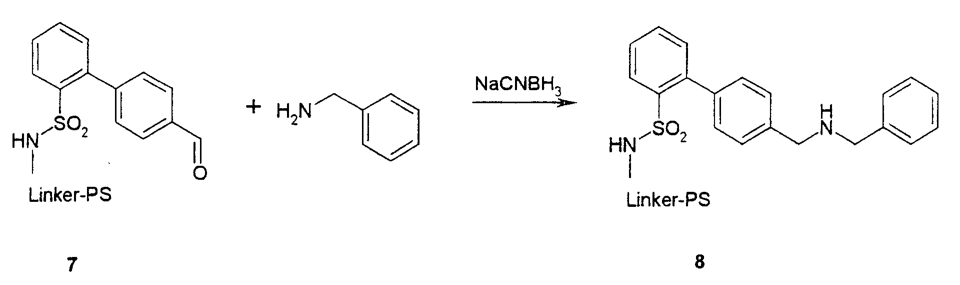

### E. N-Acylierung an der festen Phase

### hier: beispielhaft 2-Cl-Benzoylchlorid

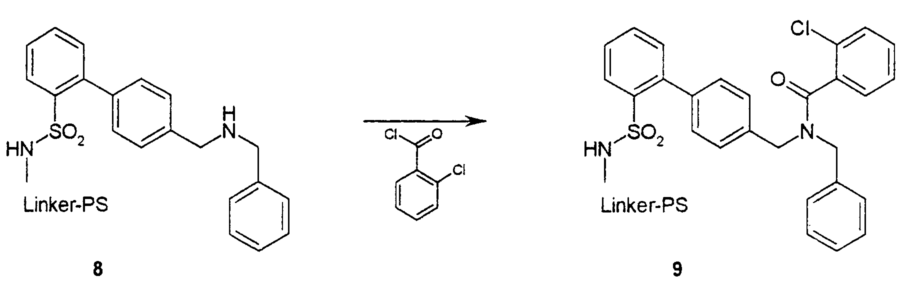

190 mg 8 wurden mit 2 ml einer 1 m Lösung von N-Ethylmorpholin in Methylenchlorid versetzt. Man kühlte auf 0□C und gab unter Rühren 0.5 mmol des Säurechlorids, gelöst in 0.5 ml Methylenchlorid, zu. Man ließ 1h bei 0□C reagieren. Es wurde abgesaugt und mit DMF und MtB gewaschen. Der Reaktionsumsatz wurde durch Abspaltung einer Probe 9 (siehe D) durch HPLC und MS kontrolliert.

### F. Abspaltung vom Harz

180 mg 9 wurden über 30 min bei RT mit 3 ml einer Lösung von Methylenchlorid/Trifluoressigsäure (3:1) behandelt. Nach Abtrennen der Lösung vom Polymer wurde im Vakuum einrotiert. Als Rückstand erhielt man 30 mg des freien Amids 10, das direkt der Cyanylierung (G) zugeführt wurde.

### G. Synthese des Sulfonylcyanamids

30 mg 10 wurde in 3 ml Acetonitril gelöst und mit 0.3 mmol Triethylamin sowie mit 0.12 mmol BrCN gelöst in Acetonitril versetzt. Man ließ 18h bei RT reagieren, versetzte dann mit 3 ml MtB und 2 ml einer wäßrigen Pufferlösung (pH6). Nach gutem Durchmischen wurde die Oberphase entnommen und auf Silicagel aufgetragen. Es wurde zuerst mit 5 ml MtB gewaschen, anschließend das Produkt mit 5 ml EE/HOAc (5:1) eluiert. Nach Einengen der Lösungsmittel im Vakuum erhielt man 12 mg des Sulfonylcyanamids in 60-90%iger Reinheit (beispielhaft 11, 90 %). Die so erhaltenen Produkte konnten durch präparative HPLC auf RP Materialien weiter gereinigt werden. Die Charakterisierung der Produkte erfolgte durch HPLC und MS sowie exemplarisch durch NMR-Spektroskopie.

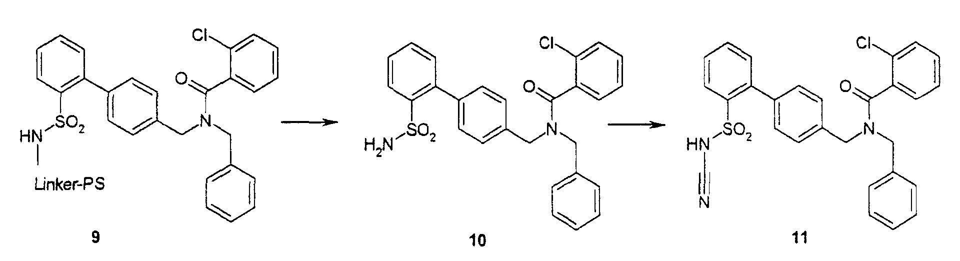

Die folgenden Verbindungen der Formel la werden analog hergestellt (R(3) bis R(7) gleich Wasserstoff).

| | R(2) | R(1)-X | MS (ES⁻):M-1)⁻ | Restaktivität% des NCBE bei 10 µM |
|---|---|---|---|---|
| 1 | -Benzyl | CH3-CH2-CH2-CO- | 446 | |
| | | | | |
| 2 | -CH2-CH2-OH | CH3-CH2-CH2-CO- | 400 | 78.1 |
| | | | | |
| 3 | 4-Methoxy-benzyl- | CH3-CH2-CH2-CO- | 476 | 80.9 |
| | | | | |
| 4 | 4-Chloro-benzyl- | CH3-CH2-CH2-CO- | 480 | |
| | | | | |
| 5 | -Phenyl | CH3-CH2-CH2-CO- | 431 | 89.5 |
| | | | | |
| 6 | -Cyclohexyl | CH3-CH2-CH2-CO- | 438 | 84.5 |
| | | | | |
| 7 | -Benzyl | Cyclohexyl-CH2-CH2-CO- | 514 | 33.0 |
| | | | | |
| 8 | -CH2-CH2-OH | Cyclohexyl-CH2-CH2-CO- | 468 | 86.7 |
| | | | | |
| 9 | 4-Methoxy-benzyl- | Cyclohexyl-CH2-CH2-CO- | 544 | 86.6 |
| | | | | |
| 10 | 4-Chloro-benzyl- | Cyclohexyl-CH2-CH2-CO- | 548 | 84.9 |
| | | | | |
| 11 | -Phenyl | Cyclohexyl-CH2-CH2-CO- | 499 | 68.1 |
| | | | | |
| 12 | -Cyclohexyl | Cyclohexyl-CH2-CH2-CO- | 506 | 92.9 |
| | | | | |
| 13 | -Benzyl | Cyclohexyl-CO- | 486 | 74.7 |
| | | | | |
| 14 | -CH2-CH2-OH | Cyclohexyl-CO- | 440 | 88.7 |
| | | | | |
| 15 | 4-Methoxy-benzyl- | Cyclohexyl-CO- | 516 | 67.6 |
| | | | | |
| 16 | 4-Chloro-benzyl- | Cyclohexyl-CO- | 520 | 56.4 |
| | | | | |
| 17 | -Phenyl | Cyclohexyl-CO- | 471 | |
| | | | | |
| 18 | -Cyclohexyl | Cyclohexyl-CO- | 478 | |
| | | | | |
| 19 | -Benzyl | 3-Methoxy-phenyl-CH2-CO- | 524 | 25.9 |
| | | | | |
| 20 | -CH2-CH2-OH | 3-Methoxy-phenyl-CH2-CO- | 478 | 37.1 |
| | | | | |
| 21 | 4-Methoxy-benzyl- | 3-Methoxy-phenyl-CH2-CO- | 554 | 52.3 |
| | | | | |
| 22 | 4-Chloro-benzyl- | 3-Methoxy-phenyl-CH2-CO- | 558 | |
| | | | | |
| 23 | -Phenyl | 3-Methoxy-phenyl-CH2-CO- | 509 | 25.9 |
| | | | | |
| 24 | -Cyclohexyl | 3-Methoxy-phenyl-CH2-CO- | 516 | 92.1 |
| | | | | |
| 25 | -Benzyl | 2-Thienyl-CH2-CO- | 500 | 16.6 |
| | | | | |
| 26 | -CH2-CH2-OH | 2-Thienyl-CH2-CO- | 454 | 47.6 |
| | | | | |
| 27 | 4-Methoxy-benzyl- | 2-Thienyl-CH2-CO- | 530 | 35.5 |
| | | | | |
| 28 | 4-chloro-benzyl- | 2-Thienyl-CH2-CO- | 534 | 95.2 |
| | | | | |
| 29 | -Phenyl | 2-Thienyl-CH2-CO- | 485 | 55.7 |
| | | | | |
| 30 | -Cyclohexyl | 2-Thienyl-CH2-CO- | 492 | 95.2 |
| | | | | |
| 31 | -Benzyl | 4-t-Butyl-phenyl-CO- | 536 | 52.2 |
| | | | | |
| 32 | -CH2-CH2-OH | 4-t-Butyl-phenyl-CO- | 490 | 68.0 |
| | | | | |
| 33 | 4-Methoxy-benzyl- | 4-t-Butyl-phenyl-CO- | 566 | 67.2 |
| | | | | |
| 34 | 4-Chloro-benzyl- | 4-t-Butyl-phenyl-CO- | 570 | |
| | | | | |
| 35 | -Phenyl | 4-t-Butyl-phenyl-CO- | 521 | 53.4 |
| | | | | |
| 36 | -Cyclohexyl | 4-t-Butyl-phenyl-CO- | 528 | |
| | | | | |
| 37 | -Benzyl | 2-Fluoro-phenyl-CO- | 498 | 52.0 |
| | | | | |
| 38 | -CH2-CH2-OH | 2-Fluoro-phenyl-CO- | 452 | 66.7 |
| | | | | |
| 39 | 4-Methoxy-benzyl- | 2-Fluoro-phenyl-CO- | 528 | 51.3 |
| | | | | |
| 40 | 4-Chloro-benzyl- | 2-Fluoro-phenyl-CO- | 532 | |
| | | | | |
| 41 | -Phenyl | 2-Fluoro-phenyl-CO- | 483 | 94.9 |
| | | | | |
| 42 | -Cyclohexyl | 2-Fluoro-phenyl-CO- | 490 | |
| | | | | |
| 43 | -Benzyl | Allyl-O-CO- | 460 | 41.6 |
| | | | | |
| 44 | -CH2-CH2-OH | Allyl-O-CO- | 414 | 82.6 |
| | | | | |
| 45 | 4-Methoxy-benzyl- | Allyl-O-CO- | 490 | 80.1 |
| | | | | |
| 46 | 4-Chloro-benzyl- | Allyl-O-CO- | 494 | 52.2 |
| | | | | |
| 47 | -Phenyl | Allyl-O-CO- | 445 | 88.1 |
| | | | | |
| 48 | -Cyclohexyl | Allyl-O-CO- | 452 | |
| | | | | |
| 49 | -Benzyl | 3,4,5-Trimethoxy-phenyl-CO- | 570 | 75.8 |
| | | | | |
| 50 | -CH2-CH2-OH | 3,4,5-Trimethoxy-phenyl-CO- | 524 | |
| | | | | |
| 51 | 4-Methoxy-benzyl- | 3,4,5-Trimethoxy-phenyl-CO- | 600 | |
| | | | | |
| 52 | 4-Chloro-benzyl- | 3,4,5-Trimethoxy-phenyl-CO- | 604 | 59.9 |
| | | | | |
| 53 | -Phenyl | 3,4,5-Trimethoxy-phenyl-CO- | 555 | |
| | | | | |
| 54 | -Cyclohexyl | 3,4,5-Trimethoxy-phenyl-CO- | 562 | |
| | | | | |
| 55 | -Benzyl | 2-Chloro-phenyl-CO- | 514 | 29.3 |
| | | | | |
| 56 | -CH2-CH2-OH | 2-Chloro-phenyl-CO- | 468 | 69.0 |
| | | | | |
| 57 | 4-Methoxy-benzyl- | 2-Chloro-phenyl-CO- | 544 | 82.1 |
| | | | | |
| 58 | 4-Chloro-benzyl- | 2-Chloro-phenyl-CO- | 548 | 59.4 |
| | | | | |
| 59 | -Phenyl | 2-Chloro-phenyl-CO- | 499 | |
| 60 | -Cyclohexyl | 2-Chloro-phenyl-CO- | 506 | |

| No. | R2 | R1-X | MS (ES-) (M-1)⁻ | Restaktivität (%) des NCBE bei 10 µM |
|---|---|---|---|---|
| 61 | Benzyl- | Phenyl-CO- | 480 | 56.6 |
| 62 | 4-Chloro-benzyl- | CH3-CO- | 452 | 94.5 |
| 63 | Phenyl- | CH3-CO- | 404 | |
| 64 | 4-Methoxy-benzyl- | E-CH3-CH=CH-CO- | 474 | 88.0 |
| 65 | 4-Chloro-benzyl- | CH3-(CH2)3-CO- | 494 | 80.0 |
| 66 | 4-Chloro-benzyl- | Phenyl-CH2-CH2-CO- | 542 | 83.1 |
| 67 | 4-Chloro-benzyl- | E-CH3-CH=CH-CO- | 478 | |
| 68 | 2-Hydroxy-ethyl- | 4-Methyl-phenyl-CO- | 448 | 94.4 |
| 69 | 4-Methoxy-benzyl- | CH3-CO- | 448 | 95.4 |
| 70 | 4-Methoxy-benzyl- | 4-Methyl-phenyl-CO- | 524 | 94.7 |
| 71 | 2-Chloro-benzyl- | Benzo[b]thiophen-2-yl-CO- | 570 | 35.2 |
| 72 | Isobutyl- | Benzo[b]thiophen-2-yl-CO- | 502 | 86.3 |
| 73 | 1(S)-Phenyl-ethyl- | Benzo[b]thiophen-2-yl-CO- | 550 | 61.0 |
| 74 | 2-Methoxy-benzyl- | Benzo[b]thiophen-2-yl-CO- | 566 | 54.7 |
| 75 | 4-Methyl-benzyl- | Benzo[b]thiophen-2-yl-CO- | 550 | 80.3 |
| 76 | 3-Methoxy-benzyl- | Benzo[b]thiophen-2-yl-CO- | 566 | 87.3 |
| 77 | 3,4-Methylenedioxy-benzyl- | Benzo[b]thiophen-2-yl-CO- | 580 | 81.8 |
| 78 | 1(R)-Phenyl-ethyl- | Benzo[b]thiophen-2-yl-CO- | 550 | 88.5 |
| 79 | 4-Trifluoromethyl-benzyl- | Benzo[b]thiophen-2-yl-CO- | 604 | 61.7 |
| 80 | 2-(4-Methoxyphenyl)-ethyl- | Benzo[b]thiophen-2-yl-CO- | 580 | 66.7 |
| 81 | 2-Chloro-benzyl- | Thien-2-yl-CO- | 520 | 36.0 |
| 82 | Isobutyi- | Thien-2-yl-CO- | 452 | 99.8 |
| 83 | 1(S)-Phenyl-ethyl- | Thien-2-yl-CO- | 500 | 97.5 |
| 84 | 2-Methoxy-benzyl- | Thien-2-yl-CO- | 516 | 54.7 |
| 85 | 4-Methyl-benzyl- | Thien-2-yl-CO- | 500 | 51.1 |
| 86 | 3-Methoxy-benzyl- | Thien-2-yl-CO- | 516 | 57.0 |
| 87 | 3,4-Methylenedioxy-benzyl- | Thien-2-yl-CO- | 530 | 54.8 |
| 88 | 1(R)-Phenyl-ethyl- | Thien-2-yl-CO- | 500 | |
| 89 | 4-Trifluoromethyl-benzyl- | Thien-2-yl-CO- | 554 | 67.6 |
| 90 | 2-(4-Methoxyphenyl)-ethyl- | Thien-2-yl-CO- | 530 | 84.2 |
| 91 | 2-Chloro-benzyl- | 2-Fluoro-phenyl-CO- | 532 | 62.7 |
| 92 | Isobutyl- | 2-Fluoro-phenyl-CO- | 464 | |
| 93 | 1(S)-Phenyl-ethyl- | 2-Fluoro-phenyl-CO- | 512 | 92.9 |
| 94 | 2-Methoxy-benzyl- | 2-Fluoro-phenyl-CO- | 528 | 87.7 |
| 95 | 4-Methyl-benzyl- | 2-Fluoro-phenyl-CO- | 512 | 74.7 |
| 96 | 3-Methoxy-benzyl- | 2-Fluoro-phenyl-CO- | 528 | |
| 97 | 3,4-Methylenedioxy-benzyl- | 2-Fluoro-phenyl-CO- | 542 | |
| 98 | 1 (R)-Phenyl-ethyl- | 2-Fluoro-phenyl-CO- | 512 | 98.0 |
| 99 | 4-Trifluoromethyl-benzyl- | 2-Fluoro-phenyl-CO- | 566 | 89.6 |
| 100 | 2-(4-Methoxyphenyl)-ethyl- | 2-Fluoro-phenyl-CO- | 542 | 94.1 |
| 101 | 2-chloro-benzyl- | 2-Chloro-phenyl-CO- | 548 | 62.6 |
| 102 | Isobutyl- | 2-Chloro-phenyl-CO- | 480 | |
| 103 | 1(S)-Phenyl-ethyl- | 2-Chloro-phenyl-CO- | 528 | |
| 104 | 2-Methoxy-benzyi- | 2-Chloro-phenyl-CO- | 544 | |
| 105 | 4-Methyl-benzyl- | 2-Chloro-phenyl-CO- | 528 | |
| 106 | 3-Methoxy-benzyl- | 2-Chloro-phenyl-CO- | 544 | 98.9 |
| 107 | 3,4-Methylenedioxy-benzyl- | 2-Chloro-phenyl-CO- | 558 | 96.4 |
| 108 | 1(R)-Phenyl-ethyl- | 2-Chloro-phenyl-CO- | 528 | 87.7 |
| 109 | 4-Trifluoromethyl-benzyl- | 2-Chloro-phenyl-CO- | 582 | 75.4 |
| 110 | 2-(4-Methoxyphenyl)-ethyl- | 2-Chloro-phenyl-CO- | 558 | |
| 111 | 2-Chloro-benzyl- | (Phenyl)2CH-CO- | 604 | 80.5 |
| 112 | Isobutyl- | (Phenyl)2CH-CO- | 536 | 90.4 |
| 113 | 1(S)-Phenyl-ethyl- | (Phenyl)2CH-CO- | 584 | 94.6 |
| 114 | 2-Methoxy-benzyl- | (Phenyl)2CH-CO- | 600 | 81.7 |
| 115 | 4-Methyl-benzyl- | (Phenyl)2CH-CO- | 584 | 84.0 |
| 116 | 3-Methoxy-benzyl- | (Phenyl)2CH-CO- | 600 | |
| 117 | 3,4-Methylenedioxy-benzyl- | (Phenyl)2CH-CO- | 614 | |
| 118 | 1(R)-Phenyl-ethyl- | (Phenyl)2CH-CO- | 584 | 95.3 |
| 119 | 4-Trifluoromethyl-benzyl- | (Phenyl)2CH-CO- | 638 | 75.7 |
| 120 | 2-(4-Methoxyphenyl)-ethyl- | (Phenyl)2CH-CO- | 614 | 81.3 |
| 121 | 2-Chloro-benzyl- | Phenyl-CH2-CO- | 528 | 72.1 |
| 122 | Isobutyl- | Phenyl-CH2-CO- | 460 | 90.6 |
| 123 | 1(S)-Phenyl-ethyl- | Phenyl-CH2-CO- | 508 | 72.2 |
| 124 | 2-Methoxy-benzyl- | Phenyl-CH2-CO- | 524 | 46.5 |
| 125 | 4-Methyl-benzyl- | Phenyl-CH2-CO- | 508 | 63.6 |
| 126 | 3-Methoxy-benzyl- | Phenyl-CH2-CO- | 524 | 53.7 |
| 127 | 3,4-Methylenedioxy-benzyl- | Phenyl-CH2-CO- | 538 | 61.7 |
| 128 | 1(R)-Phenyl-ethyl- | Phenyl-CH2-CO- | 508 | 61.4 |
| 129 | 4-Trifluoromethyl-benzyl- | Phenyl-CH2-CO- | 562 | 47.4 |
| 130 | 2-(4-Methoxyphenyl)-ethyl- | Phenyl-CH2-CO- | 538 | 77.8 |
| 131 | 2-Chloro-benzyl- | Phenyl-CH2-CO-CH(Phenyl)-CO- | 646 | 97.6 |
| 132 | 1(S)-Phenyl-ethyl- | (2-Thienyl)-CH2-CO- | 514 | 63.8 |
| 133 | 1(R)-Phenyl-ethyl- | (2-Thienyl)-CH2-CO- | 514 | 40.9 |
| 134 | Isobutyl- | Phenyl-CH2-CO-CH(Phenyl)-CO- | 578 | |
| 135 | 4-Methyl-benzyl- | Phenyl-CH2-CO-CH(Phenyl)-CO- | 626 | 97.4 |
| 136 | 1(S)-Phenyl-ethyl- | Phenyl-CH2-CO-CH(Phenyl)-CO- | 626 | 93.6 |
| 137 | 1(S)-(4-Methylphenyl)-ethyl- | (2-Thienyl)-CH2-CO- | 528 | |
| 138 | 1(R)-(4-Methylphenyl)-ethyl- | (2-Thienyl)-CH2-CO- | 528 | |
| 139 | 2-Furylmethyl- | (2-Thienyl)-CH2-CO- | 490 | 48.5 |
| 140 | 4-(Dimethylamino)-benzyl- | (2-Thienyl)-CH2-CO- | 543 | 54.2 |
| 141 | 2-(2-Thienyl)-ethyl- | (2-Thienyl)-CH2-CO- | 520 | 71.1 |
| 142 | 2-Phenoxyethyl- | (2-Thienyl)-CH2-CO- | 530 | 27.1 |
| 143 | 2-Chloro-benzyl- | (2-Thienyl)-CH2-CO- | 534 | 35.7 |
| 144 | Cyclopropylmethyl- | (2-Thienyl)-CH2-CO- | 464 | 60.3 |
| 145 | 3,4,5-Trimethoxy-benzyl- | (2-Thienyl)-CH2-CO- | 590 | 39.6 |
| 146 | (4-Pyridyl)-methyl- | (2-Thienyl)-CH2-CO- | 501 | 69.8 |
| 147 | 4-Fluoro-benzyl- | (2-Thienyl)-CH2-CO- | 518 | 53.2 |
| 148 | 2-Furylmethyl- | (4-Chlorophenyl)-CH2-CO- | 518 | 45.6 |
| 149 | 4-(Dimethylamino)-benzyl- | (4-Chlorophenyl)-CH2-CO- | 571 | 77.4 |
| 150 | 2-(2-Thienyl)-ethyl- | (4-Chlorophenyl)-CH2-CO- | 548 | 44.7 |
| 151 | 2-Phenoxyethyl- | (4-Chlorophenyl)-CH2-CO- | 558 | 29.0 |
| 152 | 2-Chloro-benzyl- | (4-Chlorophenyl)-CH2-CO- | 562 | 29.5 |
| 153 | Cyclopropylmethyl- | (4-Chlorophenyl)-CH2-CO- | 492 | 46.1 |
| 154 | 2,4-Dimethoxy-benzyl- | (4-Chlorophenyl)-CH2-CO- | 588 | 36.2 |
| 155 | 3,4,5-Trimethoxy-benzyl- | (4-Chlorophenyl)-CH2-CO- | 618 | 34.2 |
| 156 | (4-Pyridyl)-methyl- | (4-Chlorophenyl)-CH2-CO- | 529 | 86.0 |
| 157 | 4-Fluoro-benzyl- | (4-Chlorophenyl)-CH2-CO- | 546 | 24.9 |
| 158 | 2-Furylmethyl- | (CH3)3C-CH2-CO- | 464 | 57.9 |
| 159 | 2-(2-Thienyl)-ethyl- | (CH3)3C-CH2-CO- | 494 | 61.2 |
| 160 | 2-Phenoxyethyl- | (CH3)3C-CH2-CO- | 504 | 71.6 |
| 161 | Cyclopropylmethyl- | (CH3)3C-CH2-CO- | 438 | 81.6 |
| 162 | 3,45-Trimethoxy-benzyl- | (CH3)3C-CH2-CO- | 564 | 30.2 |
| 163 | 4-Fluoro-benzyl- | (CH3)3C-CH2-CO- | 492 | 93.4 |
| 164 | 2-Furylmethyl- | Phenyl-O-CH2-CO- | 500 | 76.4 |
| 165 | 2-(2-Thienyl)-ethyl- | Phenyl-O-CH2-CO- | 530 | 56.8 |
| 166 | 2-Phenoxyethyl- | Phenyl-O-CH2-CO- | 540 | 57.9 |
| 167 | 2-Chloro-benzyl- | Phenyl-O-CH2-CO- | 544 | 47.5 |
| 168 | Cyclopropylmethyl- | Phenyl-O-CH2-CO- | 474 | 96.4 |
| 169 | 3,4,5-Trimethoxy-benzyl- | Phenyl-O-CH2-CO- | 600 | |
| 170 | 4-Fluoro-benzyl- | Phenyl-O-CH2-CO- | 528 | |
| 171 | 2-Furylmethyl- | (4-Methoxyphenyl)-CH2-CO- | 514 | |
| 172 | 2-(2-Thienyl)-ethyl- | (4-Methoxyphenyl)-CH2-CO- | 544 | 89.7 |
| 173 | 2-Phenoxyethyl- | (4-Methoxyphenyl)-CH2-CO- | 554 | 76.4 |
| 174 | 2-chloro-benzyl- | (4-Methoxyphenyl)-CH2-CO- | 558 | 50.2 |
| 175 | Cyclopropylmethyl- | (4-Methoxyphenyl)-CH2-CO- | 488 | 93.2 |
| 176 | 3,4,5-Trimethoxy-benzyl- | (4-Methoxyphenyl)-CH2-CO- | 614 | 83.9 |
| 177 | 4-Fluoro-benzyl- | (4-Methoxyphenyl)-CH2-CO- | 542 | 64.4 |
| 178 | 2-Furylmethyl- | CH3-CO- | 408 | 70.6 |
| 179 | 2-(2-Thienyl)-ethyl- | CH3-CO- | 438 | 78.3 |
| 180 | 2-Phenoxy-ethyl- | CH3-CO- | 448 | 70.9 |
| 181 | 2-Chloro-benzyl- | CH3-CO- | 452 | 80.8 |
| 182 | Cyclopropylmethyl- | CH3-CO- | 382 | 74.9 |
| 183 | 2,4-Dimethoxy-benzyl- | CH3-CO- | 478 | |
| 184 | 3,4,5-Trimethoxy-benzyl- | CH3-CO- | 508 | |
| 185 | 4-Fluoro-benzyl- | CH3-CO- | 436 | 85.0 |
| 186 | 3-Phenyl-propyl- | (2-Thienyl)-CH2-CH2-CH2-CO- | 556 | 9.7 |
| 187 | 2-Methoxy-ethyl- | (2-Thienyl)-CH2-CH2-CH2-CO- | 496 | 62.4 |
| 188 | (2-Pyridyl)-methyl- | (2-Thienyl)-CH2-CH2-CH2-CO- | 529 | 52.3 |
| 189 | Cyclopropyl- | (2-Thienyl)-CH2-CH2-CH2-CO- | 478 | 72.5 |
| 190 | Methyl- | (2-Thienyl)-CH2-CH2-CH2-CO- | 452 | 43.4 |
| 191 | Ethyl- | (2-Thienyl)-CH2-CH2-CH2-CO- | 466 | 39.3 |
| 192 | (2-Benzimidazolyl)-methyl- | (2-Thienyl)-CH2-CH2-CH2-CO- | 568 | 49.1 |
| 193 | 1-Methoxycarbonyl-2-phenyl-ethyl- | (2-Thienyl)-CH2-CH2-CH2-CO- | 600 | 62.8 |
| 194 | 3-Phenyl-propyl- | (2-Thienyl)-CO-CO- | 542 | 61.1 |
| 195 | 2-Methoxy-ethyl- | (2-Thienyl)-CO-CO- | 482 | 58.3 |
| 196 | (2-Pyridyl)-methyl- | (2-Thienyl)-CO-CO- | 515 | 62.8 |
| 197 | Cyclopropyl- | (2-Thienyl)-CO-CO- | 464 | 79.9 |
| 198 | Methyl- | (2-Thienyl)-CO-CO- | 438 | 51.1 |
| 199 | Ethyl- | (2-Thienyl)-CO-CO- | 452 | 54.7 |
| 200 | (2-Benzimidazolyl)-methyl- | (2-Thienyl)-CO-CO- | 554 | 55.2 |
| 201 | 1-Methoxycarbonyl-2-phenyl- | (2-Thienyl)-CO-CO- | 586 | 69.6 |
| | ethyl- | | | |
| 202 | 3-Phenyl-propyl- | (Indol-3-yl)-CH2-CO- | 561 | 38.5 |
| 203 | 2-Methoxy-ethyl- | (Indol-3-yl)-CH2-CO- | 501 | 30.3 |
| 204 | (2-Pyridyl)-methyl- | (Indol-3-yl)-CH2-CO- | 534 | 52.4 |
| 205 | Cyclopropyl- | (Indol-3-yl)-CH2-CO- | 483 | 41.7 |
| 206 | Methyl- | (Indol-3-yl)-CH2-CO- | 457 | 23.8 |
| 207 | Ethyl- | (Indol-3-yl)-CH2-CO- | 471 | 31.0 |
| 208 | (2-Benzimidazolyl)-methyl- | (Indol-3-yl)-CH2-CO- | 573 | 50.7 |
| 209 | 1-Methoxycarbonyl-2-phenyl-ethyl- | (Indol-3-yl)-CH2-CO- | 605 | 56.2 |
| 210 | 3-Phenyl-propyl- | (Benzo[b]thiophen-3-yl)-CH2-CO- | 578 | 66.2 |
| 211 | 2-Methoxy-ethyl- | (Benzo[b]thiophen-3-yl)-CH2-CO- | 518 | 50.6 |
| 212 | (2-Pyridyl)-methyl- | (Benzo[b]thiophen-3-yl)-CH2-CO- | 551 | 60.2 |
| 213 | Cyclopropyl- | (Benzo[b]thiophen-3-yl)-CH2-CO- | 500 | 45.0 |
| 214 | Methyl- | (Benzo[b]thiophen-3-yl)-CH2-CO- | 474 | 44.3 |
| 215 | Ethyl- | (Benzo[b]thiophen-3-yl)-CH2-CO- | 488 | 65.7 |
| 216 | (2-Benzimidazolyl)-methyl- | (Benzo[b]thiophen-3-yl)-CH2-CO- | 590 | 62.2 |
| 217 | 1-Methoxycarbonyl-2-phenyl-ethyl- | (Benzo[b]thiophen-3-yl)-CH2-CO- | 622 | 46.0 |
| 218 | 3-Phenyl-propyl- | (2-Thienyl)-(CH2)4-CO- | 570 | 87.1 |
| 219 | 2-Methoxy-ethyl- | (2-Thienyl)-(CH2)4-CO- | 510 | 82.5 |
| 220 | (2-Pyridyl)-methyl- | (2-Thienyl)-(CH2)4-CO- | 543 | 73.2 |
| 221 | cyclopropyl- | (2-Thienyl)-(CH2)4-CO- | 492 | 72.3 |
| 222 | Methyl- | (2-Thienyl)-(CH2)4-CO- | 466 | 76.7 |
| 223 | Ethyl- | (2-Thienyl)-(CH2)4-CO- | 480 | 53.8 |
| 224 | (2-Benzimidazolyl)-methyl- | (2-Thienyl)-(CH2)4-CO- | 582 | 79.8 |
| 225 | 1-Methoxycarbonyl-2-phenyl-ethyl- | (2-Thienyl)-(CH2)4-CO- | 614 | 88.1 |
| 226 | 3-Phenyl-propyl- | (3-Thienyl)-CH2-CO- | 528 | 34.8 |
| 227 | 2-Methoxy-ethyl- | (3-Thienyl)-CH2-CO- | 468 | 50.6 |
| 228 | (2-Pyridyl)-methyl- | (3-Thienyl)-CH2-CO- | 501 | 94.8 |
| 229 | Cyclopropyl- | (3-Thienyl)-CH2-CO- | 450 | 86.7 |
| 230 | Methyl- | (3-Thienyl)-CH2-CO- | 424 | 68.6 |
| 231 | Ethyl- | (3-Thienyl)-CH2-CO- | 438 | 57.8 |
| 232 | (2-Benzimidazolyl)-methyl- | (3-Thienyl)-CH2-CO- | 540 | 62.6 |
| 233 | 1-Methoxycarbonyl-2-phenylethyl- | (3-Thienyl)-CH2-CO- | 572 | 68.1 |
| 234 | 2-Phenyl-ethyl- | (2-Thienyl)-CH2-CO- | 514 | |
| 235 | 4-Trifluoromethyl-benzyl- | (2-Thienyl)-CH2-CO- | 568 | 67.1 |
| 236 | 3-Phenyl-propyl- | (2-Thienyl)-CH2-CO- | 528 | 35.1 |
| 237 | 2-Methoxy-ethyl- | (2-Thienyl)-CH2-CO- | 468 | 50.3 |
| 238 | (3,4-Dihydro-quinazolin-2-yl)-methyl- | (2-Thienyl)-CH2-CO- | 554 | 54.7 |
| 239 | Methyl- | (2-Thienyl)-CH2-CO- | 424 | 56.3 |
| 240 | Ethyl- | (2-Thienyl)-CH2-CO- | 438 | 58.0 |
| 241 | Benzyl- | Phenyl-CH(NH2)-CO- | 509 | 66.0 |
| 242 | Benzyl- | Phenyl-CH(NHCO-O-Benzyl)-CO- | 643 | 72.4 |
| 243 | Benzyl- | (2-Nitrophenyl)-CH2-CO- | 539 | 99.9 |
| 244 | Benzyl- | Phenyl-(CH2)4-CO- | 536 | 60.4 |
| 245 | Benzyl- | (2-Furyl)-CO-CO- | 498 | 73.0 |
| 246 | Propargyl- | Phenyl-CH(NHCO-O-Benzyl)-CO- | 591 | 65.0 |
| 247 | Propargyl- | Phenyl-(CH2)4-CO- | 484 | 74.6 |
| 248 | Propargyl- | (2-Furyl)-CO-CO- | 446 | 66.6 |
| 249 | Propargyl- | Cyclohexyl-CH2-CH2-CO- | 462 | 76.1 |
| 250 | (2-Thienyl)-methyl- | (2-Thienyl)-CH2-CO- | 506 | 41.1 |
| 251 | (2-Thienyl)-methyl- | Phenyl-CH(NHCO-O-Benzyl)-CO- | 649 | 83.9 |
| 252 | (2-Thienyl)-methyl- | (2-Nitrophenyl)-CH2-CO- | 545 | 44.5 |
| 253 | (2-Thienyl)-methyl- | Phenyl-(CH2)4-CO- | 542 | 35.3 |
| 254 | (2-Thienyl)-methyl- | (2-Furyl)-CO-CO- | 504 | 64.5 |
| 255 | (2-Thienyl)-methyl- | Cyclohexyl-CH2-CH2-CO- | 520 | 61.0 |
| 256 | Cyclohexyl-methyl- | (2-Thienyl)-CH2-CO- | 506 | 36.4 |
| 257 | Cyclohexyl-methyl- | (2-Nitrophenyl)-CH2-CO- | 545 | 54.6 |
| 255 | Cyclohexyl-methyl- | (2-Furyl)-CO-CO- | 504 | 95.8 |
| 259 | 3,4-Dichloro-benzyl- | (2-Thienyl)-CH2-CO- | 568 | 40.1 |
| 260 | 3,4-Dichloro-benzyl- | Phenyl-CH(NHCO-O-Benzyl)-CO- | 711 | 69.8 |
| 261 | 3,4-Dichloro-benzyl- | (2-Nitrophenyl)-CH2-CO- | 607 | 67.7 |
| 262 | 3,4-Dichloro-benzyl- | Phenyl-(CH2)4-CO- | 604 | 45.3 |
| 263 | 3,4-Dichloro-benzyl- | (2-Furyl)-CO-CO- | 566 | 54.0 |
| 264 | 3,4-Dichloro-benzyl- | Cyclohexyl-CH2-CH2-CO- | 582 | 74.3 |
| 265 | Cyclohexyl-methyl- | Phenyl-CH(NHCO-O-Benzyl)-CO- | 649 | 68.9 |
| 266 | Cyclohexyl-methyl- | Phenyl-(CH2)4-CO- | 542 | 79.7 |
| 267 | Cyclohexyl-methyl- | Cyclohexyl-CH2-CH2-CO- | 520 | 57.9 |
| 268 | Phenyl-CH(COOCH3)- | (2-Thienyl)-CH2-CO- | 558 | 79.3 |
| 269 | Phenyl-CH(COOC(CH3)3)- | (2-Thienyl)-CH2-CO- | 600 | 94.4 |
| 270 | 2,4-Dichloro-benzyl- | (2-Thienyl)-CH2-CO- | 568 | 33.9 |
| 271 | (1-Naphthyl)-methyl- | (2-Thienyl)-CH2-CO- . | 550 | 15.9 |
| 272 | 2-(4-H2NSO2-Phenyl)-ethyl- | (2-Thienyl)-CH2-CO- | 593 | 65.0 |
| 273 | 3-Chloro-benzyl- | (2-Thienyl)-CH2-CO- | 534 | 32.4 |
| 274 | Phenyl-CH(COOCH3)- | (2-Bromophenyl)-CH2-CO- | 630 | 73.6 |
| 275 | Phenyl-CH(COOC(CH3)3)- | (2-Bromophenyl)-CH2-CO- | 672 | 54.6 |
| 276 | 2,4-Dichloro-benzyl- | (2-Bromophenyl)-CH2-CO- | 640 | 36.8 |
| 277 | (1-Naphthyl)-methyl- | (2-Bromophenyl)-CH2-CO- | 622 | 36.3 |
| 278 | 3-Chloro-benzyl- | (2-Bromophenyl)-CH2-CO- | 606 | 31.2 |
| 279 | 2,4-Dichloro-benzyl- | Phenyl-SO2-CH2-CH2-CO- | 640 | 90.5 |
| 280 | (1-Naphthyl)-methyl- | Phenyl-SO2-CH2-CH2-CO- | 622 | 63.3 |
| 281 | 3-Chloro-benzyl- | Phenyl-SO2-CH2-CH2-CO- | 606 | 68.6 |
| 282 | Phenyl-CH(COOH)- | (2-Thienyl)-CH2-CO- | 544 | 89.0 |
| 283 | 2,4-Dichloro-benzyl- | (4-Hydroxyphenyl)-CH2-CO- | 578 | 80.0 |
| 284 | (1-Naphthyl)-methyl- | (4-Hydroxyphenyl)-CH2-CO- | 560 | 52.9 |
| 285 | 3-Chloro-benzyl- | (4-Hydroxyphenyl)-CH2-CO- | 544 | 58.8 |
| 286 | Phenyl-CH(COOCH3)- | (2-Thienyl)-CH2-CH2-CO- | 572 | 68.8 |
| 287 | Phenyl-CH(COOC(CH3)3)- | (2-Thienyl)-CH2-CH2-CO- | 614 | 51.1 |
| 288 | 2,4-Dichloro-benzyl- | (2-Thienyl)-CH2-CH2-CO- | 582 | 63.1 |
| 289 | (1-Naphthyl)-methyl- | (2-Thienyl)-CH2-CH2-CO- | 564 | 39.2 |
| 290 | 3-Chloro-benzyl- | (2-Thienyl)-CH2-CH2-CO- | 548 | 45.8 |
| 291 | 2-Chloro-benzyl- | (2-Chlorophenyl)-CH2-CO- | 562 | 23.7 |
| 292 | 2,4-Dichloro-benzyl- | (2-Chlorophenyl)-CH2-CO- | 596 | |
| 293 | (1-Naphthyl)-methyl- | (2-Chlorophenyl)-CH2-CO- | 578 | 55.1 |
| 294 | 2-(4-H2NSO2-Phenyl)-ethyl- | (2-Chlorophenyl)-CH2-CO- | 621 | 82.7 |
| 295 | (2-Thienyl)-methyl- | Phenyl-CH2-NH-CO- | 515 | 8.0 |
| 296 | (2-Thienyl)-methyl- | N-Cyclohexyl-NH-CO- | 507 | 31.7 |
| 297 | Benzyl- | N-(2,6-Difluorophenyl)-NH-CO- | 531 | 80.4 |
| 298 | Benzyl- | N-Isopropyl-NH-CO- | 461 | 74.7 |
| 299 | Benzyl- | Phenyl-CH2-NH-CO- | 509 | 48.6 |
| 300 | Benzyl- | N-Cyclohexyl-NH-CO- | 501 | 29.4 |
| 301 | 3-Methoxy-benzyl- | N-(2,6-Difluorophenyl)-NH-CO- | 561 | 87.9 |
| 302 | 3-Methoxy-benzyl- | N-Isopropyl-NH-CO- | 491 | 80.4 |
| 303 | 3-Methoxy-benzyl- | Phenyl-CH2-NH-CO- | 539 | 65.8 |
| 304 | 3-Methoxy-benzyl- | N-Cyclohexyl-NH-CO- | 531 | 56.0 |
| 305 | 3-Chloro-benzyl- | N-(2,6-Difluorophenyl)-NH-CO- | 565 | 84.9 |
| 306 | 3-Chloro-benzyl- | N-Isopropyl-NH-CO- | 495 | 90.2 |
| 307 | 3-Chloro-benzyl- | Phenyl-CH2-NH-CO- | 543 | 55.8 |
| 308 | 3-Chloro-benzyl- | N-Cyclohexyl-NH-CO- | 535 | 32.1 |
| 309 | 2,3-Dichloro-benzyl- | (2-Thienyl)-CH2-CO- | 568 | 88.9 |
| 310 | (2-Naphthyl)-methyl- | (2-Thienyl)-CH2-CO- | 550 | 47.7 |
| 311 | 3-Methyl-benzyl- | (2-Thienyl)-CH2-CO- | 514 | 10.4 |
| 312 | 2-Methyl-benzyl- | (2-Thienyl)-CH2-CO- | 514 | 12.5 |
| 313 | (CH3)2C=CH-CH2-CH2-C(CH3)=CH-CH2- | (2-Thienyl)-CH2-CO- | 546 | 17.2 |
| 314 | 1-Indanyl- | (2-Thienyl)-CH2-CO- | 526 | 14.4 |
| 315 | 1,2,3,4-Tetrahydro-1-naphthyl- | (2-Thienyl)-CH2-CO- | 540 | 23.5 |
| 316 | 2-Fluoro-benzyl- | (2-Thienyl)-CH2-CO- | 518 | 14.4 |
| 317 | 3-Phenyl-benzyl- | (2-Thienyl)-CH2-CO- | 576 | 14.5 |
| 318 | (1,2,3,4-Tetrahydro-2-furyl)-methyl- | (2-Thienyl)-CH2-CO- | 494 | 16.4 |
| 319 | 2,3-Dichloro-benzyl- | Methoxy-CH2-CH2-CO- | 530 | 38.0 |
| 320 | (2-Naphthyl)-methyl- | Methoxy-CH2-CH2-CO- | 512 | 38.8 |
| 321 | 3-Methyl-benzyl- | Methoxy-CH2-CH2-CO- | 476 | |
| 322 | 2-Methyl-benzyl- | Methoxy-CH2-CH2-CO- | 476 | 66.3 |
| 323 | (CH3)2C=CH-CH2-CH2-C(CH3)=CH-CH2- | Methoxy-CH2-CH2-CO- | 508 | 23.6 |
| 324 | 1-Indanyl- | Methoxy-CH2-CH2-CO- | 488 | 60.5 |
| 325 | 1,2,3,4-Tetrahydro-1-naphthyl- | Methoxy-CH2-CH2-CO- | 502 | 33.8 |
| 326 | 2-Fluoro-benzyl- | Methoxy-CH2-CH2-CO- | 480 | 25.6 |
| 327 | 3-Phenyl-benzyl- | Methoxy-CH2-CH2-CO- | 538 | 17.4 |
| 328 | (1,2,3,4-Tetrahydro-2-furyl)-methyl- | Methoxy-CH2-CH2-CO- | 456 | 87.0 |
| 329 | 2,3-Dichloro-benzyl- | 2-Methoxy-phenyl-CO- | 578 | 25.7 |
| 330 | (2-Naphthyl)-methyl- | 2-Methoxy-phenyl-CO- | 560 | 14.4 |
| 331 | 3-Methyl-benzyl- | 2-Methoxy-phenyl-CO- | 524 | 37.6 |
| 332 | 2-Methyl-benzyl- | 2-Methoxy-phenyl-CO- | 524 | 31.7 |
| 333 | (CH3)2C=CH-CH2-CH2- | 2-Methoxy-phenyl-CO- | 556 | 22.3 |
| | C(CH3)=CH-CH2- | | | |
| 334 | 1-Indanyl- | 2-Methoxy-phenyl-CO- | 536 | |
| 335 | 1,2,3,4-Tetrahydro-1-naphthyl- | 2-Methoxy-phenyl-CO- | 550 | 79.6 |
| 336 | 2-Fluoro-benzyl- | 2-Methoxy-phenyl-CO- | 528 | 58.1 |
| 337 | 3-Phenyl-benzyl- | 2-Methoxy-phenyl-CO- | 586 | 51.2 |
| 338 | (1,2,3,4-Tetrahydro-2-furyl)-methyl- | 2-Methoxy-phenyl-CO- | 504 | 50.7 |
| 339 | 2,3-Dichloro-benzyl- | Phenoxy-CH2-CH2-CO- | 592 | 46.8 |
| 340 | (2-Naphthyl)-methyl- | Phenoxy-CH2-CH2-CO- | 574 | 37.2 |
| 341 | 3-Methyl-benzyl- | Phenoxy-CH2-CH2-CO- | 538 | 44.6 |
| 342 | 2-Methyl-benzyl- | Phenoxy-CH2-CH2-CO- | 538 | 85.3 |
| 343 | (CH3)2C=CH-CH2-CH2-C(CH3)=CH-CH2- | Phenoxy-CH2-CH2-CO- | 570 | 48.1 |
| 344 | 1-Indanyl- | Phenoxy-CH2-CH2-CO- | 550 | 52.2 |
| 345 | 1,2,3,4-Tetrahydro-1-naphthyl- | Phenoxy-CH2-CH2-CO- | 564 | 39.8 |
| 346 | 2-Fluoro-benzyl- | Phenoxy-CH2-CH2-CO- | 542 | 36.0 |
| 347 | 3-Phenyl-benzyl- | Phenoxy-CH2-CH2-CO- | 600 | 19.3 |
| 348 | (1,2,3,4-Tetrahydro-2-furyl)-methyl- | Phenoxy-CH2-CH2-CO- | 518 | 46.3 |
| 349 | 2,3-Dichloro-benzyl- | Cyclohexyl-CH2-CO- | 568 | 50.4 |
| 350 | (2-Naphthyl)-methyl- | Cyclohexyl-CH2-CO- | 550 | |
| 351 | 3-Methyl-benzyl- | Cyclohexyl-CH2-CO- | 514 | 27.2 |
| 352 | 2-Methyl-benzyl- | Cyclohexyl-CH2-CO- | 514 | 35.2 |
| 353 | (CH3)2C=CH-CH2-CH2-C(CH3)=CH-CH2- | Cyclohexyl-CH2-CO- | 546 | 47.9 |
| 354 | 1-Indanyl- | Cyclohexyl-CH2-CO- | 526 | 37.2 |
| 355 | 1,2,3,4-Tetrahydro-1-naphthyl- | Cyclohexyl-CH2-CO- | 540 | 46.8 |
| 356 | 2-Fluoro-benzyl- | Cyclohexyl-CH2-CO- | 518 | 47.4 |
| 357 | 3-Phenyl-benzyl- | Cyclohexyl-CH2-CO- | 576 | 61.9 |
| 358 | (1,2,3,4-Tetrahydro-2-furyl)-methyl- | Cyclohexyl-CH2-CO- | 494 | 55.5 |
| 359 | 2,3-Dichloro-benzyl- | Cyclopentyl-CH2-CO- | 554 | 37.9 |
| 360 | (2-Naphthyl)-methyl- | Cyclopentyl-CH2-CO- | 536 | 36.9 |
| 361 | 3-Methyl-benzyl- | Cyclopentyl-CH2-CO- | 500 | 29.4 |
| 362 | 2-Methyl-benzyl- | Cyclopentyl-CH2-CO- | 500 | 44.1 |
| 363 | (CH3)2C=CH-CH2-CH2-C(CH3)=CH-CH2- | Cyclopentyl-CH2-CO- | 532 | 48.3 |
| 364 | 1-Indanyl- | cyclopentyl-CH2-CO- | 512 | 52.6 |
| 365 | 1,2,3,4-Tetrahydro-1-naphthyl- | Cyclopentyl-CH2-CO- | 526 | 45.1 |
| 366 | 2-Fluoro-benzyl- | Cyclopentyl-CH2-CO- | 504 | 38.4 |
| 367 | 3-Phenyl-benzyl- | Cyclopentyl-CH2-CO- | 562 | 25.5 |
| 368 | (1,2,3,4-Tetrahydro-2-furyl)-methyl- | Cyclopentyl-CH2-CO- | 480 | 51.0 |
| 369 | 2,4-Difluorophenyl- | 4-Methylphenyl-SO2- | 590 | 33.7 |
| 370 | 4-tert.-Butylphenyl- | 3,4-Dimethoxyphenyl-SO2- | 656 | 50.1 |
| 371 | 4-tert.-Butylphenyl- | 2,5-Dimethoxyphenyl-S02- | 656 | 38.6 |
| 372 | 2,4-Difluorophenyl- | 2,3,4,5,6-Pentamethylphenyl-SO2- | 646 | 23.1 |
| 373 | 2,6-Dichlorophenyl- | 3-Fluoro-2,4-dimethylphenyl-SO2- | 654 | 20.6 |
| 374 | 3-Chloro-4-fluorophenyl- | 4-Fluoro-3,5-dimethlyphenyl-S02- | 638 | 70.3 |
| 375 | 2-Cyanophenyl- | 4-Methylphenyl-SO2- | 579 | 80.6 |
| 376 | 4-Chloro-2,5-dimethoxyphenyl- | 4-Methylphenyl-SO2- | 648 | 66.6 |
| 377 | 4-Chloro-2,5-dimethoxyphenyl- | 4-Chlorophenyl-SO2- | 668 | 36.8 |
| 378 | 4-Fluorophenyl- | 2,4,5-Trichlorophenyl-SO2- | 660 | 43.6 |
| 379 | 4-(4-Fluorophenoxy)-phenyl- | 4-Methylphenyl-SO2- | 664 | |
| 380 | 2-Chlorophenyl- | 2,5-Dimethoxyphenyl-SO2- | 634 | 28.1 |
| 381 | 2-Cyanophenyl- | 2,5-Dimethoxyphenyl-SO2- | 625 | 60.8 |
| 382 | 2,3-Dichlorophenyl- | 3,4-Dichlorophenyl-SO2- | 676 | 47.1 |
| 383 | 4-(4-Chlorophenoxy)-phenyl- | 4-Methylphenyl-SO2- | 680 | 42.2 |
| 384 | 3-Trifluoromethyl-phenyl- | 4-Methylphenyl-SO2- | 622 | 57.2 |
| 385 | 2-Cyanophenyl- | 4-tert.-Butylphenyl-SO2- | 621 | 64.8 |
| 386 | 2-Acetyl-phenyl- | 2,4-Dichlorophenyl-SO2- | 650 | 33.5 |
| 387 | 2-Acetyl-phenyl- | 2,3-Dichlorophenyl-SO2- | 650 | 29.8 |
| 388 | Benzyl- | (3-Thienyl)-CH-(CH3)-CO- | 514 | 63.7 |
| 389 | Benzyl- | (2-Furyl)-CH2-CO- | 484 | 51.5 |
| 390 | (1-Naphthyl)-methyl- | (2-Thienyl)-CH-(CH3)-CO- | 564 | 89.8 |
| 391 | (1-Naphthyl)-methyl- | (2-Furyl)-CH2-CO- | 534 | 55.4 |
| 392 | (1-Phenylcylopentyl)-methyl- | (2-Thienyl)-CH2-CO- | 568 | 55.6 |
| 393 | (1-Phenylcylopentyl)-methyl- | (2-Thienyl)-CH-(CH3)-CO- | 582 | 16.7 |
| 394 | (1-Phenylcylopentyl)-methyl- | (2-Furyl)-CH2-CO- | 552 | 72.5 |
| 395 | 2-Ethyl-2-(4-Methoxyphenyl)-butyl- | (2-Thienyl)-CH2-CO- | 600 | 50.9 |
| 396 | [3-(Pyrrolidine-1-carbonyl)-4,5-dihydro-isoxazol-5-yl]-methyl- | (2-Thienyl)-CH2-CO- | 590 | 53.2 |
| 397 | [3-(Pyrrolidine-1-carbonyl)-4,5-dihydro-isoxazol-5-yl]-methyl- | (2-Thienyl)-CH-(CH3)-CO- | 604 | 44.0 |
| 398 | Phenyl- | Phenyl-SO2- | 540 | |
| 399 | 4-Acetylamino-2-methylphenyl- | 4-Chlorophenyl-SO2- | 645 | |
| 400 | 5-Acetylamino-2-methylphenyl- | 2,5-Dichlorophenyl-SO2- | 679 | |
| 401 | 4-Acetylamino-2,6-dimethylphenyl- | 4-Methylphenyl-SO2- | 639 | |
| 402 | Phenyl- | 4-Methylphenyl-SO2- | 554 | |
| 403 | 2-Acetylphenyl- | 2,4,5-Trichlorophenyl-SO2- | 684 | |
| 404 | 2-Acetylphenyl- | 4-Trifluoromethyl-phenyl-SO2- | 650 | |
| 405 | 4-Chlorophenyl- | 4-Chlorophenyl-SO2- | 608 | |
| 406 | 4-Chlorophenyl- | Phenyl-SO2- | 574 | |
| 407 | 3-Acetylphenyl- | 4-Methoxyphenyl-SO2- | 612 | |
| 408 | 3-Acetylphenyl- | 2-Chlorophenyl-SO2- | 616 | |
| 409 | 3-Acetylphenyl- | 2-Chloro-6-methylphenyl-SO2- | 630 | |
| 410 | 3-Acetylphenyl- | 4-tert.-Butylphenyl-SO2- | 638 | |
| 411 | 3-Acetylphenyl- | 4-Fluorophenyl-SO2- | 600 | |
| 412 | 3-Acetylphenyl- | 3,4-Dichlorophenyl-SO2- | 650 | |
| 413 | 3-Acetylphenyl- | 2,4-Difluorophenyl-SO2- | 618 | |
| 414 | 3-Acetylphenyl- | 3-Trifluoromethyl-phenyl-SO2- | 650 | |
| 415 | 4-Trifluoromethyl-phenyl- | 4-Acetylaminophenyl-SO2- | 665 | |
| 416 | 2,5-Difluorophenyl- | 4-Acetylaminophenyl-SO2- | 633 | |
| 417 | 4-tert.-Butylphenyl- | 4-Acetylaminophenyl-SO2- | 653 | |
| 418 | 4-Isopropylphenyl- | 2-Chloro-4-cyanophenyl-SO2- | 641 | |
| 419 | 4-Methoxyphenyl- | 4-Acetylaminophenyl-SO2- | 627 | |
| 420 | 4-Ethoxyphenyl- | 4-Chlorophenyl-SO2- | 618 | |
| 421 | 5-Chloro-2-methoxyphenyl- | 4-Chlorophenyl-SO2- | 638 | |
| 422 | 5-Chloro-2-methoxyphenyl- | 2,4,5-Trichlorophenyl-SO2- | 706 | |
| 423 | 2-Fluorophenyl- | 4-Acetylaminophenyl-SO2- | 615 | |
| 424 | 1-Naphthyl- | 4-Acetylaminophenyl-SO2- | 647 | |
| 425 | 2-Trifluoromethyl-phenyl- | 4-Acetylaminophenyl-SO2- | 665 | |
| 426 | 2-Ethylphenyl- | 4-Chlorophenyl-SO2- | 602 | |
| 427 | 2,4-Dimethylphenyl- | Phenyl-SO2- | 568 | |
| 428 | 2,45-Trimethylphenyl- | 4-Chlorophenyl-SO2- | 616 | |
| 429 | 2,5-Dimethylphenyl- | 2,4,5-Trichiorophenyl-SO2- | 670 | |
| 430 | 5-Chloro-2-methylphenyl- | 4-Chlorophenyl-SO2- | 622 | |
| 431 | 4-Fluorophenyl- | Phenyl-SO2- | 558 | |
| 432 | 3-Chloro-2-methylphenyl- | Phenyl-SO2 | 588 | |
| 433 | 4-Chloro-2-methylphenyl- | Phenyl-SO2- | 588 | |
| 434 | 2,3-Dichlorophenyl- | 4-Chlorophenyl-SO2- | 642 | |
| 435 | 3-Trifluoromethyl-phenyl- | 2,4,5-Trichlorophenyl-SO2- | 710 | |
| 436 | 5-Chloro-2-methylphenyl- | 2,4,5-Trichlorophenyl-SO2- | 690 | |
| 437 | 2-Fluorophenyl- | 2,4,5-Trichlorophenyl-SO2- | 660 | |
| 438 | 2,6-dimethylphenyl- | Phenyl-SO2- | 568 | |
| 439 | 2,3-Dimethylphenyl- | Phenyl-SO2- | 568 | |
| 440 | 1-Naphthyl- | 3,4-Dichlorophenyl-SO2- | 658 | |
| 441 | 4-Ethoxyphenyl- | 3,4-Dichlorophenyl-SO2- | 652 | |
| 442 | 1-Naphthyl- | 4-Chlorophenyl-SO2- | 624 | |
| 443 | 3,5-Dichlorophenyl- | 3,4-Dichlorophenyl-SO2- | 676 | |
| 444 | 2,4,5-Trimethylphenyl- | 4-Acetylaminophenyl-SO2- | 639 | |
| 445 | 3,4-Dichlorophenyl- | 3,4-Dichlorophenyl-SO2- | 676 | |
| 446 | 3,4-Dimethoxyphenyl- | 4-Methylphenyl-SO2- | 614 | |
| 447 | 4-Fluorophenyl- | 4-Methylphenyl-SO2- | 572 | |
| 448 | 4-Bromophenyl- | 2-Naphthyl-SO2- | 668 | |
| 449 | 2-Chlorophenyl- | 2-Naphthyl-SO2- | 624 | |
| 450 | 2-Methylphenyl- | 2-Naphthyl-SO2- | 604 | |
| 451 | 2-Methoxyphenyl- | 2-Naphthyl-SO2- | 620 | |
| 452 | Phenyl- | 2-Naphthyl-SO2- | 590 | |
| 453 | 2,6-Diethylphenyl- | Phenyl-SO2- | 596 | |
| 454 | 2,6-Diisopropylphenyl- | Phenyl-SO2- | 624 | |
| 455 | 2-Biphenylyl- | 4-Methylphenyl-SO2- | 630 | |
| 456 | 2-Naphthyl- | 2-Naphthyl-SO2- | 640 | |
| 457 | 4-Methylphenyl- | 2-Naphthyl-SO2- | 604 | |
| 458 | 2,4-Dimethoxyphenyl- | 4-Methylphenyl-SO2- | 614 | |
| 459 | 2,5-Dimethylphenyl- | 4-Acetylaminophenyl-SO2- | 625 | |
| 460 | 2-Chloro-4-methoxyphenyl- | 4-Chlorophenyl-SO2- | 638 | |
| 461 | 2-Methoxy-5-methylphenyl- | Phenyl-SO2- | 584 | |
| 462 | 3-Methoxy-4-methylphenyl- | Phenyl-SO2- | 584 | |
| 463 | 2-Methoxyphenyl- | 4-Chlorophenyl-SO2- | 604 | |
| 464 | 3-Hydroxyphenyl- | 4-Chlorophenyl-SO2- | 590 | |
| 465 | 3,4-Dichlorophenyl- | 4-Chlorophenyl-SO2- | 642 | |
| 466 | 3-Acetylphenyl- | 4-Chlorophenyl-SO2- . | 616 | |
| 467 | 2,6-Diethylphenyl- | 4-Methylphenyl-SO2- | 610 | |
| 468 | 5-Chloro-2-methoxyphenyl- | 4-Acetylaminophenyl-SO2- | 661 | |
| 469 | 5-Chloro-2-methylphenyl- | Phenyl-SO2- | 588 | |
| 470 | 2-Chloro-5-trifluoromethylphenyl- | 4-Chlorophenyl-SO2- | 676 | |
| 471 | 2-Acetylphenyl- | 2,4-Difluorophenyl-SO2- | 618 | |
| 472 | 1-Acetylamino-2-naphthyl- | 4-Methylphenyl-SO2- | 661 | |
| 473 | 4-Ethylphenyl- | 4-Chlorophenyl-SO2- | 602 | |
| 474 | 2,5-Dichlorophenyl- | 4-Methoxyphenyl-SO2- | 638 | |
| 475 | 2,4-Difluorophenyl- | 4-Fluoro-3,5-dimethlyphenyl-SO2- | 622 | |
| 476 | 4-Trifluoromethoxyphenyl- | 2,5-Dimethoxyphenyl-SO2- | 684 | |
| 477 | 2-Chlorophenyl- | 3,4-Dimethoxyphenyl-SO2- | 634 | |
| 478 | 2-Methyl-1-naphthyl- | Phenyl-SO2- | 604 | |
| 479 | 2,6-dimethylphenyl- | 4-Acetylaminophenyl-SO2- | 625 | |
| 480 | 1,3-Dihydro-1-oxo-benzo[c]furan-6-yl- | 4-Chlorophenyl-SO2- | 630 | |
| 481 | Benzyl- | (5-methyl-2-thienyl)-CH-(CH3)-CO- | 528 | 62.6 |
| 482 | 3-Nitro-benzyl- | (2-Thienyl)-CH2-CO- | 545 | |
| 483 | Neopentyl- | (2-Thienyl)-CH2-CO- | 480 | |
| 484 | Isopropyl- | (2-Thienyl)-CH2-CO- | 452 | |
| 485 | 2-Ethoxy-benzyl- | (2-Thienyl)-CH2-CO- | 544 | |
| 486 | 3-Nitro-benzyl- | (3-Thienyl)-CH2-CO- | 545 | |
| 487 | Neopentyl- | (3-Thienyl)-CH2-CO- | 480 | |
| 488 | Isopropyl- | (3-Thienyl)-CH2-CO- | 452 | |
| 489 | 2-Ethoxy-benzyl- | (3-Thienyl)-CH2-CO- | 544 | |
| 490 | 2,2,2-Trifluoroethyl- | (3-Thienyl)-CH2-CO- | 492 | |
| 491 | 2-(3-Trifluoromethyl-phenyl)-ethyl- | (3-Thienyl)-CH2-CO- | 582 | |
| 492 | 3-Nitro-benzyl- | (2-Fluorophenyl)-CH2-CO- | 557 | |
| 493 | Neopentyl- | (2-Fluorophenyl)-CH2-CO- | 492 | |
| 494 | Isopropyl- | (2-Fluorophenyl)-CH2-CO- | 464 | |
| 495 | 2-Ethoxy-benzyl- | (2-Fluorophenyl)-CH2-CO- | 556 | |
| 496 | 2-(3-Trifluoromethyl-phenyl)-ethyl- | (2-Fluorophenyl)-CH2-CO- | 594 | |
| 497 | 1-Indanyl- | Phenyl-CH2-CO- | 520 | |
| 498 | 3-Nitro-benzyl- | Phenyl-CH2-CO- | 539 | |
| 499 | Neopentyl- | Phenyl-CH2-CO- | 474 | |
| 500 | Isopropyl- | Phenyl-CH2-CO- | 446 | |
| 501 | 2,2,2-Trifluoroethyl- | Phenyl-CH2-CO- | 486 | |
| 502 | 2-(3-Trifluoromethyl-phenyl)-ethyl- | Phenyl-CH2-CO- | 576 | |
| 503 | 1-indanyl- | (4-Chlorophenyl)-CH2-CO- | 554 | |
| 504 | 3-Nitro-benzyl- | (4-Chlorophenyl)-CH2-CO- | 573 | |
| 505 | Neopentyl- | (4-Chlorophenyl)-CH2-CO- | 508 | |
| 506 | Isopropyl- | (4-Chlorophenyl)-CH2-CO- | 480 | |
| 507 | 2-Ethoxy-benzyl- | (4-Chlorophenyl)-CH2-CO- | 572 | |
| 508 | 2-(3-Trifluoromethyl-phenyl)-ethyl- | (4-Chlorophenyl)-CH2-CO- | 610 | |
| 509 | 3-Nitro-benzyl- | 4-Chloro-phenyl-CO- | 559 | |
| 510 | Isopropyl- | 4-Chloro-phenyl-CO- | 466 | |
| 511 | 2-Ethoxy-benzyl- | 4-Chloro-phenyl-CO- | 558 | |
| 512 | 2-(3-Trifluoromethyl-phenyl)-ethyl- | 4-Chloro-phenyl-CO- | 596 | |

| No. | R2 | R1-X | R7 | MS (ES-):M-1). | Restaktivität(%) des NCBE bei 10 µm) |
|---|---|---|---|---|---|
| 513 | Benzyl- | (2-Thienyl)-CH2-CO- | 4-Methyl- | 514 | 88.6 |
| 514 | (1-Naphthyl)-methyl- | (2-Thienyl)-CH2-CO- | 4-Methyl- | 564 | 74.9 |
| 515 | (2-Naphthyl)-methyl- | (2-Thienyl)-CH2-CO- | 4-Methyl- | 564 | 68.9 |
| 516 | 2-Chloro-benzyl- | (2-Thienyl)-CH2-CO- | 4-Methyl- | 548 | 98.7 |
| 517 | (2-Thienyl)-methyl- | (2-Thienyl)-CH2-CO- | 4-Methyl- | 520 | 86.2 |
| 518 | 2-Phenyl-ethyl- | (2-Thienyl)-CH2-CO- | 4-Methyl- | 528 | 93.5 |
| 519 | 2-Fluoro-benzyl- | (2-Thienyl)-CH2-CO- | 4-Methyl- | 532 | 95.6 |
| 520 | 2,3-Dichloro-benzyl- | (2-Thienyl)-CH2-CO- | 4-Methyl- | 582 | 85.9 |
| 521 | Benzyl- | (2-Thienyl)-CH(CH3)-CO- | 4-Methyl- | 528 | 88.2 |
| 522 | (1-Naphthyl)-methyl- | (2-Thienyl)-CH(CH3)-CO- | 4-Methyl- | 578 | 85.6 |
| 523 | (2-Naphthyl)-methyl- | (2-Thienyl)-CH(CH3)-CO- | 4-Methyl- | 578 | 92.3 |
| 524 | 2-Chloro-benzyl- | (2-Thienyl)-CH(CH3)-CO- | 4-Methyl- | 562 | |
| 525 | (2-Thienyl)-methyl- | (2-Thienyl)-CH(CH3)-CO- | 4-Methyl- | 534 | 74.0 |
| 526 | 2-Phenyl-ethyl- | (2-Thienyl)-CH(CH3)-CO- | 4-Methyl- | 542 | 64.9 |
| 527 | 2-Fluoro-benzyl- | (2-Thienyl)-CH(CH3)-CO- | 4-Methyl- | 546 | 67.5 |
| 528 | 2,3-Dichloro-benzyl- | (2-Thienyl)-CH(CH3)-CO- | 4-Methyl- | 596 | 72.0 |
| 529 | Benzyl- | (4-Chlorophenyl)-CH2-CO- | 4-Methyl- | 542 | 89.4 |
| 530 | (1-Naphthyl)-methyl- | (4-Chlorophenyl)-CH2-CO- | 4-Methyl- | 592 | 89.8 |
| 531 | (2-Naphthyl)-methyl- | (4-Chlorophenyl)-CH2-CO- | 4-Methyl- | 592 | 75.6 |
| 532 | 2-Chloro-benzyl- | (4-Chlorophenyl)-CH2-CO- | 4-Methyl- | 576 | 71.0 |
| 533 | (2-Thienyl)-methyl- | (4-Chlorophenyl)-CH2-CO- | 4-Methyl- | 548 | 75.5 |
| 534 | 2-Phenyl-ethyl- | (4-Chlorophenyl)-CH2-CO- | 4-Methyl- | 556 | 80.0 |
| 535 | 2-Fluoro-benzyl- | (4-Chlorophenyl)-CH2-CO- | 4-Methyl- | 560 | 84.0 |
| 536 | 2,3-Dichloro-benzyl- | (4-Chlorophenyl)-CH2-CO- | 4-Methyl- | 610 | 76.3 |
| 537 | Benzyl- | (3-Thienyl)-CH2-CO- | 4-Methyl- | 514 | 54.4 |
| 538 | (1-Naphthyl)-methyl- | (3-Thienyl)-CH2-CO- | 4-Methyl- | 564 | 80.3 |
| 539 | (2-Naphthyl)-methyl- | (3-Thienyl)-CH2-CO- | 4-Methyl- | 564 | 65.2 |
| 540 | 2-Chloro-benzyl- | (3-Thienyl)-CH2-CO- | 4-Methyl- | 548 | 64.6 |
| 541 | (2-Thienyl)-methyl- | (3-Thienyl)-CH2-CO- | 4-Methyl- | 520 | 52.8 |
| 542 | 2-Phenyl-ethyl- | (3-Thienyl)-CH2-CO- | 4-Methyl- | 528 | 67.4 |
| 543 | 2-Fluoro-benzyl- | (3-Thienyl)-CH2-CO- | 4-Methyl- | 532 | 58.3 |
| 544 | 2,3-Dichloro-benzyl- | (3-Thienyl)-CH2-CO- | 4-Methyl- | 582 | 82.2 |
| 545 | Benzyl- | Cyclohexyl-CH2-CO- | 4-Methyl- | 514 | 70.1 |
| 546 | (1-Naphthyl)-methyl- | Cyclohexyl-CH2-CO- | 4-Methyl- | 564 | 63.6 |
| 547 | (2-Naphthyl)-methyl- | Cyclohexyl-CH2-CO- | 4-Methyl- | 564 | 76.5 |
| 548 | 2-Chloro-benzyl- | Cyclohexyl-CH2-CO- | 4-Methyl- | 548 | 65.2 |
| 549 | (2-Thienyl)-methyl- | Cyclohexyl-CH2-CO- | 4-Methyl- | 520 | 67.3 |
| 550 | 2-Phenyl-ethyl- | Cyclohexyl-CH2-CO- | 4-Methyl- | 528 | 63.3 |
| 551 | 2-Fluoro-benzyl- | Cyclohexyl-CH2-CO- | 4-Methyl- | 532 | 82.6 |
| 552 | 2,3-Dichloro-benzyl- | Cyclohexyl-CH2-CO- | 4-Methyl- | 582 | 61.5 |
| 553 | Benzyl- | (2-Thienyl)-CH2-CO- | 5-Chloro- | 534 | 85.7 |

### Beispiel 554

### 4'-{[Benzyl-(2-thiophen-2-yl-acetyl)amino]-methyl}-3'-chloro-biphenyl-2-sulfonylcyanamid

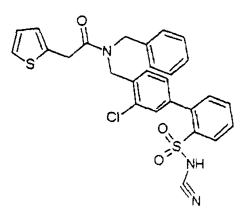

### a) 4-Brom-1-brommethyl-2-chlor-benzol

7.1 ml 4-Brom-2-chlortoluol werden in 20 ml Chlorbenzol gelöst und bei 130 □C portionsweise mit einer Mischung aus 9.4 g N-Bromsuccinimid und 200 mg Dibenzoylperoxid versetzt. 30 Minuten wird unter Rückfluß gekocht, nach dem Abkühlen mit 100 ml CH₂Cl₂ verdünnt und je 1 mal mit 50 ml einer gesättigten wäßrigen Na₂SO₃-Lösung und 100 ml einer gesättigten wäßrigen NaHCO₃-Lösung gewaschen.Über Na₂SO₄ wird getrocknet und das Solvens im Vakuum entfernt. Man erhält 11.0 g eines blaßgelben Öls.
R_{f} (EE/HEP 1:8) = 0.49 MS (DCI) : 283 (M+H)⁺

### b) Benzyl-(4-brom-2-chlor-benzyl)amin

765 µl Benzylamin werden in 10 ml THF (wasserfrei) gelöst und bei 0°C mit 1.0 g 4-Brom-1-brommethyl-2-chlorbenzol versetzt und 4 h bei RT gerührt. Anschließend werden 100 ml einer halbgesättigten wäßrigen Na₂CO₃-Lösung zugesetzt und drei mal mit je 100 ml EE extrahiert. Über MgSO₄ wird getrocknet und an Kieselgel mit DIP chromatographiert. Man erhält 590 mg eines farblosen Öls.
R_{f}(DIP) = 0.30 MS (DCI) : 310 (M+H)⁺

### c) 2-Thiophen-2-yl-essigsäure-benzyl-(4-brom-2-chlor-benzyl)amid

580 mg Benzyl-(4-brom-2-chlor-benzyl)amin werden in 10 ml CH₂Cl₂ (wasserfrei) gelöst und zunächst mit 300 µl Pyridin, dann mit 330 mg 2-Thiophen-2-ylacetylchlorid versetzt. 4 h wird bei RT gerührt, dann mit 100 ml CH₂Cl₂ verdünnt und mit 50 ml einer gesättigten wäßrigen Na₂CO₃-Lösung gewaschen. Über MgSO₄ wird getrocknet und das Solvens im Vakuum entfernt. An Kieselgel wird mit DIP chromatographiert und man erhält 490 mg eines farblosen Öls.
R_{f}(DIP) = 0.56 MS (ES): 434 (M+H)⁺

### d) 4'-{[Benzyl-(2-thiophen-2-yl-acetyl)amino]-methyl}-3'-chloro-biphenyl-2-sulfonsäure-t-butylamid

480 mg 2-Thiophen-2-yl-essigsäure-benzyl-(4-brom-2-chlor-benzyl)amid, 426 mg N-*t*-Butyl-2-dihydroxyboran-2-yl-benzolsulfonamid (J.Med.Chem. 1997, 40, 547), 26.2 mg Triphenylphosphin, 11.2 mg Pd(II)-acetat und 133 mg Na₂CO₃ werden in 1 ml Wasser, 0.5 ml EtOH sowie 5 ml Toluol suspendiert und 2 h lang unter Rückfluß gekocht. Man läßt abkühlen, anschließend werden die flüchtigen Bestandteile werden im Vakuum entfernt. Der Rückstand wird mit 2 ml CH₂Cl₂ aufgenommen und an Kieselgel mit DIP chromatographiert. Man erhält 520 mg eines farblosen, amorphen Feststoffs.
R_{f}(DIP) = 0.20 MS (ES) : 567 (M+H)⁺

### e) 4'-{[Benzyl-(2-thiophen-2-yl-acetyl)amino]-methyl}-3'-chloro-biphenyl-2-sulfonamid

510 mg 4'-{[Benzy(-(2-thiophen-2-yl-acetyl)amino]-methyl}-3'-chloro-biphenyl-2-sulfonsäure-*t*-butylamid und 110 µl Anisol werden in 3 ml Trifluoressigsäure bei RT 18 h stehen gelassen. Anschließend werden die flüchtigen Bestandteile im Vakuum entfernt, in 5 ml Toluol aufgenommen und erneut flüchtige Bestandteile im Vakuum entfernt. Man erhält 586 mg eines farblosen Öls, das ohne Reinigung weiter verwendet wird.
R_{f}(DIP) = 0.12 MS (ES) : 511 (M+H)⁺

### f) 4'-{[Benzyl-(2-thiophen-2-yl-acetyl)amino]-methyl}-3'-chloro-biphenyl-2-sulfonylcyanamid

586 mg 4'-{[Benzyl-(2-thiophen-2-yl-acetyl)amino]-methyl}-3'-chloro-biphenyl-2-sulfonamid, 372 mg K₂CO₃ sowie 180 µl einer 5 molaren Lösung von BrCN in Acetonitril werden 1.5 h unter Rückfluß gekocht. Nach dem Abkühlen wird das gesamte Reaktionsgemisch an Kieselgel mit EE/MeOH 10:1 chromatographiert. Man erhält 181 mg eines farblosen, amorphen Feststoffs.
R_{f}(EE/MeOH 10:1) = 0.22 IR (CN) : 2172.5 cm⁻¹ MS (ES) : 536
(M+H)⁺ Restaktivität des NCBE bei 10 µM : 15%

Die Titelverbindung des Beispiels 555 wurde analog Beispiel 554 synthetisiert.

### Beispiel 555

### 4'-{[Benzyl-(2-thiophen-2-yl-acetyl)amino]-methyl}-3'-methylsulfonyl-biphenyl-2-sulfonylcyanamid

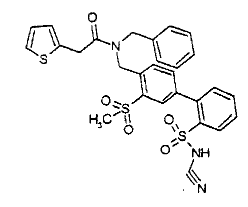

### a) 2-Brom-5-methyl-benzolsulfonyl chlorid

40 g 4-Bromtoluol werden unter Rühren langsam bei -10 °C in 250 ml Chlorsulfonsäure eingetragen. 30 Minuten wird bei dieser Temperatur nachgerührt, man läßt auf 0 °C erwärmen und gießt auf überschüssiges Eis. Das Produkt wird abgesaugt und mit wenig Wasser gewaschen. Über P₄O₁₀ wird im Vakuum getrocknet und man erhält 63 g eines farblosen Feststoffs, der direkt weiter umgesetzt wird.

### b) 2-Brom-5-methyl-benzolsulfinsäure

37.6 g Natriumsulfit werden in 500 ml Wasser gelöst und auf 70 ° C erwärmt. Bei dieser Temperatur werden portionsweise 62 g 2-Brom-5-methyl-benzolsulfonyl chlorid zugegeben. Hierbei wird gleichzeitig eine 10 N wäßrige NaOH-Lösung zugetropft, sodaß der pH-Wert der Lösung zwischen pH=9 und pH=10 gehalten wird. 1.5 Stunden wird bei 70 °C nachgerührt, die Lösung abfiltriert und anschließend im Eisbad mit einer gesättigten wäßrigen HCI-Lösung auf pH=0 gestellt. 30 Minuten wird nachgerührt, anschließend das Produkt abfiltriert, mit wenig Wasser nachgewaschen und getrocknet. Man erhält 49.6 g weißer Kristalle,
mp 120-122 °C. MS (ES) : 236 (M+H)⁺

### c) Natrium-2-brom-5-methyl-benzolsulfinat

49.6 g 2-Brom-5-methyl-benzolsulfinsäure werden in 400 ml Methanol gelöst und mit einer äquimolaren Menge NaOH in 50 ml Wasser versetzt. 3 Stunden wird bei RT nachgerührt, die Lösung abfiltriert und anschließend die Solventien im Vakuum entfernt. Zuletzt werden Wasserreste azeotrop mit 50 ml Touol entfernt. Der feste Rückstand wird über P₄O₁₀ wird im Vakuum getrocknet und man erhält 54.0 g Produkt, mp 288-290 °C (unter Zersetzung).

### d) 1-Brom-2-methansulfonyl-4-methyl-benzol

54.0 g Natrium-2-brom-5-methyl-benzolsulfinat werden in 300 ml wasserfreiem DMF suspendiert und mit 45.7 ml Methyliodid versetzt. Dabei steigt die Temperatur der Lösung auf 50 °C an. 3 Stunden wird bei 50 °C nachgerührt und das DMF im Vakuum entfernt. Der Rückstand wird mit 500 ml Wasser verrührt, 1 Stunde bei 0 °C nachgerührt und abfiltriert. Das Produkt wird mit Wasser gewaschen, getrocknet und aus 400 ml HEP/250 ml EE mit Aktivkohle umkristallisiert. Man erhält 27.0 g farbloser Kristalle, mp 110-114 °C.
R_{f}(EE/HEP 1:4) = 0.09 MS (DCI) : 250 (M+H)⁺

### e) 1-Brom-4-brommethyl-2-methansulfonyl-benzol

9.9 g 1-Brom-2-methansulfonyl-4-methyl-benzol werden in 100 ml Chlorbenzol aufgenommen, 77 mg Benzoylperoxid sowie 7.1 g N-Bromsuccinimid zugegeben und 1 Stunde unter Rückfluß gekocht. Anschließend wird das Solvens im Vakuum entfernt, der Rückstand in 100 ml CH₂Cl₂ aufgenommen und 2 mal mit 50 ml einer gesättigten wäßrigen Na₂CO₃-Lösung und 1 mal mit 50 ml Wasser gewaschen. Über Na₂SO₄ wird getrocknet und das Solvens im Vakuum entfernt. Der Rückstand wird aus 80 ml HEP/30 ml EE umkristallisiert und man erhält 6.9 g eines blaßgelben Feststoffs, mp 120-124 °C.
R_{f}(EE/HEP 1:2) = 0.38 MS (DCl) : 329 (M+H)⁺

### f) Benzyl-(4-brom-2-methylsulfonyl-benzyl)amin

652 µl Benzylamin werden in 10 ml THF (wasserfrei) gelöst und bei 0°C langsam 1.0 g 1-Brom-4-brommethyl-2-methylsulfonyl-benzol zugegeben. 4 h wird bei RT gerührt, anschließend mit 100 ml einer gesättigten wäßrigen Na₂CO₃-Lösung versetzt und 2 mal mit je 100 ml EE extrahiert. Über MgSO₄ wird getrocknet und das Solvens im Vakuum entfernt. Chromatographie an Kieselgel mit MTB/DIP 1:1 liefert 510 mg eines farblosen Öls.
R_{f}(DIP) = 0.10 MS (ES) : 354 (M+H)⁺

### g) 4'-{[Benzyl-(2-thiophen-2-yl-acetyl)amino]-methyl}-3'-methylsulfonyl-biphenyl-2-sulfonylcyanamid

R_{f}(EE/MeOH 10:1) = 0.21 IR (CN) : 2175.3 cm⁻¹ MS (ES) : 580 (M+H)⁺
Restaktivität des NCBE bei 10 µM : 31%

### Beispiel 556

### 4'-{[Benzyl-(thiophen-2-sulfonyl)amino]-methyl}-3'-chlor-biphenyl-2-sulfonylcyanamid

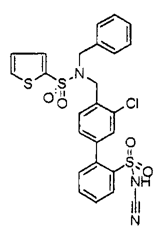

### a) Thiophen-2-sulfonsäure-benzyl-(4-brom-2-chlor-benzyl)amid

2.0 g Benzyl-(4-brom-2-chlor-benzyl)amin (Beispiel X b) und 1.0 g Pyridin werden in 20 ml CH₂Cl₂ gelöst und bei RT langsam mit 1.4 g Thiophen-2-sulfonsäurechlorid versetzt. 4 h wird bei RT gerührt, anschließend mit 200 ml EE verdünnt und 2 mal mit je 100 ml einer 5% wäßrigen Natriumhydrogensulfat-Lösung sowie 2 mal mit je 50 ml einer gesättigten wäßrigen Natriumcarbonat-Lösung gewaschen. Über Magnesiumsulfat wird getrocknet und das Solvens im Vakuum entfernt. Chromatographie an Kieselgel mit DIP liefert 1.5 g eines farblosen Öls.
R_{f}(DIP) = 0.21 MS (FAB) : 456 (M+H)⁺

Die weitere Umsetzung zur Titelverbindung des Beispiels 556 erfolgt analog Beispiel 554 d)-f).

### b) 4'-{[Benzyl-(thiophen-2-sulfonyl)amino]-methyl}-3'-chlor-biphenyl-2-sulfonylcyanamid

R_{f}(EE/MeOH 10:1) = 0.17 IR (CN) : 2178.5cm⁻¹ MS (FAB) : 580 (M+Na)⁺
Restaktivität des NCBE bei 10 µM : 0%

Die Titelverbindung des Beispiel 557 wird analog Beispiel 556 aus der Titelverbindung des Beispiel 555 b) synthetisiert:

### Beispiel 557

### 4'-{[Benzyl-(thiophen-2-sulfonyl)amino]-methyl}-3'-methylsulfonyl-biphenyl-2-sulfonylcyanamid

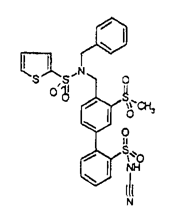

R_{f}(EE/MeOH 10:1) = 0.28 IR (CN) : 21750 cm⁻¹ MS (ES) : 602 (M+H)⁺
Restaktivität des NCBE bei 10 µM : 14%

### Beispiel 558

### 4'-{[Benzyl-(thiophen-2-sulfonyl)amino]-methyl}-3'-(2-methoxy)ethoxy-biphenyl-2-sulfonylcyanamid

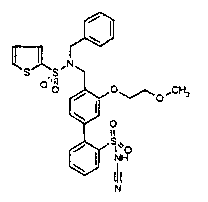

### a) 4-Methyl-3-(2-methoxy)ethoxy-anilin

22.0 g 3-Hydroxy-4-methyl-anilin, 24.9 g 2-Bromethylmethylether und 233 g Cs₂CO₃ werden in 570 ml DMF gelöst und 8 h bei 40°C gerührt. 3 I einer 10% wäßrigen Natriumhydrogencarbonat-Lösung werden zugegeben, 6 mal mit je 750 ml EE extrahiert und 2 mal mit je 1 I einer 10% wäßrigen Natriumhydrogencarbonat-Lösung gewaschen.Über Natriumsulfat wird getrocknet und das Solvens im Vakuum entfernt. Man erhälöt 32.9 g eines gelben Öls, das so weiter eingesetzt wird.
R_{f}(EE/HEP 1:1 ) = 0.33

### b) 4-Methyl-3-(2-methoxy)ethoxy-brombenzol

32.8 g 4-Methyl-3-(2-methoxy)ethoxy-anilin werden in 660 ml einer halbgesättigten wäßrigen HBr-Lösung suspendiert und bei 0°C eine Lösung von 12.5 g NaNO₃ in 25 ml Wasser langsam zugetropft. 30 Minuten wird bei 0°C nachgerührt und anschließend diese Lösung zu einer auf 50°C erwärmten Lösung von 51.9 g CuBr in 490 ml einer gesättigten wäßrigen HBr-Lösung langsam zugegeben. Das Reaktionsgemisch wird anschließend über einen Zeitraum von 6 h langsam von 50°C auf 70°C erwärmt. Nach anschließendem Abkühlen wird 4 mal mit je 500 ml Diethylether extrahiert, mit 500 ml einer gesättigten wäßrigen NaCI-Lösung gewaschen und über Natriumsulfat getrocknet. Chromatographie an Kieselgel mit EE/HEP 1:8 liefert 15.4 g eines farblosen Öls.
R_{f}(EE/HEP 1:1) = 0.41 MS (DCI) : 245 (M+H)⁺

### c) 4-Brommethyl-3-(2-methoxy)ethoxy-brombenzol

15.3 g 4-Methyl-3-(2-methoxy)ethoxy-brombenzol werden in 300 ml Chlorbenzol gelöst und unter Rückfluß ein Gemisch aus 11.1 g N-Bromsuccinimid und 125 mg Benzoylperoxid portionsweise zugegeben. 24 h wird unter Rückfluß gekocht, das Solvens im Vakuum entfernt und anschließend mit 500 ml CH₂Cl₂ aufgenommen. Mit zunächst 200 ml einer gesättigten wäßrigen Na₂SO₄-Lösung, anschließend mit 100 ml einer gesättigten wäßrigen Natriumcarbonat-Lösung wird gewaschen. Über Natriumsulfat wird getrocknet und das Solvens im Vakuum entfernt.
Chromatographie an Kieselgel mit EE/HEP 1:15 liefert 12.8 g eines blaßgelben Öls.
R_{f}(EE/HEP 1:4) = 0.42 MS (DCl) : 323 (M+H)⁺

### d) Benzyl-[4-brom-2-(2-methoxy)ethoxy-benzyl)amin

2.4 ml Benzylamin werden in 20 ml THF (wasserfrei) gelöst und bei 0°C langsam mit 3.2 g 4-Brommethyl-3-(2-methoxy)ethoxy-brombenzol versetzt. 19 h wird bei RT gerührt, anschließend mit 200 ml EE verdünnt und mit 100 ml einer gesättigten wäßrigen Natriumcarbonat-Lösung gewaschen. Über Magnesiumsulfat wird getrocknet und das Solvens im Vakuum entfernt. Chromatographie an Kieselgel mit MTB liefert 1.3 g eines farblosen Öls.
R_{f}(MTB) = 0.20 MS (DCl): 350 (M+H)⁺

### e) Thiophen-2-sulfonsäure-benzyl-[4-brom-2-(2-methoxy)ethoxy-benzyl]amid

1.2 g Benzyl-[4-brom-2-(2-methoxy)ethoxy-benzyl)amin, 713 mg Thiophen-2-sulfonsäurechlorid und 430 µl Pyridin werden in 50 ml CH₂Cl₂ gelöst und 17 h bei RT gerührt. Anschließend wird mit 100 ml CH₂Cl₂ verdünnt und zunächst 2 mal mit je 50 ml einer 5% wäßrigen Natriumhydrogensulfat-Lösung und dann mit 50 ml einer gesättigten wäßrigen Natriumcarbonat-Lösung gewaschen. Über Magnesiumsulfat wird getrocknet und das Solvens im Vakuum entfernt. Chromatographie an Kieselgel mit DIP liefert 900 mg eines farblosen Öls.
R_{f}(DIP) = 0.2 MS (ES) : 496 (M+H)⁺

### f) Dihydroxyboran-2-yl-benzolsulfonamid

50 g N-*t*-Butyl-2-dihydroxyboran-2-yl-benzolsulfonamid (J.Med.Chem. 1997, 40, 547) und 23.3 g Anisol werden in 500 ml Trifluoressigsäure gelöst und 2 Tage bei RT stehen gelassen. Die flüchtigen Bestandteile werden im Vakuum entfernt, in 100 ml Wasser aufgenommen und erneut die flüchtigen Bestandteile im Vakuum entfernt. Schließlich wird in 100 ml Toluol aufgenommen und nochmals die flüchtigen Bestandteile im Vakuum entfernt. Man erhält 56 g eines farblosen Öls, das ohne Reinigung weiter eingesetzt wird.
R_{f}(MTB) ~ 0.4

### g) Dihydroxyboran-2-yl-benzolsulfonsäure-dimethylaminomethylenamid

20 g Dihydroxyboran-2-yl-benzolsulfonamid und 66 ml Dimethylformamiddimethylacetal werden in 200 ml DMF (wasserfrei) gelöst und 18 h bei RT stehen gelassen. Das Reaktionsgemisch wird auf 1.5 I Wasser gegossen und 4 mal mit je 500 ml EE extrahiert. Über Magnesiumsulfat wird getrocknet und das Solvens im Vakuum entfernt. Kristallisation aus 100 ml EE liefert 5.2 g farbloser Kristalle, mp 175°C (unter Zersetzung).
R_{f}(EE) = 0.25

### h) {[Benzyl-(thiophen-2-sulfonyl)amino]-methyl}-3'-(2-methoxy)ethoxy-biphenyl-2-sulfonsäure-dimethylaminomethylenamid

900 mg Thiophen-2-sulfonsäure-benzyl-[4-brom-2-(2-methoxy)ethoxy-benzyl]amid, 1.4 g Dihydroxyboran-2-yl-benzolsulfonsäure-dimethylaminomethylenamid, 47 mg Triphenylphosphin, 20 mg Pd(II)acetat und 575 mg Na₂CO₃ werden in 30 ml Toluol, 5 ml Wasser sowie 5 ml EtOH suspendiert. 6 h wird unter Rückfluß gekocht, dann läßt man abkühlen und verdünnt mit 100 ml EE. 2 mal wird mit je 50 ml einer gesättigten wäßrigen NaCl-Lösung gewaschen. Über Magnesiumsulfat wird getrocknet und das Solvens im Vakuum entfernt. Chromatographie an Kieselgel mit MTB liefert 560 mg eines farblosen, viskosen Öls.
R_{f}(MTB) = 0.13 MS (ES) : 628 (M+H)⁺

### i) {[Benzyl-(thiophen-2-sulfonyl)amino]-methyl}-3'-(2-methoxy)ethoxy-biphenyl-2-sulfonamid

550 mg {[Benzyl-(thiophen-2-sulfonyl)amino]-methyl}-3'-(2-methoxy)ethoxybiphenyl-2-sulfonsäure-dimethylaminomethylenamid werden in 5 ml EtOH und 5 ml einer gesättigten wäßrigen HCl-Lösung 1 h unter Rückfluß gekocht. 100 ml einer 10% wäßrigen Natriumhydrogencarbonat-Lösung werden zugegeben und 3 mal mit je 100 ml EE extrahiert. Über Magnesiumsulfat wird getrocknet und das Solvens im Vakuum entfernt. Chromatographie an Kieselgel mit MTB/DIP 1:1 liefert 188 mg eines farblosen Öls.
R_{f}(MTB/DIP 1:1) = 0.33 MS (ES) : 573 (M+H)⁺

### j) 4'-{[Benzyl-(thiophen-2-sulfonyl)amino]-methyl}-3'-(2-methoxy)ethoxy-biphenyl-2-sulfonylcyanamid

180 mg {[Benzyl-(thiophen-2-sulfonyl)amino]-methyl}-3'-(2-methoxy)ethoxybiphenyl-2-sulfonamid, 63 µl einer 5 molaren BrCN-Lösung in Acetonitril sowie 131 mg K₂CO₃ werden in 3 ml Acetonitril (wasserfrei) suspendiert und 2 h unter Rückfluß gekocht. Man läßt abkühlen und chromatorgraphiert das gesamte Reaktionsgemisch an Kieselgel mit EE/MeOH 10:1 und erhält 149 mg eines amorphen Feststoffs.
R_{f}(EE/MeOH 10:1) = 0.13 IR (CN) : 2175.3 cm⁻¹ MS (FAB) : 598 (M+H)⁺
Restaktivität des NCBE bei 10 µM : 30%

### Beispiel 559

### 4'-{[Benzyl-(thiophen-2-sulfonyl)amino]-methyl}-3'-(2-methoxy)ethoxy-biphenyl-2-sulfonylcyanamid

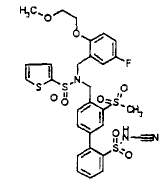

### a) 4-Fluor-1-(2-methoxy-ethoxy)-2-methyl-benzol

10 g 4-Fluor-2-methyl-phenol, 7.5 ml 1-Brom-2-methoxy-ethan sowie 22 g K₂CO₃ werden in 200 ml DMF (wasserfrei) suspendiert und 12 h bei 120°C gerührt. Man läßt abkühlen und entfernt das Solvens im Vakuum. Mit 400 ml MTB wird aufgenommen und 3 mal mit je 200 ml einer 10% wäßrigen NaOH-Lösung sowie 1 mal mit 100 ml einer gesättigten wäßrigen NaCl-Lösung gewaschen. Über Magnesiumsulfat wird getrocknet und das Solvens im Vakuum entfernt. Man erhält 10.4 g eines blaßgelben Öls.
R_{f}(EE/HEP) = 0.39 MS (DCI) : 185 (M+H)⁺

### b) 2-Brommethyl-4-fluor-1-(2-methoxy-ethoxy)-benzol

10.4 g 4-Fluor-1-(2-methoxy-ethoxy)-2-methyl-benzol werden in 100 ml Chlorbenzol gelöst und bei Rückflußtemperatur portionsweise mit einer Mischung aus 10.1 g NBS und 200 mg Benzoylperoxid versetzt. 30 Minuten wird unter Rückfluß gekocht. Man läßt abkühlen und verdünnt mit 300 ml EE. Anschließend wird zunächst 1 mal mit 100 ml einer gesättigten wäßrigen Na₂SO₃-Lösung, dann 2 mal mit je 100 ml einer gesättigten wäßrigen Natriumcarbonat-Lösung gewaschen. Über Magnesiumsulfat wird getrocknet und das Solvens im Vakuum entfernt. Chromatographie an Kieselgel mit EE/HEP 1:4 liefert 5.2 g eines farblosen Öls.
R_{f}(EE/HEP 1:4) = 0.20 MS (DCI) : 262 (M+H)⁺

### b1) 1-Brom-4-brommethyl-2-methansulfonyl-benzol

9.9 g 1-Brom-2-methansulfonyl-4-methyl-benzol werden in 100 ml Chlorbenzol aufgenommen, 77 mg Benzoylperoxid sowie 7.1 g N-Bromsuccinimid zugegeben und 1 Stunde unter Rückfluß gekocht. Anschließend wird das Solvens im Vakuum entfernt, der Rückstand in 100 ml CH₂Cl₂ aufgenommen und 2 mal mit 50 ml einer gesättigten wäßrigen Na₂CO₃-Lösung und 1 mal mit 50 ml Wasser gewaschen. Über Na₂SO₄ wird getrocknet und das Solvens im Vakuum entfernt. Der Rückstand wird aus 80 ml HEP/30 ml EE umkristallisiert und man erhält 6.9 g eines blaßgelben Feststoffs, mp 120-124 °C.
R_{f}(EE/HEP 1:2) = 0.38 MS (DCl) : 329 (M+H)⁺

### c) 2-(4-Brom-2-methansulfonyl-benzyl)-isoindol-1,3-dion

3.0 g 4-Brom-1-brommethyl-2-methanesulfonyl-benzol und 2.0 g Kalium-Phthalimid werden in 30 ml wässerfreiem DMF 1 h bei 100°C gerührt. Man läßt abkühlen, verdünnt mit 200 ml Wasser und rührt die Supension 30 Minuten lang bei RT. Das Produkt wird anschließend abfiltriert und man erhält 1.8 g eines farblosen
Feststoffs, mp 188-190°C. MS (ES) : 393 (M+H)⁺

### d) 4-Brom-2-methansulfonyl-benzylamin

1.8 g 2-(4-Brom-2-methansulfonyl-benzyl)-isoindol-1,3-dion und 1.5 ml Hydrazinhydrat werden in 30 ml EtOH zunächst 1 h bei 60°C gerührt, dann 4 h unter Rückfluß gekocht. Man läßt abkühlen, filtriert den Niederschlag ab und entfernt die flüchtigen Bestandteile des Filtrats im Vakuum. Der Rückstand wird mit 100 ml CH₂Cl₂ aufgenommen und erneut feste Bestandteile abfiltriert. Das Solvens des Filtrats wird im Vakuum entfernt und man erhält 1.3 g eines blaßgelben Öls.
R_{f}(EE/MeOH 10:1) = 0.10 MS (DCI) : 264 (M+H)⁺

### e) (4-Brom-2-methansulfonyl-benzyl)-[5-fluor-2-(2-methoxy-ethoxy)-benzyl]-amin

1.3 g 4-Brom-2-methansulfonyl-benzylamin und 1.4 ml Triethylamin werden in 20 ml THF (wasserfrei) gelöst und bei 0°C eine Lösung von 1.3 g 2-Brommethyl-4-fluor-1-(2-methoxy-ethoxy)-benzol in 5 ml THF (wasserfrei) zugetropft. 60 h wird bei RT gerührt, anschließend mit 200 ml EE verdünnt und 2 mal mit je 100 ml einer gesättigten wäßrigen Na₂CO₃-Lösung gewaschen. Über MgSO₄ wird getrocknet und das Solvens im Vakuum entfernt. Chromatographie an Kieselgel mit EE liefert 910 mg eines farblosen Öls.
R_{f}(EE) = 0.43 MS (ES) : 446 (M+H)⁺

Die weitere Umsetzung erfolgt analog Beispiel 558 f)-j)

### f) 4'-{[Benzyl-(thiophen-2-sulfonyl)amino]-methyl}-3'-(2-methoxy)ethoxy-biphenyl-2-sulfonylcyanamid

R_{f}(EE/MeOH 10:1) = 0.33 IR (CN) : 2174.3 cm⁻¹ MS (ES) : 694 (M+H)⁺
Restaktivität des NCBE bei 10 µM : 12%

Die Titelverbindung des Beispiels 560 wird analog Beispiel 558 synthetisiert:

### Beispiel 560

### 4'-{[Benzyl-2-(thiophen-2-yl)acetyl-amino]-methyl}-3'-(2-methoxy)ethoxy-biphenyl-2-sulfonylcyanamid

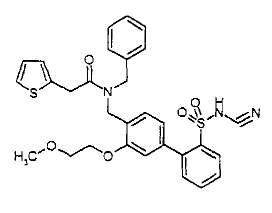

R_{f}(EE/MeOH 5:1) = 0.36 IR (CN) : 2175.0 cm⁻¹ MS (ES): 597 (M+H)⁺
mp 95°C (unter Zersetzung). Restaktivität des NCBE bei 10 µM : 8.0%.

### Beispiel 561

### 4'-{[Benzyl-2-(2-methyl-phenyl)acetyl-amino]-methyl}-biphenyl-2-sulfonylcyanamid

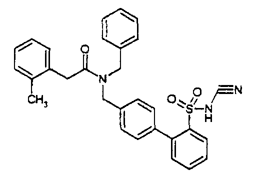

### a) 4'-(Benzylamino-methyl)-biphenyl-2-sulfonsäure-(dimethylamino)methylenamid

4.4 ml Benzylamin werden in 90 ml THF (wasserfrei) gelöst und bei 0°C portionsweise 7.6 g 4'-(Brommethyl)-*N*-[(dimethylamino)methylen]-(1,1'-biphenyl)-2-sulfonamid (J. Med. Chem. 1995, 38, 2357) zugegeben. 24 h wird bei RT gerührt, anschließend mit 500 ml EE verdünnt und 2 mal mit je 200 ml einer gesättigten wäßrigen Na₂CO₃-Lösung gewaschen. Über Na₂SO₄ wird getrocknet und das Solvens im Vakuum entfernt. Chromatographie an Kieselgel mit EE/MeOH 10:1 liefert 3.8 g eines farblosen Öls.
R_{f}(EE/MeOH 10:1) = 0.25 MS (FAB) : 408 (M+H)⁺

### b) o-Tolyl-acetylchlorid

4.8 g o-Tolylessigsäure werden in 36 ml SOCl₂ gelöst und 12 h unter Rückfluß gekocht. Die flüchtigen Bestandteile werden anschließend im Vakuum entfernt und der Rückstand danach 3 mal jeweils in 50 ml Toluol aufgenommen und die flüchtigen Bestandteile im Vakuum entfernt. Man erhält 6.6 g einer blaßgelben Flüssigkeit, die ohne Reinigung weiter eingesetzt wird.

### c) 4'-{[Benzyl-2-(2-methyl-phenyl)acetyl-amino]-methyl}-biphenyl-2-sulfonsäure-(dimethylamino)methylenamid

408 mg 4'-(Benzylamino-methyl )-biphenyl-2-sulfonsäure-(dimethylamino)methylenamid werden in 9 ml CH₂Cl₂ (wasserfrei) gelöst und bei RT zunächst 162 µl Pyridin, dann 220 mg o-Tolyl-acetylchlorid zugegeben. 24 h wird bei RT gerührt, mit 100 ml CH₂Cl₂ verdünnt und 3 mal mit je 50 ml einer gesättigten wäßrigen Na₂CO₃-Lösung gewaschen. Über Na₂SO₄ wird getrocknet und das Solvens im Vakuum entfernt. Chromatographie an Kieselgel mit EE/HEP 2:1 liefert 330 mg eines farblosen Öls.
R_{f}(EE/HEP 2:1) = 0.52 MS (FAB) : 540 (M+H)⁺

### d) 4'-{[Benzyl-2-(2-methyl-phenyl)acetyl-amino]-methyl}-biphenyl-2-sulfonamid

320 mg 4'-{[Benzyl-2-(2-methyl-phenyl)acetyl-amino]-methyl}-biphenyl-2-sulfonsäure-(dimethylamino)methylenamid werden in 6 ml MeOH gelöst und bei RT 3 ml einer gesättigten wäßrigen HCI-Lösung zugegeben. 8 h wird unter Rückfluß gekocht und nach dem Abkühlen mit einer 6 N wäßrigen NaOH-Lösung auf pH = 5-6 gestellt. Mit 70 ml Wasser wird verdünnt und 3 mal mit je 70 ml EE extrahiert. Über Na₂SO₄ wird getrocknet und das Solvens im Vakuum entfernt. Man erhält 300 mg eines farblosen Öls.
R_{f}(EE) = 0.68 MS (ES) : 485 (M+H)⁺

### e) 4'-{[Benzyl-2-(2-methyl-phenyl)acetyl-amino]-methyl}-biphenyl-2-sulfonylcyanamid

280 mg 4'-{[Benzyl-2-(2-methyl-phenyl)acetyl-amino]-methyl}-biphenyl-2-sulfonamid und 245 mg K₂CO₃ werden in 6 ml Acetonitril (wasserfrei) gelöst und bei RT 116 µl einer 5 N Lösung von BrCN in Acetonitril zugespritzt. 2 h wird unter Rückfluß gekocht und nach dem Abkühlen das gesamte Reaktionsgemisch an Kieselgel mit EE/MeOH 10:1 chromatographiert. Man erhält 130 mg eines farblosen Feststoffs, mp 108°C (unter Zersetzung).
R_{f}(EE/MeOH 10:1) = 0.27 IR (CN) : 2177.0 cm⁻¹ MS (FAB) : 532 (M+Na)⁺
Restaktivität des NCBE bei 10 µM : 16%

Die Titelverbindungen der Beispiele 562 bis 568 werden analog Beispiel 561 synthetisiert:

### Beispiel 562

### 4'-{[Benzyl-2-(2-trifluormethyl-phenyl)acetyl-amino]-methyl}-biphenyl-2-sulfonylcyanamid

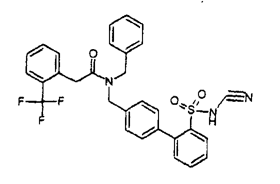

R_{f}(EE/MeOH 10:1) = 0.30 IR (CN) : 2178.0 cm⁻¹ MS (FAB) : 586 (M+Na)⁺
mp 90°C (unter Zersetzung). Restaktivität des NCBE bei 10 µM : 33%

### Beispiel 563

### 4'-{[Benzyl-2-(2-trifluormethoxy-phenyl)acetyl-amino]-methyl}-biphenyl-2-sulfonylcyanamid

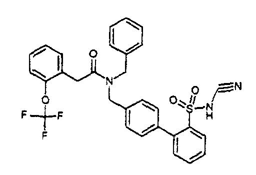

R_{f}(EE/MeOH 10:1) = 0.26 IR (CN) : 2177.0 cm⁻¹ MS (ES) : 580 (M+H)⁺
mp 125°C (unter Zersetzung). Restaktivität des NCBE bei 10 µM : 46%

### Beispiel 564

### 4'-{[Benzyl-2-(2-trifluormethylthio-phenyl)acetyl-amino]-methyl}-biphenyl-2-sulfonylcyanamid

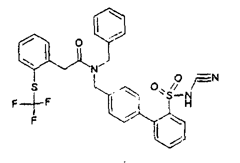

R_{f}(EE/MeOH 10:1) = 0.27 IR (CN) : 2176.0 cm⁻¹ MS (ES) : 596 (M+H)⁺
mp 122°C (unter Zersetzung). Restaktivität des NCBE bei 10 µM : 23%

### Beispiel 565

### 4'-{[Benzyl-2-methylbenzoyl-amino]-methyl}-biphenyl-2-sulfonylcyanamid

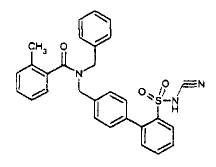

R_{f}(EE/MeOH 10:1) = 0.22 IR (CN) : 2174.0 cm⁻¹ MS (ES) : 496 (M+H)⁺
mp 173°C (unter Zersetzung). Restaktivität des NCBE bei 10 µM : 42%

### Beispiel 566

### 4'-{[Benzyl-2-trifluormethylbenzoyl-amino]-methyl}-biphenyl-2-sulfonylcyanamid

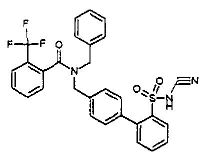

R_{f}(EE/MeOH 10:1) = 0.20 IR (CN) : 2176.0 cm⁻¹ MS (ES): 550 (M+H)⁺
mp 165°C (unter Zersetzung). Restaktivität des NCBE bei 10 µM : 72%

### Beispiel 567

### 4'-{[Benzyl-2-trifluormethoxylbenzoyl-amino]-methyl}-biphenyl-2-sulfonylcyanamid

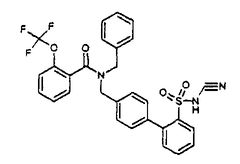

R_{f}(EE/MeOH 10:1) = 0.22 IR (CN) : 2176.0 cm⁻¹ MS (ES) : 566 (M+H)⁺
mp 160°C (unter Zersetzung). Restaktivität des NCBE bei 10µM : 69%

### Beispiel 568

### 4'-{[Benzyl-(5-methylthiophen-2-yl)carbonyl-amino]-methyl}-biphenyl-2-sulfonylcyanamid

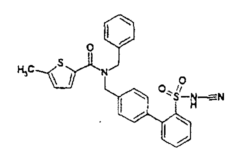

R_{f}(EE/MeOH 10:1) = 0.16 IR (CN): 2175.0 cm⁻¹ MS (FAB) : 524 (M+Na)⁺
mp 166°C (unter Zersetzung). Restaktivität des NCBE bei 10 µM : 70%.

### Pharmakologische Daten:

Inhibition des Na⁺- abhängigen Cl⁻/HCO₃⁻ - Austauschers (NCBE) in humanen Endothelzellen

Humane Endothelzellen (ECV-304) wurden aus Kulturflaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCI, 20 NH₄Cl, 5 KCI, 1 CaCl₂, 1 MgSO₄, 20 N-(2-Hydroxyethyl)piperazin-N□-2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37□C inkubiert. Anschließend wurden die Zellen gewaschen und in einer Natrium- und Bicarbonat-freien Pufferlösung (mmol/l: 5 HEPES, 133,8 Cholinchlorid, 4,7 KCI, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min). Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (pHᵢ) über den NCBE, eine Bicarbonat-und Natrium-haltige Pufferlösung (mmol/l: 5 HEPES, 93,8 NaCI, 40 NaHCO₃, 4,7 KCI, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCl, 4,7 KCI, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen wurden in verschiedenen Konzentrationen der Natrium-und Bicarbonat-haltigen Lösung zugesetzt. Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem well der Mikrotiterplatte bestimmt.
Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurden auf die beiden Kontrollen bezogen und daraus die Hemmwirkung der Substanzen ermittelt.

## Patentansprüche

1. Verbindungen der Formel (I), 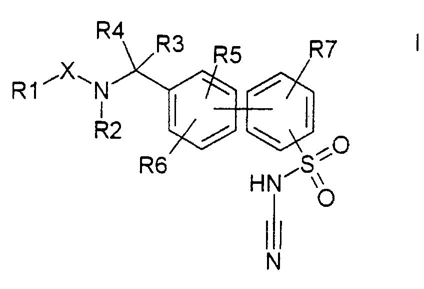 in welcher die Symbole folgende Bedeutung haben:
R(1)
1. Alkyl mit 1, 2, 3, 4, 5, 6, 7 oder 8 C-Atomen;
2. Alkyl mit 1, 2, 3, 4, 5, 6, 7 oder 8 C-Atomen, worin ein bis alle Wasserstoffatome durch Fluor ersetzt sind;
3. Alkenyl mit 2, 3, 4, 5, 6, 7, 8, 9, 10, 11 oder 12 C-Atomen; oder
4. -CₙH₂ₙ₋ₙₙ -Y,
nn Null oder 2; und
n Null, 1, 2, 3 oder 4; wobei n ungleich Null oder 1 ist, wenn nn gleich 2 ist;
5. -CₙH₂ₙ₋ₙₙ -Y,
nn Null oder 2; und
n 1, 2, 3 oder 4; wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen;
2. Amino;
3. N(R(22)R(23);
4. Alkoxycarbonyl;
5. COOR(16);
6. Alkyl mit 1, 2, 3 oder 4 C-Atomen;
7. (C₆-C₁₄)-Aryl-(C₁-C₄)-alkyl-carbonyl;
R(2)
1. Wasserstoff;
2. Alkyl mit 1, 2, 3, 4, 5, 6, 7 oder 8 C-Atomen;
3. Alkyl mit 1, 2, 3, 4, 5, 6, 7 oder 8 C-Atomen, worin ein bis alle Wasserstoffatome durch Fluor ersetzt sind;
4. Alkenyl mit 2, 3, 4, 5, 6, 7, 8, 9, 10, 11 oder 12 C-Atomen;
5. Alkinyl mit 2, 3, 4, 5, 6, 7 oder 8 C-Atomen;
6. -CₙH₂ₙ₋ₙₙ -Z,
nn Null oder 2; und
n Null, 1, 2, 3 oder 4; wobei n ungleich Null oder 1 ist, wenn nn gleich 2 ist;
7. -CₙH₂ₙ₋ₙₙ -Z,
nn Null oder 2; und
n 1, 2, 3 oder 4, wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen;
2. Amino;
3. N(R(22)R(23);
4. (C₁-C₄)-Alkoxycarbonyl;
5. COOR(16);
6. Alkyl mit 1, 2, 3 oder 4 C-Atomen;
R(3) und R(4) unabhängig voneinander Wasserstoff oder Alkyl mit 1, 2, 3, 4, 5, 6, 7 oder 8 C-Atomen;
R(5), R(6) und R(7) unabhängig voneinander Wasserstoff, Alkyl mit 1, 2, 3, 4, 5, 6, 7 oder 8 C-Atomen, F, Cl, Br, I, CF₃, -CN, -NO₂, SO_{q}-R(8), CO-R(21) oder O-R(10);
R(8) Alkyl mit 1, 2, 3, 4, 5, 6, 7 oder 8 C-Atomen, NR(11)R(12) oder Phenyl, das unsubstituiert ist oder substituiert ist mit 1, 2 oder 3 gleichen oder verschiedenen Resten aus der Reihe F, Cl, Br, I, CF₃, Methyl, Methoxy, Hydroxy oder NR(11)R(12);
R(9) und R(21) unabhängig voneinander Wasserstoff, Alkyl mit 1, 2, 3, 4, 5, 6, 7 oder 8 C-Atomen oder OR(13);
R(10) Wasserstoff, Alkyl mit 1, 2, 3, 4, 5, 6, 7 oder 8 C-Atomen gegebenenfalls substituiert mit (C₁-C₄)-Alkoxy; oder Phenyl, das unsubstituiert ist oder substituiert ist mit 1, 2 oder 3 gleichen oder verschiedenen Resten aus der Reihe F, Cl, Br, I, CF₃, Methyl, Methoxy, Hydroxy oder NR(11)R(12);
R(11), R(12), R(19) und R(20) unabhängig voneinander Wasserstoff, Alkyl mit 1, 2, 3 oder 4 C-Atomen oder (C₁-C₄)-Alkanoyl;
R(13) Wasserstoff oder Alkyl mit 1, 2, 3, 4, 5, 6, 7 oder 8 C-Atomen;
X Carbonyl, -CO-NH-, -CO-CO- oder Sulfonyl;
Y und Z unabhängig voneinander
1. Aryl mit 6, 7, 8, 9, 10, 11, 12, 13 oder 14 C-Atomen;
2. einen wie unter 1. definierten Rest, der mit 1, 2, 3, 4 oder 5 gleichen oder. verschiedenen Resten aus der Reihe Alkyl mit 1, 2, 3, 4, 5, 6, 7 oder 8 C-Atomen, Aryl mit 6, 7, 8, 9, 10, 11, 12, 13 oder 14 C-Atomen, F, Cl, Br, I, CF₃, SO_{q}R(18), OR(16), NR(19)R(20), -CN, NO₂ oder CO-R(9) substituiert ist; oder wobei zwei Reste gemeinsam einen anellierten Heterocyclylrest bilden.
3. Heteroaryl mit 1, 2, 3, 4, 5, 6, 7, 8 oder 9 C-Atomen;
4. einen wie unter 3. definierten Rest, der mit 1, 2 oder 3 gleichen oder verschiedenen Resten aus der Reihe F, Cl, Br, I, CF₃, CH₃, Methoxy, Hydroxy oder NR(11)R(12) substituiert ist;
5. Cycloalkyl mit 3, 4, 5, 6, 7, 8, 9 oder 10 C-Atomen;
6. einen wie unter 5. definierten Rest, substituiert mit Aryl mit 6, 7, 8, 9, 10, 11, 12, 13 oder 14 C-Atomen;
7. O-R(14);
8. O-R(17);
9. -SO₂-R(14);
10. Arylalkylcarbonyl; oder
11. Heterocyclyl;
R(14) und R(17) unabhängig voneinander
1. Wasserstoff;
2. Alkyl mit 1, 2, 3, 4, 5, 6, 7 oder 8 C-Atomen;
3. Alkenyl mit 2, 3, 4, 5, 6, 7, 8, 9, 10, 11 oder 12 C-Atomen;
4. -CₙH₂ₙ₋ₙₙ - Phenyl,
nn Null oder 2; und
n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3, 4, 5, 6, 7 oder 8 C-Atomen, F, Cl, Br, I, CF₃, SO_{q}R(15), OR(16), NR(11)R(12), -CN, -NO₂ oder CO-R(9); oder
R(15) und R(18) unabhängig voneinander Alkyl mit 1, 2, 3 oder 4 C-Atomen, Alkyl mit 1, 2, 3 oder 4 C-Atomen, worin ein bis alle Wasserstoffatome durch Fluor ersetzt sind, oder NR(11)R(12);
R(16)
1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind;
5. Aryl mit 6, 7, 8, 9, 10, 11, 12, 13 oder 14 C-Atomen;
6. einen wie unter 5. definierten Rest, der mit 1, 2 oder 3 gleichen oder verschiedenen Resten aus der Reihe F, Cl, Br, I, CF₃, NR(19)R(20), -CN, NO₂ substituiert ist;
R(22) und R(23) unabhängig voneinander Wasserstoff oder CO-OR(24);
R(24) Wasserstoff, Alkyl mit 1, 2, 3, 4, 5, 6, 7 oder 8 C-Atomen oder -CₙH₂ₙ - Phenyl mit n gleich 1, 2, 3 oder 4;
q unabhängig voneinander Null, 1 oder 2;
sowie deren physiologisch verträglichen Salze.

2. Verbindungen der Formel (I) gemäß Anspruch 1, in der bedeuten:
R(1)
1. Alkyl mit 1, 2, 3, 4 oder 5 C-Atomen;
2. Alkyl mit 1, 2, 3, 4 oder 5 C-Atomen, worin ein bis alle Wasserstoffatome durch Fluor ersetzt sind;
3. Alkenyl mit 2, 3, 4, 5, 6, 7, 8, 9, 10, 11 oder 12 C-Atomen; oder
4. -CₙH₂ₙ₋ₙₙ -Y,
nn Null oder 2; und
n Null, 1, 2, 3 oder 4; wobei n ungleich Null oder 1 ist, wenn nn gleich 2 ist;
5. -CₙH₂ₙ₋ₙₙ -Y,
nn Null oder 2; und
n 1, 2, 3 oder 4; wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen;
2. Amino;
3. NR(22)R(23);
4. Alkoxycarbonyl;
5. COOR(16);
6. Alkyl mit 1, 2, 3 oder 4 C-Atomen;
7. (C₆-C₁₄)-Aryl-(C₁-C₄)-alkyl-carbonyl;
R(2)
1. Wasserstoff;
2. Alkyl mit 1, 2, 3, 4, 5 oder 5 C-Atomen;
3. Alkyl mit 1, 2, 3, 4, oder 5 C-Atomen, worin ein bis alle Wasserstoffatome durch Fluor ersetzt sind;
4. Alkenyl mit 2, 3, 4, 5, 6, 7, 8, 9, 10, 11 oder 12 C-Atomen;
5. Alkinyl mit 2, 3, 4, oder 5 C-Atomen;
6. -CₙH₂ₙ₋ₙₙ -Z,
nn Null oder 2; und
n Null, 1, 2, 3 oder 4; wobei n ungleich Null oder 1 ist, wenn nn gleich 2 ist;
7. -Cₙ-H₂ₙ₋ₙₙ -Z,
nn Null oder 2; und
n 1, 2, 3 oder 4, wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen;
2. Amino;
3. N(R(22)R(23);
4. (C₁-C₄)-Alkoxycarbonyl;
5. COOR(16);
6. Alkyl mit 1, 2, 3 oder 4 C-Atomen;
R(3) und R(4) unabhängig voneinander Wasserstoff oder Alkyl mit 1, 2, 3, oder 4 C-Atomen;
R(5), R(6) und R(7) unabhängig voneinander Wasserstoff, Alkyl mit 1, 2, 3 oder 4 C-Atomen, F, Cl, Br, CF₃, -CN, SO_{q}-R(8), CO-R(21) oder O-R(10);
R(8) Alkyl mit 1, 2, 3 oder 4 C-Atomen, NR(11)R(12) oder Phenyl, das unsubstituiert ist oder substituiert ist mit 1 oder 2 gleichen oder verschiedenen Resten aus der Reihe F, Cl, Br, CF₃, Methyl, Methoxy, Hydroxy oder NR(11)R(12);
R(9) und R(21) unabhängig voneinander Wasserstoff, Alkyl mit 1, 2, 3 oder 4 C-Atomen oder OR(13);
R(10) Wasserstoff, Alkyl mit 1, 2, 3 oder 4 C-Atomen gegebenenfalls substituiert mit (C₁-C₄)-Alkoxy; oder Phenyl, das unsubstituiert ist oder substituiert ist mit 1 oder 2 gleichen oder verschiedenen Resten aus der Reihe F, Cl, Br, CF₃, Methyl, Methoxy, Hydroxy oder NR(11)R(12);
R(11), R(12), R(19) und R(20) unabhängig voneinander Wasserstoff, Alkyl mit 1, 2, 3 oder 4 C-Atomen oder (C₁-C₄)-Alkanoyl;
R(13) Wasserstoff oder Alkyl mit 1, 2, 3 oder 4 C-Atomen;
X Carbonyl, -CO-NH-, -CO-CO- oder Sulfonyl;
Y und Z unabhängig voneinander
1. Phenyl, 1-Naphthyl oder 2-Naphthyl;
2. einen wie unter 1. definierten Rest, der mit 1, 2, 3, 4 oder 5 gleichen oder verschiedenen Resten aus der Reihe Alkyl mit 1, 2, 3 oder 4 C-Atomen, Phenyl, 1-Naphthyl oder 2-Naphthyl, F, Cl, Br, CF₃, SO_{q}R(18), OR(16), NR(19)R(20), -CN oder CO-R(9) substituiert ist; oder wobei zwei Reste gemeinsam einen anellierten Heterocyclylrest bilden;
3. Heteroaryl mit 1, 2, 3, 4, 5, 6, 7, 8 oder 9 C-Atomen;
4. einen wie unter 3. definierten Rest, der mit 1 oder 2 gleichen oder verschiedenen Resten aus der Reihe F, Cl, Br, CF₃, CH₃, Methoxy, Hydroxy oder NR(11)R(12) substituiert ist;
5. Cycloalkyl mit 3, 4, 5, 6, 7, 8, 9 oder 10 C-Atomen;
6. einen wie unter 5. definierten Rest, substituiert mit Phenyl, 1-Naphthyl oder 2-Naphthyl;
7. O-R(14);
8. O-R(17);
9. -SO₂-R(14);
10. Arylalkylcarbonyl; oder
11. Heterocyclyl;
R(14) und R(17) unabhängig voneinander
1. Wasserstoff;
2. Alkyl mit 1, 2, 3 oder 4 C-Atomen;
3. Alkenyl mit 2, 3, 4, 5 oder 6 C-Atomen;
4. -CₙH₂ₙ₋ₙₙ - Phenyl,
nn Null oder 2; und
n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3 oder 4 C-Atomen, F, Cl, Br, CF₃, SO_{q}R(15), OR(16), NR(11)R(12), -CN, oder CO-R(9); oder
R(15) und R(18) unabhängig voneinander Alkyl mit 1, 2, 3 oder 4 C-Atomen, Alkyl mit 1, 2, 3 oder 4 C-Atomen, worin ein bis alle Wasserstoffatome durch Fluor ersetzt sind, oder NR(11)R(12);
R(16)
1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind;
5. Phenyl, 1-Naphthyl oder 2-Naphthyl; einen wie unter 5. definierten Rest, der mit 1, 2 oder 3 gleichen oder verschiedenen Resten aus der Reihe F, Cl, Br, CF₃, NR(19)R(20), -CN, substituiert ist;
R(22) und R(23) unabhängig voneinander Wasserstoff oder CO-OR(24);
R(24) Wasserstoff, Alkyl mit 1, 2, 3 oder 4 C-Atomen oder -CₙH₂ₙ - Phenyl mit n gleich 1, 2 oder 3;
q unabhängig voneinander Null, 1 oder 2;
sowie deren physiologisch verträglichen Salze.

3. Verbindungen der Formel (I) gemäß Anspruch 1 und/oder 2, in der bedeuten:
R(1)
1. Alkyl mit 1, 2, 3, 4 oder 5 C-Atomen,
2. Alkenyl mit 2, 3 oder 4 C-Atomen,
3. -CₙH₂ₙ₋ₙₙ - Y;
Y
1. Phenyl;
2. einen wie unter 1. definierten Rest, der mit 1, 2, 3, 4 oder 5 gleichen oder verschiedenen Resten aus der Reihe Alkyl mit 1, 2, 3 oder 4 C-Atomen, F, Cl, Br, Cyano, CF₃, Hydroxy, NO₂, SO₂R(18), OR(16), SCF₃, NR(19)R(20), CO-R(9);
3. OR(14), oder
4. SO₂-R(14);
5. 1-Naphthyl oder 2-Naphthyl;
6. einen wie unter 5. definierten Rest, der mit einem Rest aus der Reihe Alkyl mit 1, 2, 3 oder 4 C-Atomen, F, Cl, CF₃, SO₂R(18), OR(16), NR(19)R(20) oder CO-R(9) substituiert ist;
7. Heteroaryl mit 1, 2, 3, 4, 5, 6, 7, 8 oder 9 C-Atomen;
8. einen wie unter 3. definierten Rest, der mit einem Rest aus der Reihe F, Cl, CF₃, CH₃, Methoxy oder N(CH₃)₂ substituiert ist;
9. Cycloalkyl mit 3, 4, 5, 6 oder 7 C-Atomen;
nn Null oder 2; und
n Null, 1, 2, 3 oder 4, wobei n ungleich Null oder 1 ist, wenn nn gleich 2 ist;
4. -CₙH₂ₙ₋ₙₙ -Y,
Y
1. Phenyl;
2. OR(14); oder
3. Heteroaryl;
nn Null oder 2; und
n 1, 2 oder 3, wobei n ungleich 1 ist, wenn nn gleich 2 ist;
worin 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen, oder Phenylacetyl;
2. Amino;
3. NR(22)R(23); oder
4. Alkyl mit 1, 2, 3 oder 4 C-Atomen;
R(2)
1. Wasserstoff
2. Alkyl mit 1, 2, 3, 4 oder 5 C-Atomen;
3. Alkyl mit 1, 2, 3, 4 oder 5 C-Atomen, worin ein bis alle Wasserstoffatome durch Fluor ersetzt sind;
4. Alkenyl mit 2, 3, 4, 5, 6, 7, 8, 9 oder 10 C-Atomen;
5. Alkinyl mit 2, 3, 4 oder 5 C-Atomen
6. -CₙH₂ₙ₋ₙₙ - Z;
Z
1. Phenyl;
2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, Br, CF₃, SO₂R(18), OR(16), Nitro, Cyano, NR(19)R(20), CO-R(9), oder wobei zwei Reste gemeinsam einen Methylendioxy-Rest bilden;
3. 1 -Naphthyl, oder 2-Naphthyl;
4. einen wie unter 3. definierten Rest, der mit einem Rest aus der Reihe Alkyl mit 1, 2, 3 oder 4 C-Atomen, F, Cl, CF₃, SO₂R(18), OR(16), Nitro, Cyano, NR(19)R(20) oder CO-R(9) substituiert ist;
5. Heteroaryl mit 1, 2, 3, 4, 5, 6, 7, 8 oder 9 C-Atomen;
6. einen wie unter 5. definierten Rest, der mit einem Rest aus der Reihe F, Cl, CF₃, CH₃, Methoxy, Hydroxy oder N(CH₃)₂ substituiert ist;
7. Cycloalkyl mit 3, 4, 5, 6, 7, 8, 9 oder 10 C-Atomen;
8. einen wie unter 7. definierten Rest, der mit Phenyl substituiert ist;
nn Null oder 2; und
n Null, 1, 2 oder 3, wobei n ungleich Null oder 1 ist, wenn nn gleich 2 ist;
7. -CₙH₂ₙ₋ₙₙ-Z,
Z
1. Phenyl;
2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, CF₃, SO₂R(18), -OR(16), Nitro, Cyano, NR(19)R(20) oder CO-R(9) substituiert ist;
nn Null oder 2; und
n 1, 2 oder 3, wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. (C₁-C₄)-Alkoxycarbonyl;
2. COOR(16); oder
3. Alkyl mit 1, 2, 3 oder 4 C-Atomen;
8. -CₙH₂ₙ -OR(17);
n Null, 1, 2 oder 3;
R(3) und R(4) Wasserstoff oder Methyl;
R(5), R(6) und R(7) unabhängig voneinander Wasserstoff, Alkyl mit 1, 2, 3 oder 4 C-Atomen, F, Cl, CF₃, CN, SO₂-R(8), CO-R(21) oder O-R(10);
R(8) Alkyl mit 1, 2, 3 oder 4 C-Atomen, N(CH₃)₂ oder Phenyl, das unsubstituiert ist oder substituiert ist mit einem Rest aus der Reihe F, Cl, CF₃, Methyl, Methoxy, Hydroxy oder N(CH₃)₂;
R(9) und R(21) unabhängig voneinander Wasserstoff, Methyl oder OR(13);
R(10) Wasserstoff, Alkylmit 1, 2, 3 oder 4 C-Atomen, gegebenenfalls substituiert mit (C₁-C₄)-Alkoxy, oder Phenyl, das unsubstituiert ist oder substituiert ist mit einem Rest aus der Reihe F, Cl, CF₃, Methyl, Methoxy oder N(CH₃)₂;
R(11) und R(12) unabhängig voneinander Wasserstoff, Alkyl mit 1, 2, 3 oder 4 C-Atomen oder (C₁-C₄)-Alkanoyl;
R(13) Wasserstoff oder Alkyl mit 1, 2, 3 oder 4 C-Atomen;
X Carbonyl, -CO-CO-, -NH-CO- oder Sulfonyl;
R(14)
1. Wasserstoff;
2. Alkyl mit 1, 2, 3 oder 4 C-Atomen;
3. Alkenyl mit 2, 3, 4, 5 oder 6 C-Atomen;
4. -CₙH₂ₙ₋ₙₙ - Phenyl,
nn Null oder 2; und
n Null, 1, 2, 3 oder 4; wobei n ungleich Null oder 1 ist, wenn nn gleich 2 ist;
5. einen wie unter 4. definierten Rest, wobei der Phenylteil substituiert ist mit 1, 2 oder 3 gleichen oder verschiedenen Resten aus der Reihe Alkyl mit 1, 2, 3 oder 4 C-Atomen, F, Cl, Br, CF₃, SO_{q}R(15), OR(16), NR(11)R(12), -CN, oder CO-R(9);
R(15) Alkyl mit 1, 2, 3 oder 4 C-Atomen oder N(CH₃)₂;
R(16)
1. Wasserstoff,
2. Alkyl mit 1, 2, 3 oder 4 C-Atomen,
3. Alkyl mit 1, 2, 3 oder 4 C-Atomen substituiert mit (C₁-C₄)-Alkoxy,
4. Alkyl mit 1, 2, 3 oder 4 C-Atomen, worin ein bis alle Wasserstoffatome durch Fluor, ersetzt sind;
5. Phenyl, 1-Naphthyl oder 2-Naphthyl;
6. einen wie unter 5. definierten Rest, der mit 1, 2 oder 3 gleichen oder verschiedenen Resten aus der Reihe F, Cl, Br, CF₃, NR(19)R(20), -CN, substituiert ist;
R(17)
1. Wasserstoff;
2. Alkyl mit 1, 2, 3 oder 4 C-Atomen;
3. Alkenyl mit 2, 3, oder 4 C-Atomen;
4. -CₙH₂ₙ₋ₙₙ - Phenyl,
nn Null oder 2; und
n Null, 1, 2, 3 oder 4; wobei n ungleich Null oder 1 ist, wenn nn gleich 2 ist;
5. einen wie unter 4. definierten Rest, wobei der Phenylteil substituiert ist mit einem Rest aus der Reihe Alkyl mit 1, 2, 3 oder 4 C-Atomen, F, Cl, Br, CF₃, SO_{q}R(15), OR(16), NR(11)R(12), -CN, oder CO-R(9); oder
R(18) Alkyl mit 1, 2, 3 oder 4 C-Atomen, Alkyl mit 1, 2, 3 oder 4 C-Atomen, worin ein bis alle Wasserstoffatome durch Fluor ersetzt sind, oder NR(11)R(12);
R(19) und R(20) unabhängig voneinander Wasserstoff, Alkyl mit 1, 2, 3 oder 4 C-Atomen oder (C₁-C₄)-Alkanoyl;
R(22) und R(23) unabhängig voneinander Wasserstoff oder CO-OR(24);
R(24) Wasserstoff, Alkyl mit 1, 2, 3 oder 4 C-Atomen oder -CₙH₂ₙ - Phenyl mit n gleich 1 oder 2;
q unabhängig voneinander Null, 1 oder 2;
sowie deren physiologisch verträglichen Salze.

4. Verbindungen der Formel (I) gemäß einem oder mehreren der Ansprüche 1 bis 3, in der bedeuten:
R(1)
1. Alkyl mit 1, 2, 3, 4 oder 5 C-Atomen,
2. Alkenyl mit 2, 3 oder 4 C-Atomen,
3. -CₙH₂ₙ₋ₙₙ - Y;
Y
1. Phenyl;
2. einen wie unter 1. definierten Rest, der mit 1, 2, 3, 4 oder 5 gleichen oder verschiedenen Resten aus der Reihe Alkyl mit 1, 2, 3 oder 4 C-Atomen, F, Cl, Br, Cyano, CF₃, Hydroxy, NO₂, SO₂R(18), OCH₃, OCF₃, SCF₃, N(CH₃)₂, NH-CO-CH₃,CO-R(9), Phenoxy oder Phenoxy, einfach oder mehrfach substituiert mit Halogen, substituiert ist;
3. OR(14), oder
4. SO₂-R(14);
nn Null oder 2; und
n Null, 1, 2, 3 oder 4, wobei n ungleich Null oder 1 ist, wenn nn gleich 2 ist;
4. -CₙH₂ₙ₋ₙₙ -Y,
Y
1. Phenyl;
2. OR(14); oder
3. Heteroaryl, vorzugsweise Thienyl;
nn Null oder 2; und
n 1, 2 oder 3, wobei n ungleich 1 ist, wenn nn gleich 2 ist;
worin 1, 2 oder 3 Wasserstoffatome in dem zweiwertigen Rest -CₙH₂ₙ₋ₙₙ- unabhängig voneinander ersetzt sind durch einen Rest aus der Reihe
1. Aryl mit 6, 7, 8, 9, 10, 11, 12, 13 oder 14 C-Atomen; oder Phenylacetyl;
2. Amino;
3. NR(22)R(23); oder
4. Alkyl mit 1, 2, 3 oder 4 C-Atomen;
5. -CₙH₂ₙ -Y;
Y
1. 1-Naphthyl oder 2-Naphthyl;
2. einen wie unter 1. definierten Rest, der mit einem Rest aus der Reihe Alkyl mit 1, 2, 3 oder 4 C-Atomen, F, Cl, CF₃, SO₂R(18), OCH₃, N(CH₃)₂ oder CO-R(9) substituiert ist;
3. Heteroaryl mit 1, 2, 3, 4, 5, 6, 7, 8 oder 9 C-Atomen; vorzugsweise Thienyl, Benzothiophenyl, Indolyl oder Furyl;
4. einen wie unter 3. definierten Rest, der mit einem Rest aus der Reihe F, Cl, CF₃, CH₃, Methoxy oder N(CH₃)₂ substituiert ist;
5. Cycloalkyl mit 3, 4, 5, 6 oder 7 C-Atomen;
n Null, 1, 2, 3 oder 4 ;
6. -CₙH₂ₙ-OR(14);
n Null 1 oder 2;
R(2)
1. Alkyl mit 1, 2, 3, 4 oder 5 C-Atomen;
2. Alkyl mit 1, 2, 3, 4 oder 5 C-Atomen, worin ein bis alle Wasserstoffatome durch Fluor ersetzt sind;
3. Alkenyl mit 2, 3, 4, 5, 6, 7, 8, 9 oder 10 C-Atomen;
4. Alkinyl mit 2, 3, 4 oder 5 C-Atomen
5. -CₙH₂ₙ₋ₙₙ - Z;
Z
1. Phenyl;
2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, Br, CF₃, SO₂R(18), -OCH₃, -O(C₂H₄)OCH₃, Ethoxy, Hydroxy, Nitro, Cyano, N(CH₃)₂, -NH-CO-CH₃, CO-R(9), Phenoxy oder Phenoxy, einfach oder mehrfach substituiert mit Halogen, substituiert ist; oder wobei zwei Reste gemeinsam einen Methylendioxy-Rest bilden;
nn Null oder 2; und
n Null, 1, 2 oder 3, wobei n ungleich Null oder 1 ist, wenn nn gleich 2 ist;
6. -CₙH₂ₙ₋ₙₙ -Z,
Z
1. Phenyl;
2. einen wie unter 1. definierten Rest, der mit 1, 2 oder 3 gleichen oder verschiedenen Resten aus der Reihe Alkyl mit 1, 2, 3 oder 4 C-Atomen, Phenyl, F, Cl, CF₃, SO₂R(18), -OCH₃, -O(C₂H₄)OCH₃, Ethoxy, Hydroxy, Nitro, Cyano, N(CH₃)₂ oder CO-R(9) substituiert ist;
nn Null oder 2; und
n 1, 2 oder 3, wobei n ungleich 1 ist, wenn nn gleich 2 ist;
wobei 1, 2 oder 3 Wasserstoffatome in dem zweiwertigen Rest -CₙH₂ₙ₋ₙₙ- unabhängig voneinander ersetzt sind durch einen Rest aus der Reihe
1. (C₁-C₄)-Alkoxycarbonyl;
2. COOR(16); oder
3. Alkyl mit 1, 2, 3 oder 4 C-Atomen;
7. -CₙH₂ₙ -Z;
Z
1. 1-Naphthyl, oder 2-Naphthyl;
2. einen wie unter 1. definierten Rest, der mit einem Rest aus der Reihe Alkyl mit 1, 2, 3 oder 4 C-Atomen, F, Cl, CF₃, SO₂R(18), OCH₃, -O(C₂H₄)OCH₃, Ethoxy, Hydroxy, Nitro, Cyano, N(CH₃)₂, -NHCOCH₃ oder CO-R(9) substituiert ist;
3. Heteroaryl mit 1, 2, 3, 4, 5, 6, 7, 8 oder 9 C-Atomen, vorzugsweise Benzimadozolyl, Pyridyl, Thienyl, Furyl, Tetrahydrofuryl, Pyrrolidinyl, Pyrrolidine-1-carbonyl-4,5 dihydro-isoxazolyl, Benzofuranyl, beispielsweise 1,3-Dihydro-1-oxo-Benzo[c]furanyl, Quinazolinyl; beispielsweise 3,4-Dihydroquinazolinyl;
4. einen wie unter 3. definierten Rest, der mit einem Rest aus der Reihe F, Cl, CF₃, CH₃, Methoxy, Hydroxy oder N(CH₃)₂ substituiert ist;
5. Cycloalkyl mit 3, 4, 5, 6, 7, 8, 9 oder 10 C-Atomen; vorzugsweise, Cyclopropyl, Cyclopentyl, Cyclohexyl, 1,2,3,4-Tetrahydronaphthyl oder Indanyl;
6. einen wie unter 5. definierten Rest, der mit Phenyl substituiert ist; vorzugsweise Phenylcyclopentyl;
n Null, 1, 2 oder 3 ;
8. -CₙH₂ₙ-OR(17);
n 2 oder 3;
R(3) und R(4) Wasserstoff;
R(5), R(6) und R(7) unabhängig voneinander Wasserstoff, Alkyl mit 1, 2, 3 oder 4 C-Atomen, F, Cl, CF₃, SO₂-R(8), CO-R(21) oder O-R(10);
Methyl oder N(CH₃)₂;
R(9) und R(21) unabhängig voneinander Wasserstoff, Methyl oder OR(13);
R(10) Wasserstoff, Methyl oder Ethyl, gegebenenfalls substituiert mit Methoxy, oder Phenyl, das unsubstituiert ist oder substituiert ist mit einem Rest aus der Reihe F, Cl, CF₃, Methyl, Methoxy oder N(CH₃)₂;
R(13) Wasserstoff oder Alkyl mit 1, 2, 3 oder 4 C-Atomen;
Carbonyl, -CO-CO-, -NH-CO- oder Sulfonyl;
R(14)
1. Wasserstoff;
2. Methyl oder Ethyl;
3. Alkenyl mit 2, 3, 4, 5 oder 6 C-Atomen, vorzugsweise Allyl;
4. -CₙH₂ₙ- Phenyl mit n gleich Null oder 1;
5. einen wie unter 4. definierten Rest, wobei der Phenylteil substituiert ist mit einem Rest aus der Reihe Alkyl mit 1, 2, 3 oder 4 C-Atomen, F, Cl, CF₃, SO₂R(15), OCH₃, N(CH₃)₂ oder CO-R(9); oder
6. Alkenyl mit 2, 3 oder 4 C-Atomen;
R(15) Methyl oder N(CH₃)₂;
R(16) Wasserstoff oder Alkyl mit 1, 2, 3 oder 4 C-Atomen;
R(17)
1. Wasserstoff;
2. Methyl;
3. -CₙH₂ₙ- Phenyl mit n gleich Null oder 1;
4. einen wie unter 3. definierten Rest, wobei der Phenylteil substituiert ist mit einem Rest aus der Reihe Alkyl mit 1, 2, 3 oder 4 C-Atomen, F, Cl, CF₃, SO₂R(15), OCH₃, N(CH₃)₂ oder CO-R(9); oder
5. Alkenyl mit 2, 3 oder 4 C-Atomen;
R(18) Methyl, CF₃, Amino oder N(CH₃)₂;
R(22) und R(23) unabhängig voneinander
Wasserstoff oder CO-OR(24);
R(24) Wasserstoff, Alkyl mit 1, 2, 3 oder 4 C-Atomen oder -CₙH₂ₙ- Phenyl mit n gleich 1 oder 2;
sowie deren physiologisch verträglichen Salze.

5. Verbindungen der Formel (I) gemäß einem oder mehreren der Ansprüche 1 bis 4, wobei diese eine Verbindungen der Formel Ia darstellen, 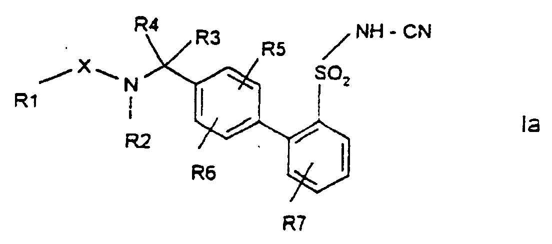 worin die Reste X und R(1) bis R(7) die in den Ansprüche 1 bis 4 genannte Bedeutung haben, sowie deren physiologisch verträglichen Salze.

6. Verbindungen der Formel (I) gemäß einem oder mehreren der Ansprüche 1 bis 5, in der bedeuten:
R(1)
CH₃-CH₂-CH₂-, Cyclohexyl-CH₂-CH₂-, Cyclohexyl-, 3-Methoxy-phenyl-CH₂-, 2-Methylphenyl-CH₂-, 2-Methylphenyl, 2-Thienyl-CH₂-, 4-t-Butyl-phenyl-, 2-Fluorophenyl-, Allyl-O-, 3, 4, 5-Trimethoxy-phenyl-, 2-Chloro-phenyl-, Phenyl-, CH₃-, E-CH₃-CH=CH-, CH₃-(CH₂)₃-, Phenyl-CH₂-CH₂-, 4-Methyl-phenyl-, Benzo[b]thiophen-2-yl-, Thien-2-yl-, (Phenyl)CH₂-, Phenyl-CH₂-CO-CH(Phenyl)-, (4-Chlorophenyl)-CH₂-, (CH₃)₃C-CH₂-, Phenyl-O-CH₂-, (4-Methoxyphenyl)-CH₂-, 2-Thienyl-CH₂-CH₂-CH₂-, (2-Thienyl)-, (indol-3-yl)-CH₂-, Benzo[b]thiophen-3-yl)-CH₂-, (3-Thienyl)-CH₂-, Phenyl-CH(NH₂)-, Phenyl-CH(NHCO-O-Benzyl)-, (2-Nitrophenyl)-CH₂-, Phenyl-(CH₂)₄-, (2-Furyl)-, Cyclohexyl-CH₂-CH₂-, (2-Bromophenyl)-CH₂-, Phenyl-SO₂-CH₂-CH₂-, (4-Hydroxyphenyl)-CH₂-, Phenyl-CH₂-NH-, N-Cyclohexyl-NH-, N-(2,6-Difluorophenyl)-NH-, N-Isopropyl-NH-, N-Cyclohexyl-NH-, Methoxy-CH₂-CH₂-, 2-Methoxy-phenyl-, Phenoxy-CH₂-CH₂-, Cyclohexyl-CH₂-, 4-Fluorophenyl-, Phenyl-CH₂-, Cyclopentyl-CH₂-, 4-Methylphenyl-, 3, 4-Dimethoxyphenyl-, 2, 5-Dimethoxyphenyl-, 2, 3, 4, 5, 6-Pentamethylphenyl-, 3-Fluoro-2, 4-dimethylphenyl-, 4-Fluoro-3, 5-dimethylphenyl-, 2, 4, 5-Trichlorophenyl-, 4-tert. Butylphenyl-, 2, 3-Dichlorophenyl-, (3-Thienyl)-CH-(CH₃)-, (2-Thienyl)-CH-(CH₃)-, 4-Chlorophenyl-, 4-Trifluoromethyl-phenyl-, 4-Methoxyphenyl-, 2-Chlorophenyl-, 2-Fluorophenyl-, (2-Thienyl)-(CH₂)₄-, Phenyl-(CH₂)₄-, (2-Thienyl)-CH₂-CH₂-, (2-Chlorophenyl)-CH₂-, 3, 4-Dichlorophenyl-, 2, 4-Dichlorophenyl-, 2, 5-Dichlorophenyl-, 2, 4-Difluorophenyl-, (2-Fluorophenyl)-CH₂-, (2-Furyl)-CH₂-, 2-Chlorophenyl-, 2-Chloro-6-methylphenyl-, (2-Thienyl)-CH₂-, (3-Thienyl)-CH₂-, 4-Acetylaminophenyl-, 3-Trifluoromethyl-phenyl-, 2-Trifluoromethyl-phenyl-, 5-Methyl-2thienyl-, 2-Trifluoromethoxyphenyl, 2-Trifluoromethylphenyl-CH₂, 2-Trifluoromethoxyphenyl-CH₂-, 2-Trifluoromethylthiophenyl-CH₂-, 2-Naphtyl-, 4-Fluoro-3, 5-dimethylphenyl-, (5-methyl-2-thienyl)-CH-(CH₃)-;
R(2)
Benzyl, CH₂-CH₂-OH, 4-Methoxy-benzyl, 4-Chloro-benzyl, Phenyl, Cyclohexyl, 2-Methoxy-benzyl, 2-Hydroxy-ethyl, 2-Chloro-benzyl, Isobutyl, 1(S)-Phenylethyl, 4-Methyl-benzyl, 3-Methoxy-benzyl, 3, 4-Methylenedioxy-benzyl, 5-Fluor-(2-Methoxy)ethoxy-benzyl, 1(R)-Phenyl-ethyl, 4-Trifluoromethyl-benzyl, 2-(4-Methoxyphenyl)-ethyl, 1(S)-(4-Methylphenyl)-ethyl, 2-Furylmethyl, 1(R)-(4-Methylphenyl)-ethyl, 4-(Dimethylamino)-benzyl, 2-(2-Thienyl)-ethyl, 2-Phenoxyethyl, 2,3-Dichloro-benzyl, Cyclopropylmethyl, 3, 4, 5-Trimethoxy-benzyl, (4-Pyridyl)-methyl, 4-Fluoro-benzyl, 2, 4-Dimethoxy-benzyl, 3-Phenyl-propyl, 2-Methoxy-ethyl, Methyl, Ethyl, (2-Pyridyl)-methyl, Cyclopropyl, (2-Benzimidazolyl)-methyl, 1-Methoxycarbonyl-2-phenyl-ethyl, (3, 4-Dihydro-quinazolin-2-yl)-methyl, Propargyl, (2-Thienyl)-methyl, Cyclohexyl-methyl, 3, 4-Dichloro-benzyl, Phenyl-CH(COOC(CH₃)3), Phenyl-CH(COOCH₃), 2, 4-Dichloro-benzyl, (1-Naphthyl)-methyl, 2-(4-H₂NSO₂-Phenyl)-ethyl, 3-Chloro-benzyl, 2, 3-Dichloro-benzyl, 3-Methyl-benzyl, 2-Methylbenzyl, (CH₃)2C=CH-CH₂-CH₂-C(CH₃)=CH-CH₂, 1-Indanyl, 1, 2, 3, 4-Tetrahydro-1-naphthyl, 2-Fluoro-benzyl, 3-Fluoro-benzyl, (1, 2, 3, 4-Tetrahydro-2-furyl)-methyl, 2, 4-Difluorophenyl, 4-tert.-Butylphenyl, 2, 6-Dichlorophenyl, 3-Chloro-4-fluorophenyl, 4-Chloro-2, 5-dimethyloxyphenyl, 2-Cyanophenyl, 2-Chlorophenyl, 2, 3-Dichlorophenyl, 4-Fluorophenyl, 2, 3-Dichlorophenyl, 4-(4-Fluorophenoxy)-phenyl, 3-Trifluoromethyl-phenyl, 2-Chloro-4-cyanophenyl, 2-Acetyl-phenyl, (1-Phenylcylopentyl)-methyl, 2-Ethyl-2-(4-Methoxyphenyl)-butyl, [3-(Pyrrolidine-1-carbonyl)-4, 5-dihydro-isoxazol-5-yl]-methyl, 4-Acetylamino-2-methylphenyl, 5-Acetylamino-2-methylphenyl, 4-Acetylamino-2, 6-dimethylphenyl, 4-Chlorophenyl, 3-Acetylphenyl, 4-Trifluoromethyl-phenyl, 2, 5-Difluorophenyl, 4-Isopropylphenyl, 4-Methoxyphenyl, 4-Ethoxyphenyl, 5-Chloro-2-methoxyphenyl, 2-Fluorophenyl, 1-Naphthyl, 2-Trifluoromethyl-phenyl, 2-Ethylphenyl, 2, 4-Dimethylphenyl, 2, 4, 5-Trimethylphenyl, 2, 5-Dimethylphenyl, 3-Chloro-2-methylphenyl, 4-Chloro-2-methylphenyl, 3-Trifluoromethyl-phenyl, 2, 6-dimethylphenyl, 2, 3-Dimethylphenyl, 4-Ethoxyphenyl, 3, 5-Dichlorophenyl, 3, 4-Dichlorophenyl, 3, 4-Dimethoxyphenyl, 4-Bromophenyl, 2-Chlorophenyl, 2-Methylphenyl, 2-Methoxyphenyl, 2, 6-Diethylphenyl, 2, 6-Diisopropylphenyl, 2-Biphenylyl, 2-Naphthyl, 4-Methylphenyl, 2, 4-Dimethoxyphenyl, 2, 5-Dimethylphenyl, 2-Chloro-4-methoxyphenyl, 2-Methoxy-5-methylphenyl, 3-Methoxy-4-methylphenyl, 2-Methoxyphenyl, 3-Hydroxyphenyl, 2-Chloro-5-trifluoromethyl-phenyl, 1-Acetylamino-2-naphthyl, 4-Ethylphenyl, 2, 5-Dichlorophenyl, 4-Trifluoromethoxyphenyl, 2 Chlorophenyl, 2-Methyl-1-naphthyl-, 1, 3-Dihydro-1-oxobenzo[c]furan-6-yl, Neopentyl, Isopropyl, 3-Nitro-benzyl, 2-Ethoxy-benzyl, 2, 2, 2-Trifluoroethyl, 2-(3-Trifluoromethyl-phenyl)ethyl, 1-Indanyl, 2-Phenyl-ethyl, (2-Naphthyl)-methyl, 2-Phenylethyl, Phenyl-CH(COOH)-, 3-Phenyl-benzyl-, 4-(4-Chlorophenoxy)-phenyl, 5-Chloro-2-methylphenyl-, 2-Fluorophenyl, 1-Naphthyl-;
R(3)
Wasserstoff;
R(4)
Wasserstoff;
R(5)
Wasserstoff, Chloro, -SO₂CH₃, (2-Methoxy)ethoxy-;
R(6)
Wasserstoff;
R(7)
Wasserstoff, Methyl-, Chloro;
X
-CO-, -CO-CO-, -CO-NH-, -SO₂-;
sowie deren physiologisch verträglichen Salze.

7. Verbindung der Formel I gemäß Anspruch 5, in der bedeuten:
R(1) 2-Chlorophenyl;
R(2) 4-Methylbenzyl;
R(3)- R(7) Wasserstoff;
X -CO-,
sowie deren physiologisch verträglichen Salze.

8. Verbindung der Formel I gemäß Anspruch 5, wobei diese 4'-{[Benzylthiophen-2-sulfonyl)amino]-methyl}-3'-chlor-biphenyl-2-sulfonylcyanamid ist, sowie deren physiologisch verträglichen Salze.

9. Verbindungen der Formel (I) gemäß einem oder mehreren der Ansprüche 1 bis 8 und/oder ihre physiologisch verträglichen Salze zur Anwendung als Arzneimittel.

10. Pharmazeutische Zubereitung, **gekennzeichnet durch** einen wirksamen Gehalt einer Verbindung der Formel (I) nach einem oder mehreren der Ansprüche 1 bis 8 und/oder eines physiologisch verträglichen Salzes davon.

11. Pharmazeutische Zubereitung gemäß Anspruch 10, **dadurch gekennzeichnet, daß** sie zusätzlich eine wirksame Menge eines NHE-Inhibitors und/oder eine wirksame Substanz aus einer anderen Herz-Kreislauf-Wirkstoffklasse, und/oder deren physiologisch verträglichen Salze enthält.

12. Verbindungen der Formel I gemäß einem oder mehreren der Ansprüche 1 bis 8 und/oder ihre physiologisch verträglichen Salze zur Verwendung als Inhibitor des Natrium-abhängigen Bicarbonat/Chlorid-Austauschers.

13. Verbindungen der Formel I gemäß einem oder mehreren der Ansprüche 1 bis 8 und/oder ihre physiologisch verträglichen Salze zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

14. Verbindungen der Formel I gemäß einem oder mehreren der Ansprüche 1 bis 8 und/oder ihre physiologisch verträglichen Salze zur Verwendung in der Theraphie und/oder Prophylaxe von Krebs.

15. Pharmazeutische Zubereitung gemäß Anspruch 10 und/oder 11 zur Verwendung in der Theraphie und/oder Prophylaxe des Herzinfarkts, der Angina 5 Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

16. Pharmazeutische Zubereitung gemäß Anspruch 10 und/oder 11 zur Verwendung in der Theraphie und/oder Prophylaxe von Krebs.

## Claims

1. A compound of the formula (I), 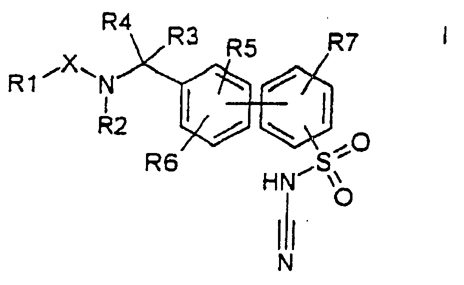 in which the symbols having the following meaning:
R(1) is
1. alkyl having 1, 2, 3, 4, 5, 6, 7 or 8 carbon atoms;
2. alkyl having 1, 2, 3, 4, 5, 6, 7 or 8 carbon atoms, in which one to all hydrogen atoms are replaced by fluorine;
3. alkenyl having 2, 3, 4, 5, 6, 7, 8, 9, 10, 11 or 12 carbon atoms; or
4. -CnH2n-nn-Y,
nn is zero or 2; and
n is zero, 1, 2, 3 or 4; where n is unequal to zero or 1 if nn is equal to 2;
5. -CnH2n-nn-Y,
nn is zero or 2; and
n is 1, 2, 3 or 4; where n is unequal to 1 if nn is equal to 2;
where 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms;
2. amino;
3. NR(22)R(23);
4. alkoxycarbonyl;
5. COOR(16);
6. alkyl having 1, 2, 3 or 4 carbon atoms;
7. (C6-C14)-aryl-(C1 -C4)-alkylcarbonyl;
R(2) is
1. hydrogen;
2. alkyl having 1, 2, 3, 4, 5, 6, 7 or 8 carbon atoms;
3. alkyl having 1, 2, 3, 4, 5, 6, 7 or 8 carbon atoms, in which one to all hydrogen atoms are replaced by fluorine;
4. alkenyl having 2, 3, 4, 5, 6, 7, 8, 9, 10, 11 or 12 carbon atoms;
5. alkynyl having 2, 3, 4, 5, 6, 7 or 8 carbon atoms;
6. -CnH2n-nn-Z,
nn is zero or 2; and
n is zero, 1, 2, 3 or 4; where n is unequal to zero or 1 if nn is equal to 2;
7. -CnH2n-nn-Z,
nn is zero or 2; and
n is 1, 2, 3 or 4, where n is unequal to 1 if nn is equal to 2;
where 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms;
2. amino;
3. NR(22)R(23);
4. (C1-C4)-alkoxycarbonyl;
5. COOR(16);
6. alkyl having 1, 2, 3 or 4 carbon atoms;
R(3) and R(4) independently of one another are hydrogen or alkyl having 1, 2, 3, 4, 5, 6, 7 or 8 carbon atoms;
R(5), R(6) and R(7) independently of one another are hydrogen, alkyl having 1, 2, 3, 4, 5, 6, 7 or 8 carbon atoms, F, Cl, Br, I, CF3, -CN, -NO2, SOq-R(8), CO-R(21) or O-R(10);
R(8) is alkyl having 1, 2, 3, 4, 5, 6, 7 or 8 carbon atoms, NR(11)R(12) or phenyl which is unsubstituted or is substituted by 1, 2 or 3 identical or different radicals from the group consisting of F, Cl, Br, I, CF3, methyl, methoxy, hydroxyl or NR(11)R(12);
R(9) and R(21) independently of one another are hydrogen, alkyl having 1, 2, 3, 4, 5, 6, 7 or 8 carbon atoms or OR(13);
R(10) is hydrogen, alkyl having 1, 2, 3, 4, 5, 6, 7 or 8 carbon atoms optionally substituted by (C1-C4)-alkoxy; or phenyl which is unsubstituted or is substituted by 1, 2 or 3 identical or different radicals from the group consisting of F, Cl, Br, I, CF3, methyl, methoxy, hydroxyl or NR(11)R(12);
R(11), R(12), R(19) and R(20) independently of one another are hydrogen, alkyl having 1, 2, 3 or 4 carbon atoms or (C1-C4)-alkanoyl;
R(13) is hydrogen or alkyl having 1, 2, 3, 4, 5, 6, 7 or 8 carbon atoms;
X is carbonyl, -CO-NH-, -CO-CO- or sulfonyl;
Y and Z independently of one another are
1. aryl having 6, 7, 8, 9, 10, 11, 12, 13 or 14 carbon atoms;
2. a radical as defined in 1., which is substituted by 1, 2, 3, 4 or 5 identical or different radicals from the group consisting of alkyl having 1, 2, 3, 4, 5, 6, 7 or 8 carbon atoms, aryl having 6, 7, 8, 9, 10, 11, 12, 13 or 14 carbon atoms, F, Cl, Br, I, CF3, SOqR(18), OR(16), NR(19)R(20), -CN, NO2 or CO-R(9); or where two radicals together form a fused heterocyclyl radical,
3. heteroaryl having 1, 2, 3, 4, 5, 6, 7, 8 or 9 carbon atoms;
4. a radical as defined in 3., which is substituted by 1, 2 or 3 identical or different radicals from the group consisting of F, Cl, Br, I, CF3, CH3, methoxy, hydroxyl or NR(11)R(12);
5. cycloalkyl having 3, 4, 5, 6, 7, 8, 9 or 10 carbon atoms;
6. a radical as defined in 5., substituted by aryl having 6, 7, 8, 9, 10, 11, 12, 13 or 14 carbon atoms;
7. O-R(14);
8. O-R(17);
9. -SO2-R(14);
10. arylalkylcarbonyl; or
11. heterocyclyl;
R(14) and R(17) independently of one another are
1. hydrogen;
2. alkyl having 1, 2, 3, 4, 5, 6, 7 or 8 carbon atoms;
3. alkenyl having 2, 3, 4, 5, 6, 7, 8, 9, 10, 11 or 12 carbon atoms;
4. -CnH2n-nn-phenyl,
nn is zero or 2; and
n is zero, 1, 2, 3 or 4; where n is unequal to zero or 1 if nn is equal to 2;
5. a radical as defined in 4., where the phenyl moiety is substituted by 1, 2 or 3 identical or different radicals from the group consisting of alkyl having 1, 2, 3, 4, 5, 6, 7 or 8 carbon atoms, F, Cl, Br, I, CF3, SOqR(15), OR(16), NR(11)R(12), -CN, -NO2 or CO-R(9); or
R(15) and R(18) independently of one another are alkyl having 1, 2, 3 or 4 carbon atoms, alkyl having 1, 2, 3 or 4 carbon atoms, in which one to all hydrogen atoms are replaced by fluorine, or NR(11)R(12);
R(16) is
1. hydrogen,
2. alkyl having 1, 2, 3 or 4 carbon atoms,
3. alkyl having 1, 2, 3 or 4 carbon atoms, substituted by (C1-C4)-alkoxy,
4. alkyl having 1, 2, 3 or 4 carbon atoms, in which one to all hydrogen atoms are replaced by fluorine;
5. aryl having 6, 7, 8, 9, 10, 11, 12, 13 or 14 carbon atoms;
6. a radical as defined in 5., which is substituted by 1, 2 or 3 identical or different radicals from the group consisting of F, Cl, Br, I, CF3, NR(19)R(20), -CN, NO2;
R(22) and R(23) independently of one another are hydrogen or CO-OR(24);
R(24) is hydrogen, alkyl having 1, 2, 3, 4, 5, 6, 7 or 8 carbon atoms or -CnH2n-phenyl where n is equal to 1, 2, 3 or 4;
q independently of one another is zero, 1 or 2;
or its physiologically tolerable salts.

2. A compound of the formula (I) as claimed in claim 1, in which:
R(1) is
1. alkyl having 1, 2, 3, 4 or 5 carbon atoms;
2. alkyl having 1, 2, 3, 4 or 5 carbon atoms, in which one to all hydrogen atoms are replaced by fluorine;
3. alkenyl having 2, 3, 4, 5, 6, 7, 8, 9, 10, 11 or 12 carbon atoms; or
4. -CnH2n-nn-Y,
nn is zero or 2; and
n is zero, 1, 2, 3 or 4; where n is unequal to zero or 1 if nn is equal to 2;
5. -CnH2n-nn-Y,
nn is zero or 2; and
n is 1, 2, 3 or 4; where n is unequal to 1 if nn is equal to 2;
where 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms;
2. amino;
3. NR(22)R(23);
4. alkoxycarbonyl;
5. COOR(16);
6. alkyl having 1, 2, 3 or 4 carbon atoms;
7. (C6-C14)-aryl-(C1-C4)-alkylcarbonyl;
R(2) is
1. hydrogen;
2. alkyl having 1, 2, 3, 4, 5 or 6 carbon atoms;
3. alkyl having 1, 2, 3, 4 or 5 carbon atoms, in which one to all hydrogen atoms are replaced by fluorine;
4. alkenyl having 2, 3, 4, 5, 6, 7, 8, 9, 10, 11 or 12 carbon atoms;
5. alkynyl having 2, 3, 4 or 5 carbon atoms;
6. -CnH2n-nn-Z,
nn is zero or 2; and
n is zero, 1, 2, 3 or 4; where n is unequal to zero or 1 if nn is equal to 2;
7. -CnH2n-nn-Z,
nn is zero or 2; and
n is 1, 2, 3 or 4, where n is unequal to 1 if nn is equal to 2;
where 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms;
2. amino;
3. NR(22)R(23);
4. (C1-C4)-alkoxycarbonyl;
5. COOR(16);
6. alkyl having 1, 2, 3 or 4 carbon atoms;
R(3) and R(4) independently of one another are hydrogen or alkyl having 1, 2, 3 or 4 carbon atoms;
R(5), R(6) and R(7) independently of one another are hydrogen, alkyl having 1, 2, 3 or 4 carbon atoms, F, Cl, Br, CF3, -ON, SOq-R(8), CO-R(21) or O-R(10);
R(8) is alkyl having 1, 2, 3 or 4 carbon atoms, NR(11)R(12) or phenyl which is unsubstituted or is substituted by 1 or 2 identical or different radicals from the group consisting of F, Cl, Br, CF3, methyl, methoxy, hydroxyl or NR(11)R(12);
R(9) and R(21) independently of one another are hydrogen, alkyl having 1, 2, 3 or 4 carbon atoms or OR(13);
R(10) is hydrogen, alkyl having 1, 2, 3 or 4 carbon atoms optionally substituted by (C1-C4)-alkoxy; or phenyl which is unsubstituted or is substituted by 1 or 2 identical or different radicals from the group consisting of F, Cl, Br, CF3, methyl, methoxy, hydroxyl or NR(11)R(12);
R(11), R(12), R(19) and R(20) independently of one another are hydrogen, alkyl having 1, 2, 3 or 4 carbon atoms or (C1-C4)-alkanoyl;
R(13) is hydrogen or alkyl having 1, 2, 3 or 4 carbon atoms;
X is carbonyl, -CO-NH-, -CO-CO- or sulfonyl;
Y and Z independently of one another are
1. phenyl, 1-naphthyl or 2-naphthyl;
2. a radical as defined in 1., which is substituted by 1, 2, 3, 4 or 5 identical or different radicals from the group consisting of alkyl having 1, 2, 3 or 4 carbon atoms, phenyl, 1-naphthyl or 2-naphthyl, F, Cl, Br, CF3, SOqR(18), OR(16), NR(19)R(20), -CN or CO-R(9);
or where two radicals together form a fused heterocyclyl radical;
3. heteroaryl having 1, 2, 3, 4, 5, 6, 7, 8 or 9 carbon atoms;
4. a radical as defined in 3., which is substituted by 1 or 2 identical or different radicals from the group consisting of F, Cl, Br, CF3, CH3, methoxy, hydroxyl or NR(11)R(12);
5. cycloalkyl having 3, 4, 5, 6, 7, 8, 9 or 10 carbon atoms;
6. a radical as defined in 5., substituted by phenyl, 1-naphthyl or 2-naphthyl;
7. O-R(14);
8. O-R(17);
9. -SO2-R(14);
10. arylalkylcarbonyl; or
11. heterocyclyl;
R(14) and R(17) independently of one another are
1. hydrogen;
2. alkyl having 1, 2, 3 or 4 carbon atoms;
3. alkenyl having 2, 3, 4, 5 or 6 carbon atoms;
4. -CnH2n-nn-phenyl,
nn is zero or 2; and
n is zero, 1, 2, 3 or 4; where n is unequal to zero or 1 if nn is equal to 2;
5. a radical as defined in 4., where the phenyl moiety is substituted by 1, 2 or 3 identical or different radicals from the group consisting of alkyl having 1, 2, 3 or 4 carbon atoms, F, Cl, Br, CF3, SOqR(15), OR(16), NR(11)R(12), -CN, or CO-R(9); or
R(15) and R(18) independently of one another are alkyl having 1, 2, 3 or 4 carbon atoms, alkyl having 1, 2, 3 or 4 carbon atoms, in which one to all hydrogen atoms are replaced by fluorine, or NR(11)R(12);
R(16) is
1. hydrogen,
2. alkyl having 1, 2, 3 or 4 carbon atoms,
3. alkyl having 1, 2, 3 or 4 carbon atoms substituted by (C1-C4)-alkoxy,
4. alkyl having 1, 2, 3 or 4 carbon atoms, in which one to all hydrogen atoms are replaced by fluorine;
5. phenyl, 1-naphthyl or 2-naphthyl;
6. a radical as defined in 5., which is substituted by 1, 2 or 3 identical or different radicals from the group consisting of F, Cl, Br, CF3, NR(19)R(20), -CN;
R(22) and R(23) independently of one another are hydrogen or CO-OR(24);
R(24) is hydrogen, alkyl having 1, 2, 3 or 4 carbon atoms or -CnH2n-phenyl where n is equal to 1, 2 or 3;
q independently of one another is zero, 1 or 2;
or its physiologically tolerable salts.

3. A compound of the formula (I) as claimed in claim 1 and/or 2, in which:
R(1) is
1. alkyl having 1, 2, 3, 4 or 5 carbon atoms,
2. alkenyl having 2, 3 or 4 carbon atoms,
3. -CnH2n-nn-Y;
Y is
1. phenyl;
2. a radical as defined in 1., which is substituted by 1, 2, 3, 4 or 5 identical or different radicals from the group consisting of alkyl having 1, 2, 3 or 4 carbon atoms, F, Cl, Br, cyano, CF3, hydroxyl, NO2, SO2R(18) OR(16), SCF3, NR(19)R(20), CO-R(9);
3. OR(14), or
4. SO2-R(14);
5. 1-naphthyl or 2-naphthyl;
6. a radical as defined in 5., which is substituted by a radical from the group consisting of alkyl having 1, 2, 3 or 4 carbon atoms, F, Cl, CF3, SO2R(18), OR(16), NR(19)R(20) or CO-R(9);
7. heteroaryl having 1, 2, 3, 4, 5, 6, 7, 8 or 9 carbon atoms;
8. a radical as defined in 3., which is substituted by a radical from the group consisting of F, Cl, CF3, CH3, methoxy or N(CH3)2;
9. cycloalkyl having 3, 4, 5, 6 or 7 carbon atoms;
nn is zero or 2; and
n is zero, 1, 2, 3 or 4, where n is unequal to zero or 1 if nn is equal to 2;
4. -CnH2n-nn-Y,
Y is
1. phenyl;
2. OR(14); or
3. heteroaryl;
nn is zero or 2; and
n is 1, 2 or 3, where n is unequal to 1 if nn is equal to 2;
in which 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms, or phenylacetyl;
2. amino;
3. NR(22)R(23); or
4. alkyl having 1, 2, 3 or 4 carbon atoms;
R(2) is
1. hydrogen
2. alkyl having 1, 2, 3, 4 or 5 carbon atoms;
3. alkyl having 1, 2, 3, 4 or 5 carbon atoms, in which one to all hydrogen atoms are replaced by fluorine;
4. alkenyl having 2, 3, 4, 5, 6, 7, 8, 9 or 10 carbon atoms;
5. alkynyl having 2, 3, 4 or 5 carbon atoms;
6. -CnH2n-nn-Z;
Z is
1. phenyl;
2. a radical as defined in 1., which is substituted by 1, 2 or 3 identical or different radicals from the group consisting of alkyl having 1, 2, 3 or 4 carbon atoms, phenyl, F, Cl, Br, CF3, SO2R(18), OR(16), nitro, cyano, NR(19)R(20), CO-R(9), or where two radicals together form a methylenedioxy radical;
3. 1-naphthyl, or 2-naphthyl;
4. a radical as defined in 3., which is substituted by a radical from the group consisting of alkyl having 1, 2, 3 or 4 carbon atoms, F, Cl, CF3, SO2R(18), OR(16), nitro, cyano, NR(19)R(20) or CO-R(9);
5. heteroaryl having 1, 2, 3, 4, 5, 6, 7, 8 or 9 carbon atoms;
6. a radical as defined in 5., which is substituted by a radical from the group consisting of F, Cl, CF3, CH3, methoxy, hydroxyl or N(CH3)2;
7. cycloalkyl having 3, 4, 5, 6, 7, 8, 9 or 10 carbon atoms;
8. a radical as defined in 7., which is substituted by phenyl;
nn is zero or 2; and
n is zero, 1, 2 or 3, where n is unequal to zero or 1 if nn is equal to 2;
7. -CnH2n-nn-Z,
Z is
1. phenyl;
2. a radical as defined in 1., which is substituted by 1, 2 or 3 identical or different radicals from the group consisting of alkyl having 1, 2, 3 or 4 carbon atoms, phenyl, F, Cl, CF3, SO2R(18), -OR(16), nitro, cyano, NR(19)R(20) or CO-R(9);
nn is zero or 2; and
n is 1, 2 or 3, where n is unequal to 1 if nn is equal to 2;
where 1, 2 or 3 hydrogen atoms in the divalent radical -CnH2n-nn- independently of one another are replaced by a radical from the group consisting of
1. (C1-C4)-alkoxycarbonyl;
2. COOR(16); or
3. alkyl having 1, 2, 3 or 4 carbon atoms;
8. -CnH2n-OR(17);
n is zero, 1, 2 or 3;
R(3) and R(4) are hydrogen or methyl;
R(5), R(6) and R(7) independently of one another are hydrogen, alkyl having 1, 2, 3 or 4 carbon atoms, F, Cl, CF3, CN, SO2-R(8), CO-R(21) or O-R(10);
R(8) is alkyl having 1, 2, 3 or 4 carbon atoms, N(CH3)2 or phenyl which is unsubstituted or is substituted by a radical from the group consisting of F, Cl, CF3, methyl, methoxy, hydroxyl or N(CH3)2;
R(9) and R(21) independently of one another are hydrogen, methyl or OR(13);
R(10) is hydrogen, alkyl having 1, 2, 3 or 4 carbon atoms, optionally substituted by (C1-C4)-alkoxy, or phenyl which is unsubstituted or is substituted by a radical from the group consisting of F, Cl, CF3, methyl, methoxy or N(CH3)2;
R(11) and R(12) independently of one another are hydrogen, alkyl having 1, 2, 3 or 4 carbon atoms or (C1-C4)-alkanoyl;
R(13) is hydrogen or alkyl having 1, 2, 3 or 4 carbon atoms;
X is carbonyl, -CO-CO-, -NH-CO- or sulfonyl;
R(14) is
1. hydrogen;
2. alkyl having 1, 2, 3 or 4 carbon atoms;
3. alkenyl having 2, 3, 4, 5 or 6 carbon atoms;
4. -CnH2n-nn-phenyl,
nn is zero or 2; and
n is zero, 1, 2, 3 or 4; where n is unequal to zero or 1 if nn is equal to 2;
5. a radical as defined in 4., where the phenyl moiety is substituted by 1, 2 or 3 identical or different radicals from the group consisting of alkyl having 1, 2, 3 or 4 carbon atoms, F, Cl, Br, CF3, SOqR(15), OR(16), NR(11)R(12), -CN, or CO-R(9);
R(15) is alkyl having 1, 2, 3 or 4 carbon atoms or N(CH3)2;
R(16) is
1. hydrogen,
2. alkyl having 1, 2, 3 or 4 carbon atoms,
3. alkyl having 1, 2, 3 or 4 carbon atoms substituted by (C1-C4)-alkoxy,
4. alkyl having 1, 2, 3 or 4 carbon atoms, in which one to all hydrogen atoms are replaced by fluorine;
5. phenyl, 1-naphthyl or 2-naphthyl;
6. a radical as defined in 5., which is substituted by 1, 2 or 3 identical or different radicals from the group consisting of F, Cl, Br, CF3, NR(19)R(20), -CN;
R(17) is
1. hydrogen;
2. alkyl having 1, 2, 3 or 4 carbon atoms;
3. alkenyl having 2, 3, or 4 carbon atoms;
4. -CnH2n-nn-phenyl,
nn is zero or 2; and
n is zero, 1, 2, 3 or 4; where n is unequal to zero or 1 if nn is equal to 2;
5. a radical as defined in 4., where the phenyl moiety is substituted by a radical from the group consisting of alkyl having 1, 2, 3 or 4 carbon atoms, F, Cl, Br, CF3, SOqR(15), OR(16), NR(11)R(12), -CN, or CO-R(9); or
R(18) is alkyl having 1, 2, 3 or 4 carbon atoms, alkyl having 1, 2, 3 or 4 carbon atoms, in which one to all hydrogen atoms are replaced by fluorine, or NR(11)R(12);
R(19) and R(20) independently of one another are hydrogen, alkyl having 1, 2, 3 or 4 carbon atoms or (C1-C4)-alkanoyl;
R(22) and R(23) independently of one another are hydrogen or CO-OR(24);
R(24) is hydrogen, alkyl having 1, 2, 3 or 4 carbon atoms or -Cn-H2n-phenyl where n is equal to 1 or 2;
q independently of one another is zero, 1 or 2;
or its physiologically tolerable salts.

4. A compound of the formula (I) as claimed in one or more of claims 1 to 3, in which:
R(1) is
1. alkyl having 1, 2, 3, 4 or 5 carbon atoms,
2. alkenyl having 2, 3 or 4 carbon atoms,
3. -CnH2n-nn-Y;
Y is
1. phenyl;
2. a radical as defined in 1., which is substituted by 1, 2, 3, 4 or 5 identical or different radicals from the group consisting of alkyl having 1, 2, 3 or 4 carbon atoms, F, Cl, Br, cyano, CF3, hydroxyl, NO2, SO2R(18), OCH3, OCF3, SCF3, N(CH3)2, NH-CO-CH3, CO-R(9), phenoxy or phenoxy, mono- or polysubstituted by halogen;
3. OR(14), or
4. SO2-R(14);
nn is zero or 2; and
n is zero, 1, 2, 3 or 4, where n is unequal to zero or 1 if nn is equal to 2;
4. -CnH2n-nn-Y,
Y is
1. phenyl;
2. OR(14); or
3. heteroaryl, preferably thienyl;
nn is zero or 2; and
n is 1, 2 or 3, where n is unequal to 1 if nn is equal to 2;
in which 1, 2 or 3 hydrogen atoms in the divalent radical -CnH2n-nn- independently of one another are replaced by a radical from the group consisting of
1. aryl having 6, 7, 8, 9, 10, 11, 12, 13 or 14 carbon atoms; or phenylacetyl;
2. amino;
3. NR(22)R(23); or
4. alkyl having 1, 2, 3 or 4 carbon atoms;
5. -CnH2n-Y;
Y is
1. 1-naphthyl or 2-naphthyl;
2. a radical as defined in 1., which is substituted by a radical from the group consisting of alkyl having 1, 2, 3 or 4 carbon atoms, F, Cl, CF3, SO2R(18) OCH3, N(CH3)2 or CO-R(9);
3. heteroaryl having 1, 2, 3, 4, 5, 6, 7, 8 or 9 carbon atoms; preferably thienyl, benzothiophenyl, indolyl or furyl;
4. a radical as defined in 3., which is substituted by a radical from the group consisting of F, Cl, CF3, CH3, methoxy or N(CH3)2;
5. cycloalkyl having 3, 4, 5, 6 or 7 carbon atoms;
n is zero, 1, 2, 3 or 4;
6. -CnH2n-OR(14);
n is zero 1 or 2;
R(2) is
1. alkyl having 1, 2, 3, 4 or 5 carbon atoms;
2. alkyl having 1, 2, 3, 4 or 5 carbon atoms, in which one to all hydrogen atoms are replaced by fluorine;
3. alkenyl having 2, 3, 4, 5, 6, 7, 8, 9 or 10 carbon atoms;
4. alkynyl having 2, 3, 4 or 5 carbon atoms;
5. -CnH2n-nn-Z;
Z is
1. phenyl;
2. a radical as defined in 1., which is substituted by 1, 2 or 3 identical or different radicals from the group consisting of alkyl having 1, 2, 3 or 4 carbon atoms, phenyl, F, Cl, Br, CF3, SO2R(18) -OCH3, -O(C2H4)OCH3, ethoxy, hydroxyl, nitro, cyano, N(CH3)2, -NH-CO-CH3, CO-R(9), phenoxy or phenoxy, monosubstituted or polysubstituted by halogen; or where two radicals together form a methylenedioxy radical;
nn is zero or 2; and
n is zero, 1, 2 or 3, where n is unequal to zero or 1 if nn is equal to 2;
6. -CnH2n-nn-Z,
Z is
1. phenyl;
2. a radical as defined in 1., which is substituted by 1, 2 or 3 identical or different radicals from the group consisting of alkyl having 1, 2, 3 or 4 carbon atoms, phenyl, F, Cl, CF3, SO2R(18) -OCH3, -O(C2H4)OCH3, ethoxy, hydroxyl, nitro, cyano, N(CH3)2 or CO-R(9);
nn is zero or 2; and
n is 1, 2 or 3, where n is unequal to 1 if nn is equal to 2;
where 1, 2 or 3 hydrogen atoms in the divalent radical -CnH2n-nn- independently of one another are replaced by a radical from the group consisting of
1. (C1-C4)-alkoxycarbonyl;
2. COOR(16); or
3. alkyl having 1, 2, 3 or 4 carbon atoms;
7. -CnH2n-Z;
Z is
1. 1-naphthyl, or 2-naphthyl;
2. a radical as defined in 1., which is substituted by a radical from the group consisting of alkyl having 1, 2, 3 or 4 carbon atoms, F, Cl, CF3, SO2R(18), OCH3, -O(C2H4)OCH3, ethoxy, hydroxyl, nitro, cyano, N(CH3)2, -NHCOCH3 or CO-R(9);
3. heteroaryl having 1, 2, 3, 4, 5, 6, 7, 8 or 9 carbon atoms, preferably benzimidazolyl, pyridyl, thienyl, furyl, tetrahydrofuryl, pyrrolidinyl, pyrrolidine-1-carbonyl-4,5-dihydroisoxazolyl, benzofuranyl, for example 1,3-dihydro-1-oxobenzo[c]furanyl, quinazolinyl; for example 3,4-dihydroquinazolinyl;
4. a radical as defined in 3., which is substituted by a radical from the group consisting of F, Cl, CF3, CH3, methoxy, hydroxyl or N(CH3)2;
5. cycloalkyl having 3, 4, 5, 6, 7, 8, 9 or 10 carbon atoms; preferably, cyclopropyl, cyclopentyl, cyclohexyl, 1,2,3,4-tetrahydronaphthyl or indanyl;
6. a radical as defined in 5., which is substituted by phenyl; preferably phenylcyclopentyl;
n is zero, 1, 2 or 3;
8. -CnH2n-OR(17);
n is 2 or 3;
R(3) and R(4) are hydrogen;
R(5), R(6) and R(7) independently of one another are hydrogen, alkyl having 1, 2, 3 or 4 carbon atoms, F, Cl, CF3, SO2-R(8), CO-R(21) or O-R(10);
R(8) is methyl or N(CH3)2;
R(9) and R(21) independently of one another are hydrogen, methyl or OR(13);
R(10) is hydrogen, methyl or ethyl, optionally substituted by methoxy, or phenyl which is unsubstituted or is substituted by a radical from the group consisting of F, Cl, CF3, methyl, methoxy or N(CH3)2;
R(13) is hydrogen or alkyl having 1, 2, 3 or 4 carbon atoms;
X is carbonyl, -CO-CO-, -NH-CO- or sulfonyl;
R(14) is
1. hydrogen;
2. methyl or ethyl;
3. alkenyl having 2, 3, 4, 5 or 6 carbon atoms, preferably allyl;
4. -CnH2n-phenyl where n is equal to zero or 1;
5. a radical as defined in 4., where the phenyl moiety is substituted by a radical from the group consisting of alkyl having 1, 2, 3 or 4 carbon atoms, F, Cl, CF3, SO2R(15), OCH3, N(CH3)2 or CO-R(9); or
6. alkenyl having 2, 3 or 4 carbon atoms;
R(15) is methyl or N(CH3)2;
R(16) is hydrogen or alkyl having 1, 2, 3 or 4 carbon atoms;
R(17) is
1. hydrogen;
2. methyl;
3. -CnH2n-phenyl where n is equal to zero or 1;
4. a radical as defined in 3., where the phenyl moiety is substituted by a radical from the group consisting of alkyl having 1, 2, 3 or 4 carbon atoms, F, Cl, CF3, SO2R(15), OCH3, N(CH3)2 or CO-R(9); or
5. alkenyl having 2, 3 or 4 carbon atoms;
R(18) is methyl, CF3, amino or N(CH3)2;
R(22) and R(23) independently of one another are hydrogen or CO-OR(24);
R(24) is hydrogen, alkyl having 1, 2, 3 or 4 carbon atoms or -CnH2n-phenyl where n is equal to 1 or 2;
or its physiologically tolerable salts.

5. A compound of the formula (I) as claimed in one or more of claims 1 to 4, where this is a compound of the formula la, 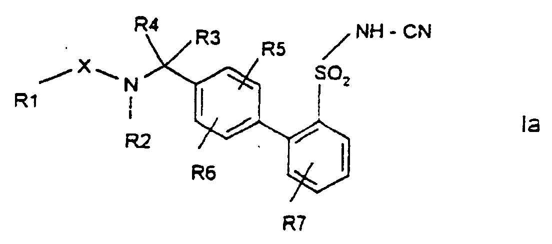 in which the radicals X and R(1) to R(7) have the meaning mentioned in claims 1 to 4, or its physiologically tolerable salts.

6. A compound of the formula (I) as claimed in one or more of claims 1 to 5, in which
R(1)is
CH3-CH2-CH2-, cyclohexyl-CH2-CH2-, cyclohexyl-, 3-methoxyphenyl-CH2-, 2-methylphenyl-CH2-, 2-methylphenyl, 2-thienyl-CH2-, 4-t-butylphenyl-, 2-fluorophenyl-, allyl-O-, 3,4,5-trimethoxyphenyl-, 2-chlorophenyl-, phenyl-, CH3-, E-CH3-, CH=CH-, CH3-(CH2)3-, phenyl-CH2-CH2-, 4-methylphenyl-, benzo[b]thiophen-2-yl-, thien-2-yl-, (phenyl)CH2-, phenyl-CH2-CO-CH(phenyl)-, (4-chlorophenyl)-CH2-, (CH3)3C-CH2-, phenyl-O-CH2-, (4-methoxyphenyl)-CH2-, 2-thienyl-CH2-CH2-CH2-, (2-thienyl)-, (indol-3-yl)-CH2-, benzo[b]thiophen-3-yl)-CH2-, (3-thienyl)-CH2-, phenyl-CH(NH2)-, phenyl-CH(NHCO-O-benzyl)-, (2-nitrophenyl)-CH2-, phenyl-(CH2)4-, (2-furyl)-, cyclohexyl-CH2-CH2-, (2-bromophenyl)-CH2-, phenyl-SO2-CH2-CH2-, (4-hydroxyphenyl)-CH2-, phenyl-CH2-NH-, N-cyclohexyl-NH-, N-(2,6-difluorophenyl)-NH-, N-isopropyl-NH-, N-cyclohexyl-NH-, methoxy-CH2-CH2-, 2-methoxyphenyl-, phenoxy-CH2-CH2-, cyclohexyl-CH2-, 4-fluoro-phenyl-, phenyl-CH2-, cyclopentyl-CH2-, 4-methylphenyl-, 3, 4-dimethoxy-phenyl-, 2,5-dimethoxyphenyl-, 2,3,4,5,6-pentamethylphenyl-, 3-fluoro-2, 4-dimethylphenyl-, 4-fluoro-3, 5-dimethylphenyl-, 2, 4, 5-trichlorophenyl-, 4-tert-butylphenyl-, 2,3-dichlorophenyl-, (3-thienyl)-CH-(CH3)-, (2-thienyl)-CH-(CH3)-, 4-chlorophenyl-, 4-trifluoromethylphenyl-, 4-methoxyphenyl-, 2-chlorophenyl-, 2-fluorophenyl-, (2-thienyl)-(CH2)4-, phenyl-(CH2)4-, (2-thienyl)-CH2-CH2-, (2-chlorophenyl)-CH2-, 3,4-dichlorophenyl-, 2,4-dichlorophenyl-, 2,5-dichlorophenyl-, 2,4-difluorophenyl-, (2-fluorophenyl)-CH2-, (2-furyl)-CH2-, 2-chlorophenyl-, 2-chloro-6-methylphenyl-, (2-thienyl)-CH2-, (3-thienyl)-CH2-, 4-acetylaminophenyl-, 3-trifluoromethylphenyl-, 2-tri-fluoromethylphenyl-, 5-methyl-2-thienyl-, 2-trifluoromethoxyphenyl, 2-trifluoromethylphenyl-CH2, 2-trifluoromethoxyphenyl-CH2-, 2-trifluoromethylthiophenyl-CH2-, 2-naphthyl-, 4-fluoro-3, 5-dimethylphenyl-, (5-methyl-2-thienyl)-CH-(CH3)-;
R (2) is
benzyl, CH2-CH2-OH, 4-methoxybenzyl, 4-chlorobenzyl, phenyl, cyclohexyl, 2-methoxybenzyl, 2-hydroxyethyl, 2-chlorobenzyl, isobutyl, 1(S)-phenylethyl, 4-methylbenzyl, 3-methoxybenzyl, 3,4-methylenedioxybenzyl, 5-fluoro- (2-methoxy)ethoxybenzyl, 1(R)-phenylethyl, 4-trifluoromethylbenzyl, 2-(4-methoxyphenyl)ethyl, 1(S)-(4-methylphenyl)ethyl, 2-furylmethyl, 1(R)-(4-methylphenyl)ethyl, 4-(dimethylamino)benzyl; 2-(2-thienyl)ethyl, 2-phenoxyethyl, 2,3-dichlorobenzyl, cyclopropylmethyl, 3,4,5-trimethoxy-benzyl, (4-pyridyl)methyl, 4-fluorobenzyl, 2,4-dimethoxybenzyl, 3-phenyl-propyl, 2-methoxyethyl, methyl, ethyl, (2-pyridyl)methyl, cyclopropyl, (2-benzimidazolyl)methyl, 1-methoxycarbonyl-2-phenylethyl, (3,4-dihydroquinazolin-2-yl)methyl, propargyl, (2-thienyl)methyl, cyclohexylmethyl, 3, 4-dichlorobenzyl, phenyl-CH(COOC(CH3)3), phenyl-CH(COOCH3), 2,4-dichlorobenzyl, (1-naphthyl)methyl, 2-(4-H2NSO2-phenyl)ethyl, 3-chlorobenzyl, 2,3-dichlorobenzyl, 3-methylbenzyl, 2-methylbenzyl, (CH3)2C=CH-CH2-CH2-C(CH3)=CH-CH2, 1-indanyl, 1,2,3,4-tetrahydro-1-naphthyl, 2-fluorobenzyl, 3-fluorobenzyl, (1,2,3,4-tetrahydro-2-furyl)methyl, 2, 4-difluorophenyl, 4-tert-butylphenyl, 2,6-dichlorophenyl, 3-chloro-4-fluorophenyl, 4-chloro-2, 5-dimethyloxyphenyl, 2-cyanophenyl, 2-chlorophenyl, 2,3-dichlorophenyl, 4-fluorophenyl, 2,3-dichlorophenyl, 4-(4-fluorophenoxy)phenyl, 3-trifluoromethylphenyl, 2-chloro-4-cyanophenyl, 2-acetylphenyl, (1-phenylcyclopentyl)methyl, 2-ethyl-2-(4-methoxyphenyl)butyl, [3-(pyrrolidine-1-carbonyl)-4,5-dihydroisoxazol-5-yl]methyl, 4-acetylamino-2-methylphenyl, 5-acetylamino-2-methylphenyl, 4-acetylamino-2, 6-dimethylphenyl, 4-chlorophenyl, 3-acetylphenyl, 4-trifluoromethylphenyl, 2,5-difluorophenyl, 4-isopropylphenyl, 4-methoxyphenyl, 4-ethoxyphenyl, 5-chloro-2-methoxyphenyl, 2-fluorophenyl, 1-naphthyl, 2-trifluoromethylphenyl, 2-ethylphenyl, 2,4-dimethylphenyl, 2,4,5-trimethylphenyl, 2,5-dimethylphenyl, 3-chloro-2-methylphenyl, 4-chloro-2-methylphenyl, 3-trifluoromethylphenyl, 2,6-dimethylphenyl, 2,3-dimethylphenyl, 4-ethoxyphenyl, 3,5-dichlorophenyl, 3,4-dichlorophenyl, 3,4-dimethoxyphenyl, 4-bromophenyl, 2-chlorophenyl, 2-methylphenyl, 2-methoxyphenyl, 2, 6-diethylphenyl, 2,6-diisopropylphenyl, 2-biphenylyl, 2-naphthyl, 4-methylphenyl, 2,4-dimethoxyphenyl, 2,5-dimethylphenyl, 2-chloro-4-methoxyphenyl, 2-methoxy-5-methylphenyl, 3-methoxy-4-methylphenyl, 2-methoxyphenyl, 3-hydroxy-phenyl, 2-chloro-5-trifluoromethylphenyl, 1-acetylamino-2-naphthyl, 4-ethyl-phenyl, 2,5-dichlorophenyl, 4-trifluoromethoxyphenyl, 2-chlorophenyl, 2-methyl-1-naphthyl-, 1,3-dihydro-1-oxobenzo[c]furan-6-yl, neopentyl, isopropyl, 3-nitrobenzyl, 2-ethoxybenzyl, 2,2,2-trifluoroethyl, 2-(3-tri-fluoromethylphenyl)ethyl, 1-indanyl, 2-phenylethyl, (2-naphthyl)-methyl, 2-phenylethyl, phenyl-CH(COOH)-, 3-phenylbenzyl-, 4-(4-chloro-phenoxy)phenyl, 5-chloro-2-methylphenyl, 2-fluorophenyl, 1-naphthyl-;
R (3) is
hydrogen;
R (4) is
hydrogen;
R (5) is
hydrogen, chloro, -SO2CH3, (2-methoxy)ethoxy-;
R (6) is
hydrogen;
R (7) is
hydrogen, methyl-, chloro;
X is
-CO-, -CO-CO-, -CO-NH-, -SO2-;
or its physiologically tolerable salts.

7. A compound of the formula I as claimed in claim 5, in which:
R (1) is 2-chlorophenyl;
R (2) is 4-methylbenzyl;
R (3)- R(7) is hydrogen;
X is -CO-,
or its physiologically tolerable salts.

8. A compound of the formula I as claimed in claim 5, this being 4'-{[benzylthiophene-2-sulfonyl)amino]methyl}-3'-chlorobiphenyl-2-sulfonylcyanamide, or its physiologically tolerable salts.

9. A compound of the formula (I) as claimed in one or more of claims 1 to 8 and/or its physiologically tolerable salts for use as a pharmaceutical.

10. A pharmaceutical preparation which comprises an efficacious amount of a compound of the formula (I) as claimed in one or more of claims 1 to 8 and/or a physiologically tolerable salt thereof.

11. A pharmaceutical preparation as claimed in claim 10, which additionally comprises an efficacious amount of an NHE inhibitor and/or an active substance from another class of cardiovascular active compound, and/or its physiologically tolerable salts.

12. A compound of the formula I as claimed in one or more of claims 1 to 8 and/or its physiologically tolerable salts for use as an inhibitor of the sodium-dependent bicarbonate/chloride exchanger.

13. A compound of the formula I as claimed in one or more of claims 1 to 8 and/or its physiologically tolerable salts for use in the therapy and/or prophylaxis of cardiac infarct, of angina pectoris, of illnesses caused by ischemic conditions, of impaired respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

14. A compound of the formula I as claimed in one or more of claims 1 to 8 and/or its physiologically tolerable salts for use in the therapy and/or prophylaxis of cancer.

15. A pharmaceutical preparation as claimed in claim 10 and/or 11 for use in the therapy and/or prophylaxis of cardiac infarct, of angina pectoris, of illnesses caused by ischemic conditions, of impaired respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

16. A pharmaceutical preparation as claimed in claim 10 and/or 11 for use in the therapy and/or prophylaxis of cancer.

## Revendications

1. Composé de formule (I) 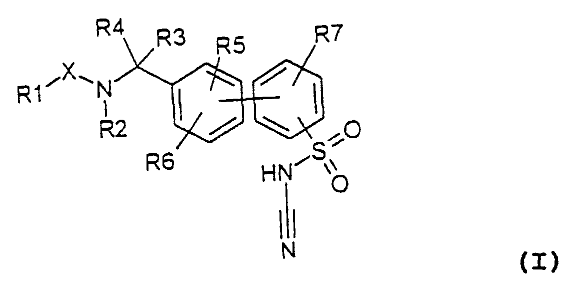 où les symboles possèdent la signification suivante :
R(1) représente des groupes
1. alkyle présentant 1, 2, 3, 4, 5, 6, 7 ou 8 atomes de carbone ;
2. alkyle présentant 1, 2, 3, 4, 5, 6, 7 ou 8 atomes de carbone, où un à tous les atomes d'hydrogène sont remplacés par des atomes de fluor ;
3. alcényle présentant 2, 3, 4, 5, 6, 7, 8, 9, 10, 11 ou 12 atomes de carbone ; ou
4. -CₙH₂ₙ₋ₙₙ-Y,
nn vaut zéro ou 2 ; et
n vaut zéro, 1, 2, 3 ou 4 ; n n'étant pas égal à zéro ou 1, lorsque nn est égal à 2 ;
5. -CₙH₂ₙ₋ₙₙ-Y,
nn vaut zéro ou 2 ; et
n vaut 1, 2, 3 ou 4 ; n n'étant pas égal à 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série
1. aryle présentant 6, 7, 8, 9, 10, 11, 12, 13 ou 14 atomes de carboné ;
2. amino ;
3. N(R(22)R(23) ;
4. alkoxycarbonyle ;
5. COOR(16) ;
6. alkyle présentant 1, 2, 3 ou 4 atomes de carbone ;
7. (aryl en C₆-C₁₄)-(alkyl en C₁-C₄)-carbonyle ;
R(2) représente des groupes
1. hydrogène ;
2. alkyle présentant 1, 2, 3, 4, 5, 6, 7 ou 8 atomes de carbone ;
3. alkyle présentant 1, 2, 3, 4, 5, 6, 7 ou 8 atomes de carbone, où un à tous les atomes d'hydrogène sont remplacés par des atomes de fluor ;
4. alcényle présentant 2, 3, 4, 5, 6, 7, 8, 9, 10, 11 ou 12 atomes de carbone ;
5. alcynyle présentant 2, 3, 4, 5, 6, 7 ou 8 atomes de carbone
6. -CₙH₂ₙ₋ₙₙ-Z,
nn vaut zéro ou 2 ; et
n vaut 1, 2, 3 ou 4, n n'étant pas égal à zéro ou 1, lorsque nn est égal à 2 ;
7. -CₙH₂ₙ₋ₙₙ-Z,
nn vaut zéro ou 2 ; et
n vaut 1, 2, 3 ou 4, n n'étant pas égal à 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série
1. aryle présentant 6, 7, 8, 9, 10, 11, 12, 13 ou 14 atomes de carbone ;
2. amino ;
3. N(R(22)R(23) ;
4. (alkoxy en C₁-C₄)carbonyle ;
5. COOR(16) ;
6. alkyle présentant 1, 2, 3 ou 4 atomes de carbone ;
R(3) et R(4) représentent, indépendamment l'un de l'autre, des groupes hydrogène ou alkyle présentant 1, 2, 3, 4, 5, 6, 7 ou 8 atomes de carbone ;
R(5), R(6) et R(7) représentent, indépendamment l'un de l'autre, des groupes hydrogène, alkyle présentant 1, 2, 3, 4, 5, 6, 7 ou 8 atomes de carbone, F, Cl, Br, I, CF₃, -CN, -NO₂, SO_{q}-R(8), CO-R(21) ou O-R(10) ;
R(8) représente des groupes alkyle présentant 1, 2, 3, 4, 5, 6, 7 ou 8 atomes de carbone, NR(11)R(12) ou phényle, qui est non substitué ou substitué par 1, 2 ou 3 restes identiques ou différents pris dans la série des restes F, Cl, Br, I, CF₃, méthyle, méthoxy, hydroxy ou NR(11)R(12) ;
R(9) et R(21) représentent, indépendamment l'un de l'autre, des groupes hydrogène, alkyle présentant 1, 2, 3, 4, 5, 6, 7 ou 8 atomes de carbone ou OR(13) ;
R(10) représente des groupes hydrogène, alkyle présentant 1, 2, 3, 4, 5, 6, 7 ou 8 atomes de carbone éventuellement substitué par un substituant alkoxy en C₁-C₄ ; ou phényle qui est non substitué ou substitué par 1, 2 ou 3 restes identiques ou différents pris dans la série des restes F, Cl, Br, I, CF₃, méthyle, méthoxy, hydroxy ou NR(11)R(12) ;
R(11), R(12), R(19) et R(20) représentent, indépendamment l'un de l'autre, des groupes hydrogène, alkyle présentant 1, 2, 3 ou 4 atomes de carbone ou alcanoyle en C₁-C₄ ;
R(13) représente des groupes hydrogène ou alkyle présentant 1, 2, 3, 4, 5, 6, 7 ou 8 atomes de carbone ;
X représente des groupes carbonyle, -CO-NH-, -CO-CO- ou sulfonyle ;
Y et Z représentent, indépendamment l'un de l'autre, des groupes
1. aryle présentant 6, 7, 8, 9, 10, 11, 12, 13 ou 14 atomes de carbone ;
2. un reste défini comme au point 1., qui est substitué par 1, 2, 3, 4 ou 5 restes identiques ou différents pris dans la série des restes alkyle présentant 1, 2, 3, 4, 5, 6, 7 ou 8- atomes de carbone ; aryle présentant 6, 7, 8, 9, 10, 11, 12, 13 ou 14 atomes de carbone, F, Cl, Br, I, CF₃, SO_{q}-R(18), OR(16), NR(19)R(20), -CN, NO₂ ou CO-R(9) ; ou deux restes conjointement formant un reste hétérocyclyle condensé ;
3. hétéroaryle présentant 1, 2, 3, 4, 5, 6, 7, 8 ou 9 atomes de carbone ;
4. un reste comme défini au point 3., qui est substitué par 1, 2 ou 3 restes identiques ou différents pris dans la série des restes F, Cl, Br, I, CF₃, CH₃, méthoxy, hydroxy ou NR(11)R(12) ;
5. cycloalkyle présentant 3, 4, 5, 6, 7, 8, 9 ou 10 atomes de carbone ;
6. un reste défini comme au point 5., substitué par un substituant aryle présentant 6, 7, 8, 9, 10, 11, 12, 13 ou 14 atomes de carbone ;
7. O-R(14) ;
8. O-R(17) ;
9. -SO₂-R(14) ;
10. arylalkylcarbonyle ; ou
11. hétérocyclyle ;
R(14) et R(17) représentent, indépendamment l'un de l'autre, des groupes
1. hydrogène ;
2. alkyle présentant 1, 2, 3, 4, 5, 6, 7 ou 8 atomes de carbone ;
3. alcényle présentant 2, 3, 4, 5, 6, 7, 8, 9, 10, 11 ou 12 atomes de carbone ;
4. -CₙH₂ₙ₋ₙₙ-phényle,
nn vaut zéro ou 2 ; et
n vaut zéro, 1, 2, 3 ou 4 ; n n'étant pas égal à zéro ou 1, lorsque nn est égal à 2 ;
5. un reste défini comme au point 4., le fragment phényle étant substitué par 1, 2 ou 3 substituants identiques ou différents pris dans la série des restes alkyle présentant 1, 2, 3, 4, 5, 6, 7 ou 8 atomes de carbone, F, Cl, Br, I, CF₃, SO_{q}-R(15), OR(16), NR(11)R(12), -CN, NO₂ ou CO-R(9) ; ou
R(15) et R(18) représentent, indépendamment l'un de l'autre, des groupes
alkyle présentant 1, 2, 3 ou 4 atomes de carbone, alkyle présentant 1, 2, 3 ou 4 atomes de carbone, où un à tous les atomes d'hydrogène sont remplacés par des atomes de fluor, ou NR(11)R(12) ;
R(16) représente des groupes
1. hydrogène,
2. alkyle présentant 1, 2, 3 ou 4 atomes de carbone,
3. alkyle présentant 1, 2, 3 ou 4 atomes de carbone substitué par un substituant alkoxy en C₁-C₄,
4. alkyle présentant 1, 2, 3 ou 4 atomes de carbone, où un à tous les atomes d'hydrogène sont remplacés par des atomes de fluor ;
5. aryle présentant 6, 7, 8, 9, 10, 11, 12, 13 ou 14 atomes de carbone ;
6. un reste défini comme au point 5., qui est substitué par 1, 2 ou 3 restes identiques ou différents pris dans la série des restes F, Cl, Br, I, CF₃, NR(19)R(20), -CN, NO₂ ;
R(22) et R(23) représentent indépendamment l'un de l'autre, des groupes hydrogène ou CO-OR(24) ;
R(24) représente des groupes hydrogène, alkyle présentant 1, 2, 3, 4, 5, 6, 7 ou 8 atomes de carbone ou -CₙH₂ₙ-phényle, n est égal à 1, 2, 3 ou 4 ;
q valent, indépendamment l'un de l'autre, zéro, 1 ou 2 ;
ainsi que leurs sels tolérés du point de vue physiologique.

2. Composés de formule (I) selon la revendication 1, dans laquelle
R(1) représente des groupes
1. alkyle présentant 1, 2, 3, 4 ou 5 atomes de carbone ;
2. alkyle présentant 1, 2, 3, 4 ou 5 atomes de carbone, où un à tous les atomes d'hydrogène sont remplacés par des atomes de fluor ;
3. alcényle présentant 2, 3, 4, 5, 6, 7, 8, 9, 10, 11 ou 12 atomes de carbone ;
4. -CₙH₂ₙ₋ₙₙ-Y,
nn vaut zéro ou 2 ; et
n vaut zéro, 1, 2, 3 ou 4 ; n n'étant pas égal à zéro ou 1, lorsque nn est égal à 2 ;
5. -CₙH₂ₙ₋ₙₙ-Y,
nn vaut zéro ou 2 ; et
n vaut 1, 2, 3 ou 4 ; n n'étant pas égal à 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série
1. aryle présentant 6, 7, 8, 9, 10, 11, 12, 13 ou 14 atomes de carbone ;
2. amino ;
3. N(R(22)R(23) ;
4. alkoxycarbonyle ;
5. COOR(16) ;
6. alkyle présentant 1, 2, 3 ou 4 atomes de carbone ;
7. (aryl en C₆-C₁₄)-(alkyl C₁-C₄)-carbonyle ;
R(2) représente des groupes
1. hydrogène
2. alkyle présentant 1, 2, 3, 4 ou 5 atomes de carbone ;
3. alkyle présentant 1, 2, 3, 4 ou 5 atomes de carbone, où un à tous les atomes d'hydrogène sont remplacés par des atomes de fluor ;
4. alcényle présentant 2, 3, 4, 5, 6, 7, 8, 9, 10, 11 ou 12 atomes de carbone ;
5. alcynyle présentant 2, 3, 4 ou 5 atomes de carbone ;
6. -CₙH₂ₙ₋ₙₙ-Z,
nn vaut zéro ou 2 ; et
n vaut zéro, 1, 2, 3 ou 4, n n'étant pas égal à zéro ou 1, lorsque nn est égal à 2 ;
7. -CₙH₂ₙ₋ₙₙ-Y,
nn vaut zéro ou 2 ; et
n vaut 1, 2, 3 ou 4, n n'étant pas égal à zéro ou 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série des restes
1. aryle présentant 6, 7, 8, 9, 10, 11, 12, 13 ou 14 atomes de carbone ;
2. amino ;
3. N(R(22)R(23) ;
4. alkoxycarbonyle en C₁-C₄ ;
5. COOR(16) ;
6. alkyle présentant 1, 2, 3 ou 4 atomes de carbone ;
R(3) et R(4) représentent, indépendamment l'un de l'autre, des groupes
hydrogène ou alkyle présentant 1, 2, 3 ou 4 atomes de carbone ;
R(5), R(6) et R(7) représentent, indépendamment l'un de l'autre, des groupes
hydrogène, alkyle présentant 1, 2, 3 ou 4 atomes de carbone, F, Cl, Br, CF₃, -CN, SO_{q}-R(8), CO-R(21) ou O-R(10) ;
R(8) représente des groupes alkyle présentant 1, 2, 3 ou 4 atomes de carbone, NR(11)R(12) ou phényle, qui est non substitué ou substitué par 1 ou 2 restes identiques ou différents pris dans la série des restes F, Cl, Br, CF₃, méthyle, méthoxy, hydroxy ou NR(11)R(12) ;
R(9) et R(21) représentent, indépendamment l'un de l'autre, des groupes
hydrogène, alkyle présentant 1, 2, 3 ou 4 atomes de carbone ou OR(13) ;
R(10) représente des groupes hydrogène, alkyle présentant 1, 2, 3 ou 4 atomes de carbone éventuellement substitué par un substituant alkoxy en C₁-C₄ ; ou phényle qui est non substitué ou substitué par 1 ou 2 restes identiques ou différents pris dans la série des restes F, Cl, Br, CF₃, méthyle, méthoxy, hydroxy ou NR(11)R(12) ;
R(11), R(12), R(19) et R(20) représentent, indépendamment l'un de l'autre, des groupes hydrogène, alkyle présentant 1, 2, 3 ou 4 atomes de carbone ou alcanoyle en C₁-C₄ ;
R(13) représente des groupes hydrogène ou alkyle présentant 1, 2, 3 ou 4 atomes de carbone ;
X représente des groupes carbonyle, -CO-NH-, -CO-CO- ou sulfonyle ;
Y et Z représentent, indépendamment l'un de l'autre, des groupes
1. phényle, 1-naphtyle ou 2-naphtyle ;
2. un reste défini comme au point 1., qui est substitué par 1, 2, 3, 4 ou 5 restes identiques ou différents pris dans la série des restes alkyle présentant 1, 2, 3 ou 4 atomes de carbone, phényle, 1-naphtyl ou 2-naphtyle, F, Cl, Br, CF₃, SO_{q}-R(18), OR(16), NR(19)R(20), -CN ou CO-R(9) ; ou deux restes conjointement formant un reste hétérocyclyle condensé ;
3. hétéroaryle présentant 1, 2, 3, 4, 5, 6, 7, 8 ou 9 atomes de carbone ;
4. un reste défini comme au point 3., qui est substitué par 1 ou 2 restes identiques ou différents pris dans la série des restes F, Cl, Br, CF₃, CH₃, méthoxy, hydroxy ou NR(11)R(12) ;
5. cycloalkyle présentant 3, 4, 5, 6, 7, 8, 9 ou 10 atomes de carbone ;
6. un reste défini comme au point 5., substitué par un substituant phényle, 1-naphtyle ou 2-naphtyle ;
7. O-R(14) ;
8. O-R(17) ;
9. -SO₂-R(14) ;
10. arylalkylcarbonyle ; ou
11. hétérocyclyle ;
R(14) et R(17) représentent, indépendamment l'un de l'autre, des groupes
1. hydrogène ;
2. alkyle présentant 1, 2, 3 ou 4 atomes de carbone ;
3. alcényle présentant 2, 3, 4, 5 ou 6 atomes de carbone ;
4. -CₙH₂ₙ₋ₙₙ-phényle,
nn vaut zéro ou 2 ; et
n vaut zéro, 1, 2, 3 ou 4 ; n n'étant pas égal à zéro ou 1, lorsque nn est égal à 2 ;
5. un reste défini comme au point 4., le fragment phényle étant substitué par 1, 2 ou 3 substituants identiques ou différents pris dans la série des restes alkyle présentant 1, 2, 3 ou 4 atomes de carbone, F, Cl, Br, CF₃, SO_{q}-R(15), OR(16), NR(11)R(12), -CN ou CO-R(9) ; ou
R(15) et R(18) représentent, indépendamment l'un de l'autre, des groupes
alkyle présentant 1, 2, 3 ou 4 atomes de carbone, alkyle présentant 1, 2, 3 ou 4 atomes de carbone, où un à tous les atomes d'hydrogène sont remplacés par des atomes de fluor, ou NR(11)R(12) ;
R(16) représente des groupes
1. hydrogène,
2. alkyle présentant 1, 2, 3 ou 4 atomes de carbone,
3. alkyle présentant 1, 2, 3 ou 4 atomes de carbone substitué par un substituant alkoxy en C₁-C₄,
4. alkyle présentant 1, 2, 3 ou 4 atomes de carbone, où un à tous les atomes d'hydrogène sont remplacés par des atomes de fluor ;
5. phényle, 1-naphtyle ou 2-naphtyle ; un reste défini comme au point 5., qui est substitué par 1, 2 ou 3 restes identiques ou différents pris dans la série des restes F, Cl, Br, CF₃, NR(19)R(20), -CN, ;
R(22) et R(23) représentent, indépendamment l'un de l'autre, des groupes hydrogène ou CO-OR(24) ;
R(24) représente des groupes hydrogène, alkyle présentant 1, 2, 3 ou 4 atomes de carbone ou -CₙH₂ₙ-phényle n étant égal à 1, 2 ou 3 ;
q valent, indépendamment l'un de l'autre, zéro, 1 ou 2 ;
ainsi que leurs sels tolérés du point de vue physiologique.

3. Composés de formule (I) selon la revendication 1 et/ou 2, où
R(1) représente des groupes
1. alkyle présentant 1, 2, 3, 4 ou 5 atomes de carbone ;
2. alcényle présentant 2, 3 ou 4 atomes de carbone ;
3. -CₙH₂ₙ₋ₙₙ-Y,
Y représente des groupes
1. phényle ;
2. un reste défini comme au point 1., qui est substitué par 1, 2, 3, 4 ou 5 restes identiques ou différents pris dans la série des restes alkyle présentant 1, 2, 3 ou 4 atomes de carbone, F, Cl, Br, cyano, CF₃, hydroxy, NO₂, SO₂R(18), OR(16), SCF₃, NR(19)R(20), CO-R(9) ;
3. OR(14), ou
4. SO₂-R(14) ;
5. 1-naphtyle ou 2-naphtyle ;
6. un reste défini comme au point 5., qui est substitué par un reste pris dans la série des restes alkyle présentant 1, 2, 3 ou 4 atomes de carbone, F, Cl, CF₃, hydroxy, SO₂R(18), OR(16), NR(19)R(20), CO-R(9) ;
7. hétéroaryle présentant 1, 2, 3, 4, 5,- 6, 7, 8 ou 9 atomes de carbone ;
8. un reste défini comme au point 3., qui est substitué par un reste pris dans la série des restes F, Cl, CF₃, CH₃, méthoxy ou N(CH₃)₂ ;
9. cycloalkyle présentant 3, 4, 5, 6 ou 7 atomes de carbone ;
nn vaut zéro ou 2 ; et
n vaut zéro, 1, 2, 3 ou 4, n n'étant pas égal à zéro ou 1, lorsque nn est égal à 2 ;
4. -CₙH₂ₙ₋ₙₙ-Y,
Y représente des groupes
1. phényle ;
2. OR(14) ; ou
3. hétéroaryle ;
nn vaut zéro ou 2 ; et
n vaut 1, 2 ou 3, n n'étant pas égal à 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série des restes
1. aryle présentant 6, 7, 8, 9, 10, 11, 12, 13 ou 14 atomes de carbone, ou phénylacétyle ;
2. amino ;
3. NR(22)R(23) ; ou
4. alkyle présentant 1, 2, 3 ou 4 atomes de carbone ;
R(2) représente des groupes
1. hydrogène
2. alkyle présentant 1, 2, 3, 4 ou 5 atomes de carbone ;
3. alkyle présentant 1, 2, 3, 4 ou 5 atomes de carbone, où un à tous les atomes d'hydrogène sont remplacés par des atomes de fluor ;
4. alcényle présentant 2, 3, 4, 5, 6, 7, 8, 9 ou 10 atomes de carbone ;
5. alcynyle présentant 2, 3, 4 ou 5 atomes de carbone ;
6. -CₙH₂ₙ₋ₙₙ-Z,
Z représente des groupes
1. phényle ;
2. un reste défini comme au point 1., qui est substitué par 1, 2 ou 3 restes identiques ou différents pris dans la série des restes alkyle présentant 1, 2, 3 ou 4 atomes de carbone, phényle, F, Cl, Br, CF₃, SO₂R(18), OR(16), nitro, cyano, NR(19)R(20), CO-R(9), ou deux restes formant conjointement un reste méthylènedioxy ;
3. 1-naphtyle ou 2-naphtyle ;
4. un reste défini comme au point 3., qui est substitué par un reste pris dans la série des restes alkyle présentant 1, 2, 3 ou 4 atomes de carbone, F, Cl, CF₃, SO₂R(18), OR(16), nitro, cyano, NR(19)R(20) ou CO-R(9) ;
5. hétéroaryle présentant 1, 2, 3, 4, 5, 6, 7, 8 ou 9 atomes de carbone ;
6. un reste défini comme au point 5., qui est substitué par un reste pris dans la série des restes F, Cl, CF₃, CH₃, méthoxy, hydroxy ou N(CH₃)₂ ;
7. cycloalkyle présentant 3, 4, 5, 6, 7, 8, 9 ou 10 atomes de carbone ;
8. un reste défini comme au point 7., qui est substitué par un substituant phényle ;
nn vaut zéro ou 2 ; et
n vaut zéro, 1, 2 ou 3, n n'étant pas égal à zéro ou 1, lorsque nn est égal à 2 ; et
7. -CₙH₂ₙ₋ₙₙ-Z,
Z représente des groupes
1. phényle
2. un reste défini comme au point 1., qui est substitué par 1, 2 ou 3 restes identiques ou différents pris dans la série des restes alkyle présentant 1, 2, 3 ou 4 atomes de carbone, phényle, F, Cl, CF₃, SO₂R(18), OR(16), nitro, cyano, NR(19)R(20), CO-R(9) ;
nn vaut zéro ou 2 ; et
n vaut 1, 2 ou 3, n n'étant pas égal à 1, lorsque nn est égal à 2,
où 1, 2 ou 3 atomes d'hydrogène dans le reste bivalent -CₙH₂ₙ₋ₙₙ- sont remplacés, indépendamment l'un de l'autre, par un reste pris dans la série
1. alkoxycarbonyle en C₁-C₄ ;
2. COOR(16) ; ou
3. alkyle présentant 1, 2, 3 ou 4 atomes de carbone ;
8 . -CₙH₂ₙ-OR(17) ;
n vaut zéro, 1, 2 ou 3 ;
R(3) et R(4) représentent des groupes hydrogène ou méthyle ;
R(5), R(6) et R(7) représentent, indépendamment l'un de l'autre, des groupes hydrogène, alkyle présentant 1, 2, 3 ou 4 atomes de carbone, F, Cl, CF₃, -CN, SO₂-R(8), CO-R(21) ou O-R(10) ;
R(8) représente des groupes alkyle présentant 1, 2, 3 ou 4 atomes de carbone, N((H₃)₂ ou phényle, qui est non substitué ou substitué par un reste pris dans la série des restes F, Cl, CF₃, méthyle, méthoxy, hydroxy ou N(CH₃)₂ ;
R(9) et R(21) représentent, indépendamment l'un de l'autre, des groupes hydrogène, méthyle ou OR(13) ;
R(10) représente des groupes hydrogène, alkyle présentant 1, 2, 3 ou 4 atomes de carbone éventuellement substitué par un substituant alkoxy en C₁-C₄, ou phényle, qui est non substitué ou substitué par un reste pris dans la série des restes F, Cl, CF₃, méthyle, méthoxy ou N(CH₃)₂ ;
R(11) et R(12), représentent, indépendamment l'un de l'autre, des groupes hydrogène, alkyle présentant 1, 2, 3 ou 4 atomes de carbone ou alcanoyle en C₁-C₄ ;
R(13) représente des groupes hydrogène ou alkyle présentant 1, 2, 3 ou 4 atomes de carbone ;
X représente des groupes carbonyle, -CO-CO-, -NH-CO-, ou sulfonyle ;
R(14) représente des groupes,
1. hydrogène ;
2. alkyle présentant 1, 2, 3 ou 4 atomes de carbone ;
3. alcényle présentant 2, 3, 4, 5 ou 6 atomes de carbone ;
4. -CₙH₂ₙ₋ₙₙ-phényle,
nn vaut zéro ou 2 ; et
n vaut zéro, 1, 2, 3 ou 4 ; n n'étant pas égal à zéro ou 1, lorsque nn est égal à 2 ;
5. un reste défini comme au point 4., le fragment phényle étant substitué par 1, 2 ou 3 restes identiques ou différents pris dans la série des restes alkyle présentant 1, 2, 3 ou 4 atomes de carbone, F, Cl, Br, CF₃, SO_{q}-R(15), OR(16), NR(11)R(12), -CN ou CO-R(9) ;
R(15) représente des groupes alkyle présentant 1, 2, 3 ou 4 atomes de carbone ou N(CH₃)₂ ;
R(16) représente des groupes
1. hydrogène,
2. alkyle présentant 1, 2, 3 ou 4 atomes de carbone,
3. alkyle présentant 1, 2, 3 ou 4 atomes de carbone substitué par un substituant alkoxy en C₁-C₄,
4. alkyle présentant 1, 2, 3 ou 4 atomes de carbone, où un à tous les atomes d'hydrogène sont remplacés par des atomes de fluor ;
5. phényle, 1-naphtyle ou 2-naphtyle ;
6. un reste défini comme au point 5., qui est substitué par 1, 2 ou 3 restes identiques ou différents pris dans la série des restes F, Cl, Br, CF₃, NR(19)R(20), -CN, ;
R(17) représente des groupes
1. hydrogène
2. alkyle présentant 1, 2, 3 ou 4 atomes de carbone,
3. alcényle présentant 2, 3 ou 4 atomes de carbone ;
4. -CₙH₂ₙ₋ₙₙ-phényle,
nn vaut zéro ou 2 ; et
n vaut zéro, 1, 2, 3 ou 4 ; n n'étant pas égal à zéro ou 1, lorsque nn est égal à 2 ;
5. un reste défini comme au point 4., le fragment phényle étant substitué par un reste pris dans la série des restes alkyle présentant 1, 2, 3 ou 4 atomes de carbone, F, Cl, Br, CF₃, SO_{q}-R(15), OR(16), NR(11)R(12), -CN ou CO-R(9) ; ou
R(18) représente des groupes alkyle présentant 1, 2, 3 ou 4 atomes de carbone, alkyle présentant 1, 2, 3 ou 4 atomes de carbone, où un à tous les atomes d'hydrogène sont remplacés par des atomes de fluor, ou un groupe NR(11)R(12) ;
R(19) et R(20) représentent, indépendamment l'un de l'autre, des groupes
hydrogène, alkyle présentant 1, 2, 3 ou 4 atomes de carbone ou alcanoyle en C₁-C₄ ;
R(22) et R(23) représentent, indépendamment l'un de l'autre, des groupes hydrogène ou CO-OR(24) ;
R(24) représente des groupes hydrogène, alkyle présentant 1, 2, 3 ou 4 atomes de carbone ou -CₙH₂ₙ-phényle avec n égal à 1 ou 2 ;
q valent, indépendamment, zéro, 1 ou 2 ;
ainsi que leurs sels tolérés du point de vue physiologique.

4. Composés de formule (I) selon une ou plusieurs des revendications 1 à 3, où
R(1) représente des groupes
1. alkyle présentant 1, 2, 3, 4 ou 5 atomes de carbone ;
2. alcényle présentant 2, 3 ou 4 atomes de carbone ;
3. -CₙH₂ₙ₋ₙₙ-Y,
Y représente des groupes
1. phényle ;
2. un reste défini comme au point 1., qui est substitué par 1, 2, 3, 4 ou 5 restes identiques ou différents pris dans la série des restes alkyle présentant 1, 2, 3 ou 4 atomes de carbone, F, Cl, Br, cyano, CF₃, hydroxy, NO₂, SO₂R(18), OCH₃, OCF₃, SCF₃, N(CH₃)₂, NH-CO-CH₃, CO-R(9), phénoxy ou phénoxy substitué une ou plusieurs fois par des substituants halogènes ;
3. OR(14), ou
4. SO₂-R(14) ;
nn vaut zero ou 2 ; et
n vaut zéro, 1, 2, 3 ou 4, n n'étant pas égal à zéro ou 1, lorsque nn est égal à 2 ;
4 . -CₙH₂ₙ₋ₙₙ-Y,
Y représente des groupes
1. phényle ;
2. OR(14) ; ou
3. hétéroaryle, de préférence thiényle ;
nn vaut zéro
n vaut 1, 2 ou 3, n n'étant pas égal à 1, lorsque nn est égal à 2 ;
où 1, 2 ou 3 atomes d'hydrogène dans le reste bivalent -CₙH₂ₙ₋ₙₙ- sont remplacés, indépendamment l'un de l'autre, par un reste pris dans la série des restes
1. aryle présentant 6, 7, 8, 9, 10, 11, 12, 13 ou 14 atomes de carbone ; ou phénylacétyle ;
2. amino ;
3. NR(22)R(23) ; ou
4. alkyle présentant 1, 2, 3 ou 4 atomes de carbone ;
5. -CₙH₂ₙ₋-Y ;
Y représente des groupes
1. 1-naphtyle ou 2-naphtyle ;
2. un reste-défini comme au point 1., qui est substitué par un reste pris dans la série des restes alkyle présentant 1, 2, 3 ou 4 atomes de carbone, F, Cl, CF₃, SO₂R(18), OCH₃, N(CH₃)₂ ou CO-R(9) ;
3. hétéroaryle présentant 1, 2, 3, 4, 5, 6, 7, 8 ou 9 atomes de carbone, de préférence thiényle, benzothiophényle, indolyle ou furyle ;
4. un reste défini comme au point 3., qui est substitué par un reste pris dans la série des restes F, Cl, CF₃, CH₃, méthoxy ou N(CH₃)₂ ;
5. cycloalkyle présentant 3, 4, 5, 6 ou 7 atomes de carbone ;
n vaut zéro, 1, 2, 3 ou 4 ;
6. -CₙH₂ₙ₋-OR(14) ;
n vaut zéro, 1 ou 2 ;
R(2) représente des groupes
1. alkyle présentant 1, 2, 3, 4 ou 5 atomes de carbone ;
2. alkyle présentant 1, 2, 3, 4 ou 5 atomes de carbone, où un à tous les atomes d'hydrogène sont remplacés par des atomes de fluor ;
3. alcényle présentant 2, 3, 4, 5, 6, 7, 8, 9 ou 10 atomes de carbone ;
4. alcynyle présentant 2, 3, 4 ou 5 atomes de carbone ;
5. -CₙH₂ₙ₋ₙₙ-Z,
Z représente des groupes
1. phényle ;
2. un reste défini comme au point 1., qui est substitué par 1, 2 ou 3 restes identiques ou différents pris dans la série des restes alkyle présentant 1, 2, 3 ou 4 atomes de carbone, phényle, F, Cl, Br, CF₃, SO₂R(18), -OCH₃, -O(C₂H₄)OCH₃, éthoxy, hydroxy, nitro, cyano, N(CH₃)₂, -NH-CO-CH₃, CO-R(9), phénoxy ou phénoxy substitué une ou plusieurs fois par des substituants halogène ; deux restes formant conjointement un reste méthylènedioxy ;
nn vaut zéro ou 2 ;
n vaut zéro, 1, 2 ou 3, n n'étant pas égal à zéro ou 1, lorsque nn est égal à 2 ;
6. -CₙH₂ₙ₋ₙₙ-Z,
Z représente des groupes
1. phényle ;
2. un reste défini comme au point 1., qui est substitué par 1, 2 ou 3 restes identiques ou différents pris dans la série des restes alkyle présentant 1, 2, 3 ou 4 atomes de carbone, phényle, F, Cl, CF₃, SO₂R(18), -OCH₃, -O(C₂H₄)OCH₃, éthoxy, hydroxy, nitro, cyano, N(CH₃)₂ ou CO-R(9) ;
nn vaut zéro ou 2 ;
n vaut 1, 2 ou 3, n n'étant pas égal à 1, lorsque nn est égal à 2 ;
1, 2 ou 3 atomes d'hydrogène dans le reste bivalent -CₙH₂ₙ₋ₙₙ- étant remplacés, indépendamment l'un de l'autre, par un reste pris dans la série des restes
1. alkoxycarbonyle en C₁-C₄ ;
2. COOR(16) ; ou
3. alkyle présentant 1, 2, 3 ou 4 atomes de carbone ;
7. -CₙH₂ₙ₋-Z,
Z représente des groupes
1. 1-naphtyle ou 2-naphtyle ;
2. un reste défini comme au point 1., qui est substitué par un reste pris dans la série des restes alkyle présentant 1, 2, 3 ou 4 atomes de carbone, F, Cl, CF₃, SO₂R (18), OCH₃, -O(C₂H₄)OCH₃, éthoxy, hydroxy, nitro, cyano; N(CH₃)₂, -NHCOCH₃ ou CO-R(9) ;
3. hétéroaryle présentant 1, 2, 3, 4, 5, 6, 7, 8 ou 9 atomes de carbone, de préférence benzimidazolyle, pyridyle, thiényle, furyle, tétrahydrofuryle, pyrrolidinyle, pyrrolidine-1-carbonyl-4,5-dihydro-isoxazolyle, benzofuranyle, par exemple dihydro-1-oxo-benzo[c]furanyle, quinazolinyle ; par exemple 3,4-dihydroquinazolinyle;
4. un reste défini comme au point 3., qui est substitué par un reste pris dans la série des restes F, Cl, CF₃, CH₃, méthoxy, hydroxy ou N(CH₃)₂ ;
5. cycloalkyle présentant 3, 4, 5, 6, 7, 8, 9 ou 10 atomes de carbone ; de préférence des groupes cyclopropyle, cyclopentyle, cyclohexyle, 1,2,3,4-tétrahydronaphtyle ou indanyle ;
6. un reste défini comme au point 5., substitué par un substituant phényle ; de préférence phénylcyclopentyle ;
n vaut zéro, 1, 2 ou 3 ;
8. -CₙH₂ₙ-OR(17) ;
n vaut 2 ou 3 ;
R(3) et R(4) représentent un atome d'hydrogène ;
R(5), R(6) et R(7) représentent, indépendamment l'un de l'autre, des groupes hydrogène, alkyle présentant 1, 2, 3 ou 4 atomes de carbone, F, Cl, CF₃, SO₂-R(8), CO-R(21) ou O-R(10) ;
R(8) représente des groupes méthyle ou N(CH₃)₂ ;
R(9) et R(21) représentent, indépendamment l'un de l'autre, des groupes hydrogène, méthyle ou OR(13) ;
R(10) représente des groupes hydrogène, méthyle ou éthyle, éventuellement substitué par des substituants méthoxy, ou phényle, qui est non substitué ou substitué par un reste pris dans la série des restes F, Cl, CF₃, méthyle, méthoxy ou N(CH₃)₂ ;
R(13) représente des groupes hydrogène ou alkyle présentant 1, 2, 3 ou 4 atomes de carbone ;
X représente des groupes carbonyle, -CO-CO-, -NH-CO- ou sulfonyle ;
R(14) représente des groupes,
1. hydrogène ;
2. méthyle ou éthyle ;
3. alcényle présentant 2, 3, 4, 5 ou 6 atomes de carbone, de préférence allyle ;
4. -CₙH₂ₙ-phényle, n est égal à zéro ou 1 ;
5. un reste défini comme au point 4., le fragment phényle étant substitué par un reste pris dans la série des restes alkyle présentant 1, 2, 3 ou 4 atomes de carbone, F, Cl, CF₃, SO₂R(15), OCH₃, N((H₃)₂ ou CO-R(9) ; ou
6. alcényle présentant 2, 3 ou 4 atomes de carbone ;
R(15) représente des groupes méthyle ou N(CH₃)₂ ;
R(16) représente des groupes hydrogène ou alkyle présentant 1, 2, 3 ou 4 atomes de carbone ;
R(17) représente des groupes
1. hydrogène ;
2. méthyle ;
3. -CₙH₂ₙ-phényle, n étant égal à zéro ou 1 ;
4. un reste défini comme au point 3., le fragment phényle étant substitué par un reste pris dans la série des restes alkyle présentant 1, 2, 3 ou 4 atomes de carbone, F, Cl, CF₃, SO₂-R(15), OCH₃, N(CH₃)₂ ou CO-R(9) ; ou
5. alcényle présentant 2, 3 ou 4 atomes de carbone ;
R(18) représente des groupes méthyle, CF₃, amino ou N(CH₃)₂ ;
R(22) et R(23) représentent, indépendamment l'un de l'autre, des groupes hydrogène ou CO-OR(24) ;
R(24) représente des groupes hydrogène, alkyle présentant 1, 2, 3 ou 4 atomes de carbone ou -CₙH₂ₙ-phényle avec n égal à 1 ou 2 ;
ainsi que leurs sels tolérés du point de vue physiologique.

5. Composés de formule (I) selon une ou plusieurs des revendications 1 à 4, ces composés représentant un composé de formule Ia 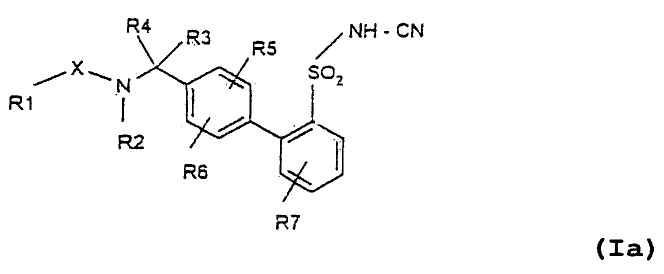 où les restes X et R(1) à R(7) possèdent les significations données aux revendications 1 à 4, ainsi que leurs sels tolérés du point de vue physiologique.

6. Composés de formule (I) selon une ou plusieurs des revendications 1 à 6, où :
R(1) représente des groupes CH₃-CH₂-CH₂-, cyclohexyl-CH₂-CH₂-, cyclohexyle, 3-méthoxy-phényl-CH₂-, 2-méthylphényle-CH₂-, 2-méthylphényle, 2-thiényl-CH₂-, 4-t-butyl-phényle, 2-fluorophényle, allyl-O-, 3,4,5-triméthoxy-phényle, 2-chlorophényle, phényle, CH₃-, E-CH₃-CH=CH, CH₃-(CH₂)₃-, phényl-CH₂-CH₂-, 4-méthyl-phényle, benzo[b)thiophén-2-yle, thiényl-2-yle, (phényl)CH₂-, phényl-CH₂-CO-CH(phényle), (4-chlorophényl)-CH₂-, (CH₃)₃C-CH₂-, phényl-O-CH₂-, (4-méthoxyphényl)-CH₂-, 2-thiényl-CH₂-CH₂-CH₂-, (2-thiényle), (indol-3-yl)-CH₂-, benzo[b]-thiophén-3-yl)-CH₂-, (3-thiényl)-CH₂-, phényl-CH(NH₂)-, phényl-CH(NHCO-O-benzyle), (2-nitrophényl)-CH₂-, phényl-(CH₂)₄-, (2-furyle), cyclohexyl-CH₂-CH₂-, (2-bromophényl)-CH₂-, phényl-SO₂-CH₂-CH₂-, (4-hydroxyphényl)-CH₂-, phényl-CH₂-NH-, N-cyclohexyl-NH-, N-(2,6-difluorophényl)NH-, N-iso-propyl-NH-, N-cyclohexyl-NH-, méthoxy-CH₂-CH₂-, 2-méthoxy-phényle, phénoxy-CH₂-CH₂-, cyclohexyl-CH₂-, 4-fluorophényle, phényl-CH₂-, cyclopentyl-CH₂-, 4-méthylphényle, 3,4-diméthoxyphényle, 2,5-diméthoxy-phényle, 2,3,4,5,6-pentaméthylphényle, 3-fluoro-2,4-diméthylphényle, 4-fluoro-3,5-diméthylphényle, 2,4,5-trichlorphényle, 4-tert-butylphényle, 2,3-dichloro-phényle, (3-thiényl)-CH-(CH₃)-, (2-thiényl)-CH₂-(CH₃)-, 4-chlorophényle, 4-trifluorométhyl-phényle, 4-méthoxy-phényle, 2-chlorophényle, 2-fluorophényle, (2-thiényl)-(CH₂)₄-, phényl-(CH₂)₄-, (2-thiényl)-CH₂-CH₂-, (2-chlorophényl)-CH₂-, 3,4-dichlorophényle, 2,4-dichlorophényle, 2,5-dichlorophényle, 2,4-difluoro-phényle, (2-fluorophényl)-CH₂-, (2-furyl)-CH₂-, 2-chlorophényle, 2-chloro-6-méthylphényle, (2-thiényl)-CH₂-, (3-thiényl)-CH₂-, 4-acétaminophényle, 3-trifluorométhyl-phényle, 2-trifluorométhyl-phényle, 5-méthyl-2-thiényle, 2-trifluorométhoxyphényle, 2-trifluorométhylphényl-CH₂-, 2-trifluorométhoxyphényl-CH₂-, 2-trifluorométhoxythiophényl-CH₂-, 2-naphtyle, 4-fluoro-3,5-diméthylphényle, (5-méthyl-2-thiényl)-CH-(CH₃)-,
R(2) représente des groupes benzyle, CH₂-CH₂-OH, 4-méthoxybenzyle, 4-chlorobenzyle, phényle, cyclohexyle, 2-méthoxybenzyle, 2-hydroxyéthyle, 2-chloro-benzyle, isobutyle, 1(S)-phényléthyle, 4-méthyl-benzyle, 3-méthoxy-benzyle, 3,4-mèthylènedioxy-benzyle, 5-fluoro-(2-méthoxy)éthoxybenzyle, 1(R)-phényl-éthyle, 4-trifluorométhylbenzyle, 2- (4-méthoxyphényl)éthyle, 1(S)-(4-méthylphényl)-éthyle, 2-furylméthyle, 1(R)-(4-méthylphényl)-éthyle, 4-(diméthylamino)-benzyle, 2-(2-thiényl)éthyle, 2-phénoxyéthyle, 2,3-dichlorobenzyle, cyclopropylméthyle, 3,4,5-triméthoxybenzyle, (4-pyridyl)-méthyle, 4-fluorobenzyle, 2,4-diméthoxybenzyle, 3-phénylpropyle, 2-méthoxy-éthyle, méthyle, éthyle, (2-pyridyl)-méthyle, cyclopropyle, (2-benzimidazolyl)-méthyle, 1-méthoxycarbonyl-2-phényl-éthyle, (3,4-dihydro-quinazolin-2-yl)-méthyle, propargyle, (2-thiényl)-méthyl, cyclohexyl-méthyle, 3,4-dichlorobenzyle, phényl-CH(COOC(CH₃)₃), phényl-CH(COOCH₃), 2,4-dichloro-benzyle, (1-naphtyl)-méthyle, 2-(4-H₂NSO₂-phényl)éthyle, 3-chlorobenzyle, 2,3-dichloro-benzyle, 3-méthyl-benzyle, 2-méthyl-benzyle, (CH₃)₂-C=CH-CH₂-CH₂-C(CH₃)=CH-CH₂, 1-indanyle, 1,2,3,4-tétrahydro-1-naphtyle, 2-fluorobenzyle, 3-fluorobenzyle, (1,2,3,4-tétrahydro-2-furyl)-méthyle, 2,4-difluorophényle, 4-tert-butylphényle, 2,6-dichlorophényle, 3-chloro-4-fluorophényle, 4-chloro-2,5-diméthyloxyphényle, 2-cyanophényle, 2-chloro-phényle, 2,3-dichlorophényle, 4-fluorophényle, 2,3-dichlorophényle, 4-(4-fluorophénoxy)-phényle, 3-trifluorométhylphényle, 2-chloro-4-cyanophényle, 2-acétyl-phényle, (1-phénylcyclopentyl)-méthyle, 2-éthyl-2-(4-méthoxyphényl)-butyle, [3-(pyrrolidine-1-carbonyl)-4,5-dihydro-isoxazol-5-yl]-méthyle, 4-acétyl-amino-2-méthylphényle, 5-acétylamino-2-méthylphényle, 4-acétylamino-2,6-diméthylphényle, 4-chlorophényle, 3-acétylphényle, 4-trifluorométhylphényle, 2,5-difluorophényle, 4-isopropylphényle, 4-méthoxyphényle, 4-éthoxyphényle, 5-chloro-2-méthoxyphényle, 2-fluoro-phényle, 1-naphtyle, 2-trifluorométhyl-phényle, 2-éthylphényle, 2,4-diméthylphényle, 2,4,5-triméthyl-phényle, 2,5-diméthylphényle, 3-chloro-2-méthyl-phényle, 4-chloro-2-méthylphényle, 3-trifluorométhyl-phényle, 2,6-diméthylphényle, 2,3-diméthylphényle, 4-éthoxyphényle, 3,5-dichlorophényle, 3,4-dichloro-phényle, 3,4-diméthoxyphényle, 4-bromophényle, 2-chlorophényle, 2-méthylphényle, 2-méthoxyphényle, 2,6-diéthylphényle, 2,6-diisopropylphényle, 2-biphénylyle, 2-naphtyle, 4-méthylphényle, 2,4-diméthoxyphényle, 2,5-diméthylphényle, 2-chloro-4-méthoxyphényle, 2-méthoxy-5-méthylphényle, 3-méthoxy-4-méthylphényle, 2-méthoxyphényle, 3-hydroxyphényle, 2-chloro-5-tri-fluorométhyl-phényle, 1-acétylamino-2-naphtyle, 4-éthylphényle, 2, 5-dichlorophényle, 4-trifluoro-méthoxyphényle, 2-chlorophényle, 2-méthyl-1-naphtyle, 1,3-dihydro-1-oxo-benzo[c]furan-6-yle, néopentyle, isopropyle, 3-nitro-benzyle, 2-éthoxy-benzyle, 2,2,2-trifluoroéthyle, 2-(3-trifluorométhyl-phényl)éthyle, 1-indanyle, 2-phényl-éthyle, (2-naphtyl)-méthyle, 2-phényléthyle, phényl-CH(COOH)-, 3-phényl-benzyle, 4-(4-chlorophénoxy)-phényle, 5-chloro-2-méthylphényle, 2-fluorophényle, 1-naphtyle ;
R(3) représente un atome d'hydrogène ;
R(4) représente un atome d'hydrogène ;
R(5) représente un atome d'hydrogène, des groupes chloro, -SO₂CH₂, (2-méthoxy)éthoxy- ;
R(6) représente un atome d'hydrogène ;
R(7) représente un atome d'hydrogène, des groupes méthyle, chloro ;
X représente des groupes -CO-, -CO-CO-, -CO-NH-, -SO₂- ;
ainsi que leurs sels tolérés du point de vue physiologique.

7. Composés de formule I, selon la revendication 5, où
R(1) représente le groupe 2-chlorophényle ;
R(2) représente le groupe 4-méthylbenzyle ;
(R(3)-(R7) représentent un atome d'hydrogène ;
X représente un groupe -CO- ;
ainsi que leurs sels tolérés du point de vue physiologique.

8. Composé de formule I selon la revendication 5, celui-ci étant le 4'-{[benzylthiophène-2-sulfonyl)amino]méthyl}-3'-chlorobiphényl-2-sulfonylcyanamide, ainsi que ses sels tolérés du point de vue physiologique.

9. Composés de formule I selon une ou plusieurs des revendications 1 à 8 et/ou leurs sels tolérés du point de vue physiologique pour l'utilisation comme médicament.

10. Préparation pharmaceutique **caractérisée par** une teneur efficace en un composé de formule (I) selon une ou plusieurs des revendications 1 à 8 et/ou leurs sels tolérés du point de vue physiologique.

11. Préparation pharmaceutique selon la revendication 10 **caractérisée en ce qu'**elle présente en plus une teneur efficace en un inhibiteur de NHE et/ou une substance efficace d'une autre classe de principes actifs cardio-vasculaires, et/ou leurs sels tolérés du point de vue physiologique.

12. Composés de formule I selon une ou plusieurs des revendications 1 à 8 et/ou leurs sels tolérés du point de vue physiologique, pour l'utilisation comme inhibiteur de l'échangeur bicarbonate/chlorure dépendant du sodium.

13. Composés de formule I selon une ou plusieurs des revendications 1 à 8 et/ou leurs sels tolérés du point de vue physiologique pour l'utilisation dans la thérapie et/ou la prévention de l'infarctus du myocarde, de l'angine de poitrine, de maladies induites par des états ischémiques, des insuffisances respiratoires, des états ischémiques du coeur, des états ischémiques du système nerveux périphérique et central et de l'apoplexie, des états ischémiques des organes périphériques et des extrémités, de maladies où la prolifération cellulaire constitue la cause primaire ou secondaire ou dans le traitement des états de choc, ou dans les interventions chirurgicales ou greffes d'organes ou pour la conservation et le stockage de greffes pour des interventions chirurgicales.

14. Composés de formule I selon une ou plusieurs des revendications 1 à 8 et/ou leurs sels tolérés du point de vue physiologique pour l'utilisation dans la thérapie et/ou prévention du cancer.

15. Préparation pharmaceutique selon la revendication 10 et/ou 11 pour l'utilisation dans la thérapie et/ou la prévention de l'infarctus du myocarde, de l'angine de poitrine, de maladies induites par des états ischémiques, des insuffisances respiratoires, des états ischémiques du coeur, des états ischémiques du système nerveux périphérique et central et de l'apoplexie, des états ischémiques des organes périphériques et des extrémités, de maladies où la prolifération cellulaire constitue la cause primaire ou secondaire ou dans le traitement des états de choc, ou dans les interventions chirurgicales ou greffes d'organes ou pour la conservation et le stockage de greffes pour des interventions chirurgicales.

16. Préparation pharmaceutique selon la revendication 10 et/ou 11 pour l'utilisation dans la thérapie et/ou la prévention du cancer.
